# EUROPEAN PATENT SPECIFICATION

(11) **EP 4 412 527 B1**
(45) Date of publication and mention of the grant of the patent: **03.06.2026**
(21) Application number: 22879263.6
(22) Date of filing: 06.10.2022
(51) Int. Cl.: A61B 5/369, A61B 5/055, A61B 5/16, A61B 5/246, A61B 5/38, A61B 5/28, G16H 30/40, G16H 40/60

(54) **BRAIN WAVE ANALYSIS OF HUMAN BRAIN CORTICAL FUNCTION**
GEHIRNWELLENANALYSE DER MENSCHLICHEN GEHIRNRINDENFUNKTION
ANALYSE DES ONDES CÉRÉBRALES DE LA FONCTION CORTICALE CÉRÉBRALE HUMAINE

(30) Priority: 06.10.2021 US 202163252977 P
(43) Date of publication of application: 14.08.2024
(73) Proprietor: Brain F.I.T. Imaging, LLC, Unadilla, NY 13849 (US)
(72) Inventor: FORD, John, P., NY 13849 (US); SUDRE, Gustavo, P., NY 13849 (US)
(74) Representative: Korenberg, Alexander Tal
(86) International application number: PCT/US2022/045839
(87) International publication number: WO 2023/059758

(56) References cited:
- CN-A- 105 852 885
- US-A1- 2017 281 071
- US-A1- 2020 321 124

## Description

### CROSS REFERENCE TO RELATED APPLICATION

This application claim priority to US Provisional Application No. 63/252,977, filed on October 6, 2021.

### FIELD OF THE ART

The present description is directed to the field of medical imaging. More particularly, this description pertains to systems and methods of detecting and evaluating electromagnetic activity in the brain.

### BACKGROUND

Despite rapidly increasing societal burden, progress in developing treatments for neurodegenerative disorders, such as Alzheimer's disease ("AD"), remains slow.

Part of the challenge in developing effective therapeutic agents is the requirement that the molecule cross the blood-brain barrier ("BBB") in order to engage a disease-relevant target. Another challenge, particularly relevant to efforts to develop disease-modifying agents, is the need for non-invasive techniques that can repeatedly be used to monitor disease status and progression. Although several imaging approaches have been used to monitor efficacy of potential disease-modifying antibodies in AD clinical trials - notably positron emission tomography ("PET") detection of β-amyloid plaque burden - these radioisotopic imaging techniques detect a presumptive pathophysiological correlate of disease and do not directly measure the primary symptom, the loss of cognitive function.

Existing approaches to measuring brain function are likewise poorly suited to monitoring neurodegenerative disease status and progression.

Cerebral cortex functional imaging approaches currently in clinical use do not image neural function directly: functional magnetic resonance imaging ("fMRI") images blood flow; positron emission tomography ("PET"), when used to monitor glucose consumption, images metabolism.

In addition, there can be a mismatch between the temporal resolution of certain functional imaging approaches and the duration of signaling events in the brain. fMRI, for example, is sensitive on a time frame of seconds, but normal events in the brain occur in the time frame of milliseconds ("msec").

US2017281071A1 discloses techniques for determining cognitive impairment based on magnetoencephalography (MEG) data.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
FIG. 1 shows schematically a side view of a neuroimaging device, in accordance with some embodiments.
FIG. 2 shows schematically a top view of the neuroimaging device of FIG. 1, in accordance with some embodiments.
FIG. 3 shows schematically a process of inventorying human brain cortical function, in accordance with some embodiments.
FIG. 4A shows schematically the relative locations of neuroimaging sensors from which the neuroimaging data for certain heatmaps was drawn, in accordance with some embodiments.
FIG. 4B schematically shows the layout of neuroimaging sensors of a neuroimaging system used to acquire the neuroimaging data for further analysis, in accordance with some embodiments.
FIG. 5A shows an example response to a stimulus represented as an epoch of MEG data, in accordance with some embodiments.
FIG. 5B shows an example response to a stimulus represented as an epoch of EEG data, in accordance with some embodiments.
FIG. 5C is a conceptual diagram illustrating a heatmap, in accordance with some embodiments.
FIG. 6 is a flowchart depicting a process for generating a graphical representation of neuroimaging data of a subject, in accordance with some embodiments.
FIG. 7 is an illustration of certain timing parameters, in accordance with some embodiments.
FIG. 8 illustrates examples of graphical representations of neuroimaging data of a subject, in accordance with some embodiments.
FIG. 9 is an example of graphical representation that shows differences in neuroimaging data between two runs, in accordance with some embodiments.
FIG. 10 is a graphical representation of neuroimaging data of multiple subjects and a number of parameters, in accordance with some embodiments.
FIG. 11A shows an example heatmap of the epochs from a single session for a normal subject, in accordance with some embodiments.
FIG. 11B shows an example heatmap of the epochs from a single session for an Alzheimer's Disease ("AD") patient, in accordance with some embodiments.
FIG. 11C shows an example heatmap of the epochs from a single session for a second normal subject, in accordance with some embodiments.
FIG. 11D shows a procedure for estimating the candidate parameter nB.
FIG. 12A is a flowchart depicting an example process of processing and analyzing neuroimaging data, according to an embodiment.
FIG. 12B is a conceptual diagram illustrating a sensor selection process, according to an embodiment.
FIGS. 13A, 13B, and 13C illustrate graphical user interfaces for presenting features and heatmaps, in accordance with some embodiments.
FIGS. 14A and 14B illustrate graphical user interfaces for presenting features and comparing multiple heatmaps, in accordance with some embodiments.
FIG. 15 shows schematically a partial display of a report of results of inventorying human brain cortical function, in accordance with some embodiments.
FIG. 16 is a conceptual diagram illustrating a computer-implemented process of generating a background of the normal range of evoked potential of normal volunteers, in accordance with some embodiments.
FIG. 17 shows two example summary plots of a test patient P11 for the first run and the second run, in accordance with some embodiments.
FIG. 18 shows two example summary plots of a test patient P15 for the first run and the second run, in accordance with some embodiments.
FIG. 19 shows two example summary plots of a test patient P16 for the first run and the second run, in accordance with some embodiments.
FIG. 20 shows two example summary plots of a test patient P24 for the first run and the second run, in accordance with some embodiments.
FIG. 21 shows two example summary plots of a test patient P24 for the first run and the second run, in accordance with some embodiments.
FIG. 22 shows two example summary plots of a test patient P27 for the first run and the second run, in accordance with some embodiments.
FIG. 23 shows two example summary plots of a test patient P30 for the first run and the second run, in accordance with some embodiments.
FIG. 24 shows two example summary plots of a test patient P31 for the first run and the second run, in accordance with some embodiments.
FIG. 25 shows two example summary plots of a test patient P32 for the first run and the second run, in accordance with some embodiments.
FIG. 26 shows two example summary plots of a test patient P33 for the first run and the second run, in accordance with some embodiments.
FIG. 27 shows the result of a regression model in predicting MMSE score of a number of subjects, in accordance with some embodiments.
FIG. 28 is a flowchart depicting an example process for analyzing and graphically representing EEG data, in accordance with some embodiments.
FIG. 29 is a graphical illustration of a parameter for the gradual change in number of B peaks between two channels, in accordance with some embodiments.
FIG. 30 is a graphical illustration of a parameter for the percentage of epochs with A peaks, in accordance with some embodiments.
FIG. 31 is a graphical illustration of a parameter for C peak AUC in epochs without A peaks.
FIG. 32 is a graphical illustration of a parameter for C1 AUC ratio between epochs with and without A peaks.
FIG. 33 is a graphical illustration of a parameter for C2 AUC ratio between epochs with and without A peaks.
FIG. 34 is a graphical illustration of a parameter for C3 AUC ratio between epochs with and without A peaks.
FIG. 35 is a graphical illustration of a parameter for average B peak AUC.
FIG. 36 is a graphical illustration of a parameter for stimulus response variability for B peak window.
FIG. 37 is a graphical illustration of a parameter for stimulus response variability for C peak window.
FIG. 38 is a graphical illustration of a parameter for C peak duration.
FIG. 39 is a graphical illustration of a parameter for the ratio between B and C peak AUCs in epochs with those peaks.
FIG. 40 is a graphical illustration of a parameter for percentage of epochs with B peaks.
FIG. 41 is a graphical illustration of a parameter for percentage of epochs with C peaks.
FIG. 42 is a graphical illustration of a parameter for area under the curve in C1 peak.
FIG. 43 is a graphical illustration of a parameter for area under the curve in C2 peak.
FIG. 44 is a graphical illustration of a parameter for area under the curve in C3 peak.
FIG. 45 is a graphical illustration of a parameter for C1 to C3 AUC ratio.
FIG. 46 is a graphical illustration of a parameter for C2 to C3 AUC ratio.
FIG. 47 is a graphical illustration of various timing features, in accordance with some embodiments.

Wherever possible, the same reference numbers will be used throughout the drawings to represent the same parts.

### DETAILED DESCRIPTION

### I. Neuroimaging Technique

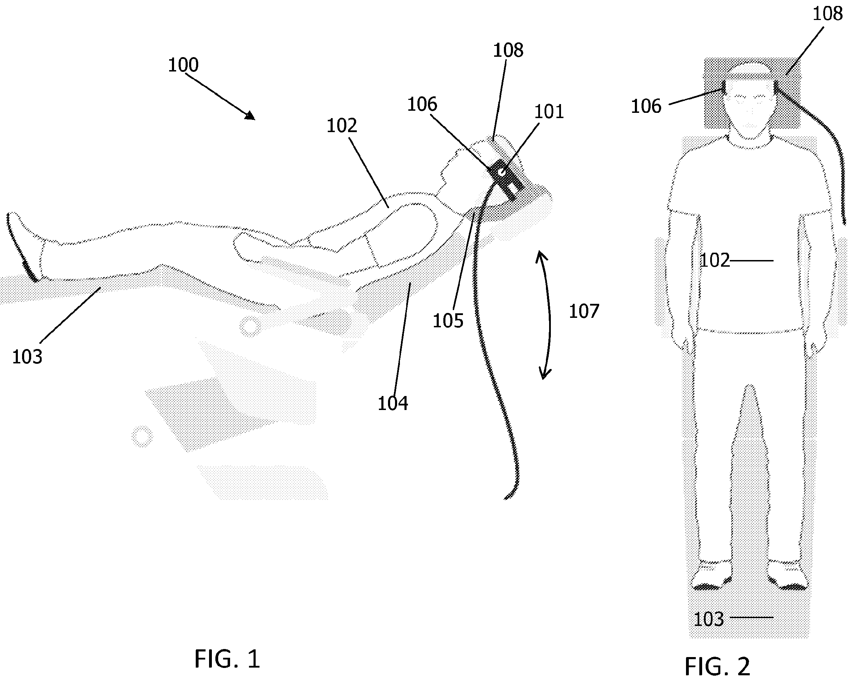

Referring to FIG. 1 and FIG. 2, a neuroimaging device 100 includes one or more sensor 101 sized to collect data from the brain region of interest of the test subject 102. The neuroimaging sensor 101 is applied close to the head. In exemplary embodiments, the neuroimaging device 100 also includes a support apparatus 103, preferably a very comfortable reclining chair, such as, for example, a conventional dental chair with an adjustable support back 104, for the comfort of the test subject 102 that also largely immobilizes the back of the head to stabilize the head position with respect to the support back 104. In some embodiments, the support back 104 includes a neck support 105 that aids in immobilizing the head by immobilizing the neck of the test subject 102. The neuroimaging sensor 101 is also immobilized with respect to the support back 104 such that variability in the placement of the head of the test subject 102 with respect to the neuroimaging sensor 101 is reduced or minimized. The neuroimaging sensor 101 is operatively connected to a computer with appropriate software for the collection of neuroimaging data associated with the auditory stimulus.

In various embodiments, the neuroimaging device may take different forms. In some embodiments, the neuroimaging device is a magnetoencephalography (MEG) device. In some embodiments, the neuroimaging device is an electroencephalography (EEG) device

The neuroimaging sensor 101 is located on a probe 106 that preferably places the neuroimaging sensor 101 as close to the scalp as possible or in direct contact with the scalp and that may be contoured to a part of the contour of the head and also may help to stabilize the head position with respect to the support back 104 and the neuroimaging sensor 101. The probe 106 shown in FIG. 1 and FIG. 2 only covers a small portion of the scalp while locating the neuroimaging sensor 101 over the region of interest of the brain of the test subject 102. In some embodiments, the probe 106 may be a full or near-full helmet that covers all or most of the scalp. In some embodiments, the inner contour of the probe 106 is selected or the configuration of the probe 106 is adjustable based on a measured size and/or contour of the head of the test subject 102. The support back 104 may be adjustable 107 across a range of inclinations.

In some embodiments, the neuroimaging device 100 further includes a strap 108 extending from the support back 104 or the probe 106 for placement around the head of the test subject 102 to further stabilize the head position with respect to the support back 104 and probe 106 and hence with respect to the neuroimaging sensor 101. A second similar strap (not shown) may extend from the support back 104 or the probe 106 on the other side of the head as well. The straps 108 may be flexible or rigid, may extend partially or fully around the head, and may be reversibly fastened to each other or to another structure on the opposite side of the head. The straps 108 may contact the face over the cheekbones to prevent lateral movement of the head.

Neuroimaging sensors 101 are generally cylindrical with a diameter in the range of about 0.25 mm to about 1.5 mm. In some embodiments, the single sensor of a neuroimaging device 100 of the present disclosure is larger than a conventional sensor. The increased sensor detection area based on the increased sensor size increases the timing/amplitude sensitivity of the sensor at the cost of spatial localization. Spatial localization of the signal, however, is not of particular importance for methods of the present disclosure. Appropriate diameters of the single neuroimaging sensor 101 of a neuroimaging device 100 of the present disclosure are in the range of about 0.25 mm to about 2 cm, alternatively about 0.5 mm to about 2 cm, alternatively about 1 mm to about 2 cm, alternatively at least 2 mm, alternatively about 2 mm to about 2 cm, alternatively at least 5 mm, alternatively about 5 mm to about 2 cm, alternatively at least 1 cm, alternatively about 1 cm to about 2 cm, or any value, range, or sub-range therebetween. Other setups are possible in various embodiments. US Patent 10,736,557, entitled "Methods and magnetic imaging devices to inventory human brain cortical function," patented on August 11, 2020, is incorporated by reference for all purposes.

The subject 102 may be stimulated by an activation procedure that may include a series of stimulus steps. In an MEG system, one or more sensors detect electrical activity in the human brain after the stimulus steps, in the form of the magnetic fields generated by the electrical activity. In an EEG system, one or more sensors detect electrical activity in the human brain after the stimulus steps, in the form of the electrical fields generated by the electrical activity. In some embodiments, in an MEG system, signals are captured following an auditory stimulus provided to the subject. Generally, the activation procedure may include providing multiple iterations of an auditory stimulus to the subject. One or more sensors such as SQUID sensors may be used. An epoch may refer to a single measured response or single output over a single predetermined period of time, such as with respect to a single stimulus event. As a specific example, to build an Alzheimer's Disease Detection ("ADD") or Cognitive Impairment (CI) model or evaluate any given patient with respect to the ADD model or CI model, generally multiple epochs are collected. In some embodiment, for each test session, the number of epochs collected was approximately 250, however this may vary by implementation.

The frequency of auditory stimulus, duration of stimulus, and pattern of stimulus may vary by implementation. For example, patients may be presented with a series of 700 Hz standard tones of 50 msec duration, spaced every 2500 msec. With a proportion of 1 to 5, a deviant tone (600 Hz) was randomly presented. All tones were presented to the test patient's left ear, for a total of 250 samples. Test patients were scanned in three different runs, with two of those runs being performed during the same visit. In one embodiment, only the responses to standard tones were analyzed, and responses to deviant tones were discarded.

In some embodiments, specific tone frequencies, tone durations, inter-trial intervals, and numbers of epochs may be used to collect the neuroimaging data. In some embodiments, a range of values may be selected for each. The tone frequencies may be in the range of 500 to 1000 Hz or alternatively in the range of 600 to 700 Hz. The tone duration may be in the range of 25 to 75 msec. The inter-trial intervals may be at least 500 msec or alternatively in the range of 500 to 3000 msec. The total number of epoch collected in a single session may be at least 200 or alternatively at least 250.

The measurement setup and computer particularly may map the magnetic field strength to the surface of the cerebral cortex. An array of SQUID sensors may be located over the cortical region controlling the function to be inventoried. For auditory evoked potential, the sensor heads are placed over the superior temporal gyrus to record initial response to a repeated sound stimulus. The patient support device may be moved to refine the topological image quality. The contour maps of magnetic field intensity may be collected over a 500-600 msec epoch after a defined stimulus (e.g., pitch, intensity, duration, and repetition). To achieve adequate data homogenization in order to render the content of the collected neuroimaging data understandable without degrading it, the data collection may be limited to neural transmission originating in the most superficial neurons lining the sulci of the relevant gyrus of the human cortex.

Data collected from a neuroimaging system may be band-pass filtered, for example by retaining frequencies in the range of 1-30 Hz and removing frequencies outside that range. This helps keep most of the variance in the power of the recordings and also to remove any slow drifts in the data, normally related to recording artifacts. The data may also be otherwise processed, one example of which is segmenting an incoming data stream into separate epochs by time. For example, a computer may determine the timing of the presentation of each standard tone, and data in the 100 msec preceding the presentation, and 500 msec after, may be recorded and averaged over all presentations. This filtering results in one time series per channel, containing 600 samples from -100 msec to 500 msec, where time zero determined the presentation of the standard tone. In some cases, the number of averaged presentations was between 207 and 224, depending on subjects and runs.

Other types of signal processing may also be performed. For example, data collected by the ELEKTA NEUROMAG^{®} 306 channel system may be further processed using Elekta NEUROMAG' S MAXFILTER^{™} software to remove sources of outside noise. Depending upon the physical setting of data collection and specific data collection tools used, additional or even fewer signal processing steps than described herein may be helpful as well, particularly due to variation based on the physical location of the recording (e.g., the amount of external noise in the site). Thus, signal processing may not be necessary based on the recording instrument and site used in future applications of this description.

In some embodiment, the neuroimaging system may be an EEG system. The activation procedure may include a series of auditory tones. The stimuli may include tones at certain frequencies such as those that are commonly referred as the standard tones (1000 Hz). In some embodiments, standard tones, target tones (2000 Hz), and unexpected distractor tones (white noise) may be played with probabilities of .75, .15, and .10. Tones may be presented in pseudorandom order. In some embodiments, the target and distractor tones are presented sequentially. Subjects may be instructed to respond to the target stimuli by pressing a button with their dominant hand. For each test session, between 300 and 400 stimuli may be presented binaurally through insert ear phones at 70-dB volume. The tone duration for each stimulus may be 100 ms with rise and fall times of 10 ms. The interstimulus interval may be randomized between 1.5 and 2s.

In some embodiments, EEG activity may be recorded from one or more electrode sites. For example, electrode sites Fz, Cz, Pz, F3, P3, F4, and/or P4 of the international 10-20 system using a COGNISION Headset (NEURONETRIX) may be used. Electrodes may be referenced to averaged mastoids (M1, M2), and Fpz served as the common electrode. The headset used for data collection may be validated to perform reliable ERP recordings when skin contact impedance is 70 kU. Impedance may be checked at all electrodes after each target or distractor tone, and was kept below this limit throughout each test. Data may be collected from 2240 to 1000 ms around the stimuli, digitized at 125 Hz, and bandpass filtered from 0.3 to 35 Hz. In some embodiments, an automatic artifact threshold detection limit of 6100 mV may be set for the tests. Trial sets of a deviant tone and the immediately preceding standard tones (epoch sets) with artifacts exceeding the threshold are rejected in real time and immediately repeated. The physical set up of EEG sensors is known in the art. An example set up of EEG sensors is described in publication by Cecchi et al., entitled "A clinical Trail to Validate Event-Related Potential Markers of Alzheimer's Disease in Outpatient Settings," published at Alzheimer's & Dementia: Diagnosis, Assessment & Disease Monitoring 1 (2015) 387-394, which is incorporated by reference herein for all purposes.

Trial averaging and extraction of event-related potential (ERP) measures may be collected. EEG data from each trial may be baseline corrected using the pre-stimulus period and averaged according to stimulus. In some embodiments, for standard tones, only the trials immediately preceding target and distractor stimuli are averaged. During data preprocessing, recordings that exceeded two times the root mean square value (RMS) for the EEG test data or with wrong button presses may be rejected and excluded from averaging. ERP waves that averaged less than 20 trials after preprocessing may be eliminated from the analysis.

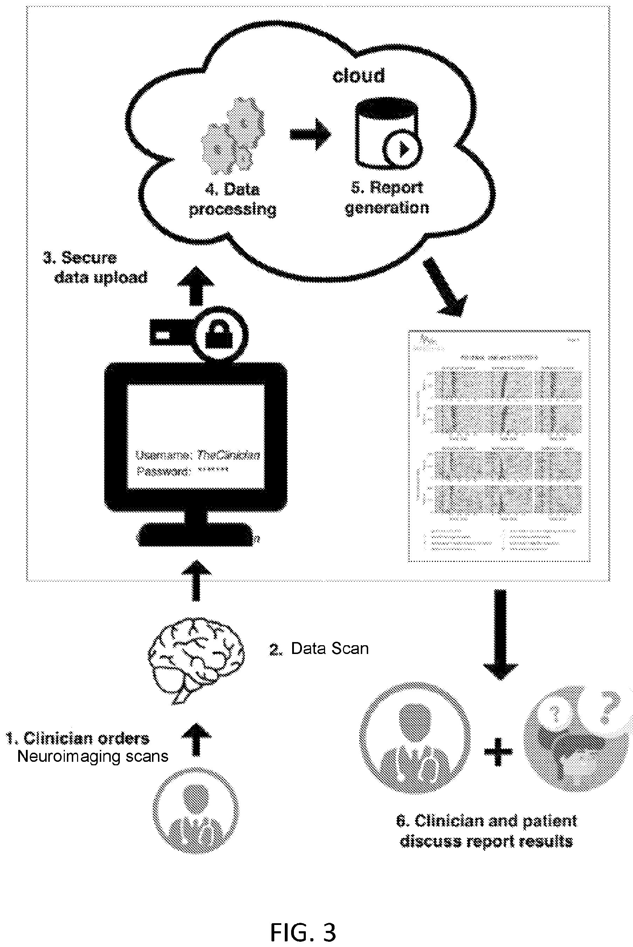

FIG. 3 shows a system and a process for acquiring and analyzing neuroimaging data and reporting results of the analysis for a test subject. The system and process may be used for any suitable types of neuroimaging data, including EEG data, MEG data, and any combination of data. The system and process include a web application that consumes the data files generated by a neuroscan of a patient and returns to the clinician a detailed report of features reflecting the patient's cognitive function based on our proprietary algorithms.

The system may be broken down between two parts: the analysis of the data and the portal. The analysis of the data includes a script that processes data and another script that generates the visual report. The portal encompasses all the online infrastructure for user authentication, data upload, providing the report back to the user, and additional functionalities. The portal receives, organizes, and pipes the data uploaded by clinicians into the processing script and then stores and feeds the report back to the clinician.

Since the system is designed as a web application, it is deployed in a secure virtual private cloud (VPC) using a web service, such as, for example, Amazon Web Services (AWS), and is accessed through online computers in a clinic. The subject sits in a comfortable chair while the sensor helmet covers at least the relevant portion of the subject's head. The activation procedure may include the subject listening for an identical sound repeatedly while keeping her eyes closed. The subject merely needs to stay still and is sometimes distracted by a different sound to help maintain focus. The sensor helmet is part of a device approved by the Food and Drug Administration (FDA) for clinical use (for example, ELEKTA NEUROMAG' S System: K050035 or CTF's OMEGA System: K030737). The data acquired in the device is the input signal (e.g., files to be uploaded), which later returns the visual report to clinicians.

In some embodiments, the subject may be exposed to about 250 stimuli sound tones with loudness adjusted for the subject's hearing. The sound tones occur one every 2.5 seconds, and the series of epochs (a run) lasts about 20 minutes. After a 45 minute break, there is a repeat 20-minute run. The entire visit, including the break, takes about 1.5 hours and includes two test sessions. In some embodiments, if the subject has extensive dental hardware that cannot be removed, or other ferromagnetic metal in their bodies that interfere with the neuroimaging signal, the data may not be useful.

The clinician then securely transfers the neuroimaging data to the system cloud, where it is analyzed and the system report is generated within a few minutes. The clinician can then discuss those results with the patient.

The data analysis may be performed on secure servers. Results may be ready in less than ten minutes, and the practitioner then gets notified that a report for that patient's visit is ready for review.

After logging in to her account, the practitioner can see all of her patients in a single list and also can edit, remove, or view visit information for each patient. Results for each assessment are stored in Visit records. In the Visit view, the practitioner can also see the visit date, analysis status, and any comments entered when creating that visit record. Finally, three familiar icons can be seen to the right of each visit entry that allows the practitioner to remove, view visit details, or view the report for a visit.

When data is successfully acquired for a visit, one file for each run should be uploaded, along with the visit date. The data are uploaded in the background, and processing commences as soon as the files are received by the servers. When the processing is complete and the report is ready, the visit status is updated on the website, and the practitioner is notified by e-mail that a report is ready for viewing.

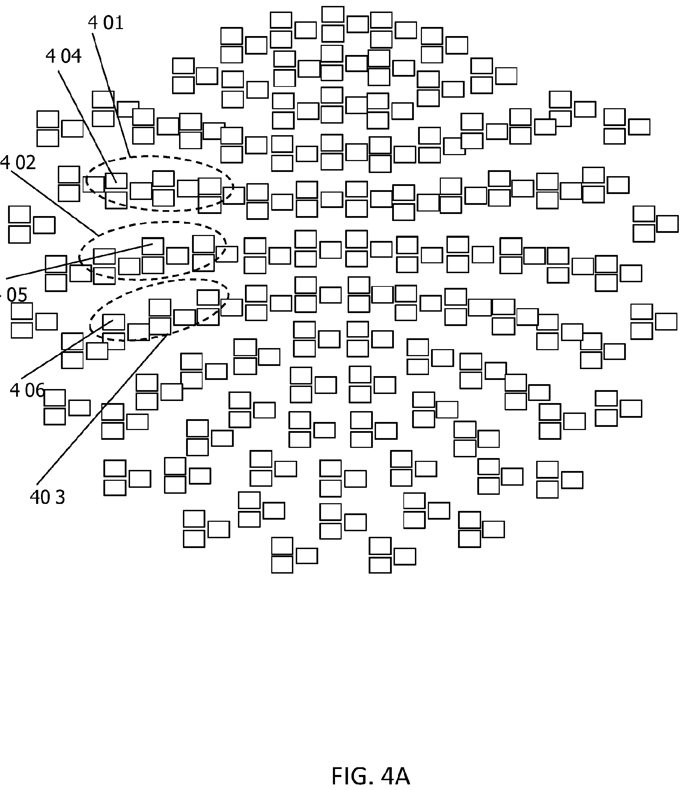

FIG. 4A schematically shows the layout of neuroimaging sensors in a sensor helmet of a neuroimaging system used to acquire the neuroimaging data for further analysis, in accordance with some embodiments. The neuroimaging system in this example may be a MEG system. The dashed ellipsoids 401, 402, 403 show the three spatially-closest neuroimaging sensors in the candidate pool, sharing the same gradiometer orientation, with the neuroimaging signal being similar for all three of them for two different patients, one being a cognitively-impaired patient and the other being a normal patient.

Neuroimaging data was acquired from each of the two patients in one session on a first day and in two separate sessions on a second day. In some embodiments, data from the indicated MEG sensors 404, 405, 406 may be selected for further analysis. The neuroimaging sensor 405 within the middle dashed ellipsoid 402 may be used from the first run for the cognitively-impaired patient. The neuroimaging sensor 404 within the top dashed ellipsoid 401 may be used from the first run for the normal patient. The neuroimaging sensor 406 within the bottom dashed ellipsoid 403 was used from the second and third runs for the cognitively-impaired patient. The neuroimaging sensor 405 within the middle dashed ellipsoid 402 was used from the second and third runs for the normal patient. The spatial resolution of the neuroimaging sensor does not significantly affect the quality of the acquired data, and a single sensor placed anywhere in that vicinity is expected to be capable of acquiring an appropriate signal for analysis. The variability in the location of the selected sensor in FIG. 4A is believed to be based on a change in patient head position with respect to the neuroimaging sensor between runs rather than a different best data acquisition location in the brain, indicating the importance of placing the neuroimaging sensor as close as possible to the head.

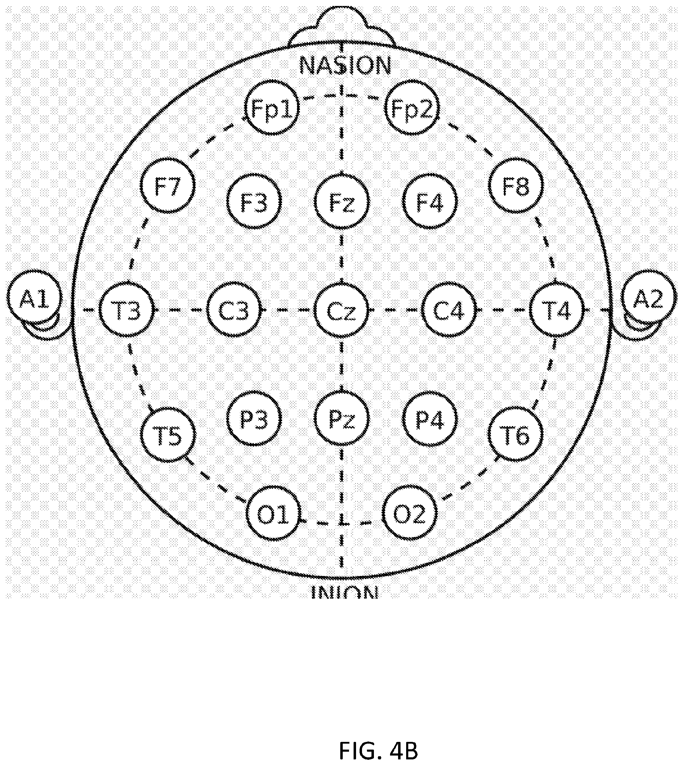

FIG. 4B schematically shows the layout of neuroimaging sensors of a neuroimaging system used to acquire the neuroimaging data for further analysis, in accordance with some embodiments. The neuroimaging system in this example may be an EEG system. The system may include various electrode sites. The positions and nomenclature of the electrode sites are known in the art. In some embodiments, signal data from various electrode sites may be analyzed separately and compared to determine patterns of signals across different electrode sites. In some embodiments, a headset may have 7 channels that capture the signals from the electrode sites Fz, Cz, Pz, F3, P3, F4, and P4. In some embodiments, a binaural stimulus is used for capturing signals from a subject. Centrally located channels may be used to extract peak timing. Features from various channels (e.g., all 7 channels in the headset) may be used in data analysis.

### II. Signal Form and Measurement

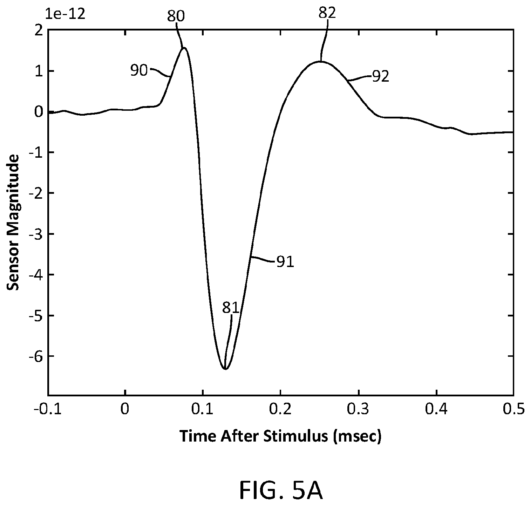

FIG. 5A illustrates the averaged response of a signal (a "signal illustration") to the standard tone for a SQUID sensor, both gradiometers and magnetometers, with each signal illustration being arranged in a location in FIG. 4A corresponding to the relative location of the SQUID sensor 32 in the array in the sensor head, according to one embodiment. Each signal illustration in FIG. 5A represents one of the sensors (not separately labeled), where the horizontal axis goes from -100 to 500 msec, where 0 represents the time at which the tone was presented to the patient. As discussed above, the Y axis value for signal received from the SQUID sensor 32 is a quantification of magnetic activity measured in a particular part of the brain, as indicated by magnetic fields detected by the SQUID sensors 32.

Zooming in on an example SQUID sensor's response provides a prototypical waveform pattern such as shown in FIG. 5A, which shows an example of an averaged evoked stimulus response in an area of interest in the brain as measured by a single SQUID sensor of the sensor head. The positive and negative sensor magnitude depends on the position of the sensor and are therefore arbitrary, but peak B 91is shown and described as a negative peak throughout the present disclosure for consistency. The example waveform pattern of FIG. 5A was collected from a test patient with no measured cognitive dysfunction.

The human brain's response to the auditory stimulus, on average and for particularly placed SQUID sensors, includes several curves that peak. In MEG data, these peaks include a peak A 90 defining a first local maximum 80, followed by a peak B 91 defining a local minimum 81, followed by a peak C 92 defining a second local maximum 82, followed by a return to a baseline. Peak A 90 is commonly known in the literature as "P50" or "m50". Peak B 91 is commonly known in the literature as "N100", "m100", or an awareness related negativity ("ARN") peak. Peak C 92 is commonly known in the literature as "P200". On average, the first local maximum 80 is generally observed within about 50 to 100 msec after the stimulus. The local minimum 81 is generally observed between about 100 and 150 msec after the stimulation. The second local maximum 82 is generally observed between about 200 and 400 msec after the stimulation event.

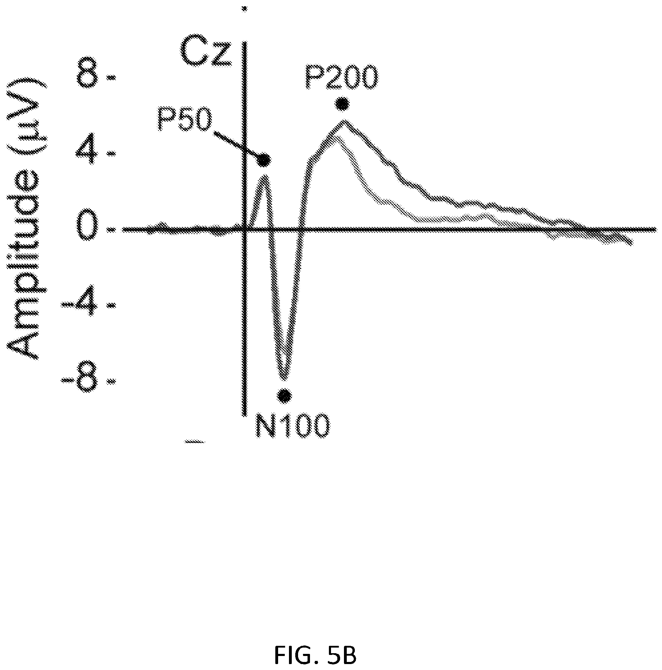

FIG. 5B illustrates the averaged response of a signal to the standard tone for using the EEG technique. Similar to an MEG epoch, an EEG epoch may include Peak A, which is commonly known in the literature as "P50" or "m50". The EEG epoch may also include Peak B, which is commonly known in the literature as "N100", "m100", or an awareness related negativity ("ARN") peak. The EEG epoch may further include Peak C, which is commonly known in the literature as "P200".

Various features may be extracted from an epoch. For example, peak amplitude of the ERP features may be measured as the difference between the mean pre-stimulus baseline and maximum peak amplitude. Peak latency may be defined as the time point corresponding to the maximum amplitude and was calculated relative to stimulus onset. P50 and N100 may be measured from all stimuli. P200 may be measured from standard and target tones. N200, P3b, and slow wave may be measured from the target tone and P3a from the distractor tone.

In some embodiments, the Peak A P50 may be defined as the maximum positivity between 24 and 72 ms poststimulus. Peak B N100 may be defined as the maximum negativity between 70 and 130 ms. Peak C P200 may be defined as the maximum positivity between 180 and 235 ms. In some cases, N200 may be defined as the maximum negativity between 205 and 315ms. The P3a may be defined as the maximum positivity between 325 and 500 ms. The P3b may be defined as the maximum positivity between 325 and 580 ms. A slow wave may be defined as the maximum negativity between 460 and 680 ms. The time windows may be determined by inspecting individual averages and group grand averages

Throughout the remainder of this description and in the claims, it is sometimes useful to refer to these peaks without reference to which specific peak is intended. For this purpose, the terms "first peak", "second peak", and "third peak" are used. Where the "first peak" is either peak A 90, peak B 91, or peak C 92, the "second peak" is a different one of the peaks from the "first peak", and the "third peak" is the remaining peak different from the "first peak" and the "second peak". For example, the "first peak" may be arbitrarily associated with peak B 91 for this example, with the "second peak" being peak A 90 and the "third peak" being peak C 92, and so on.

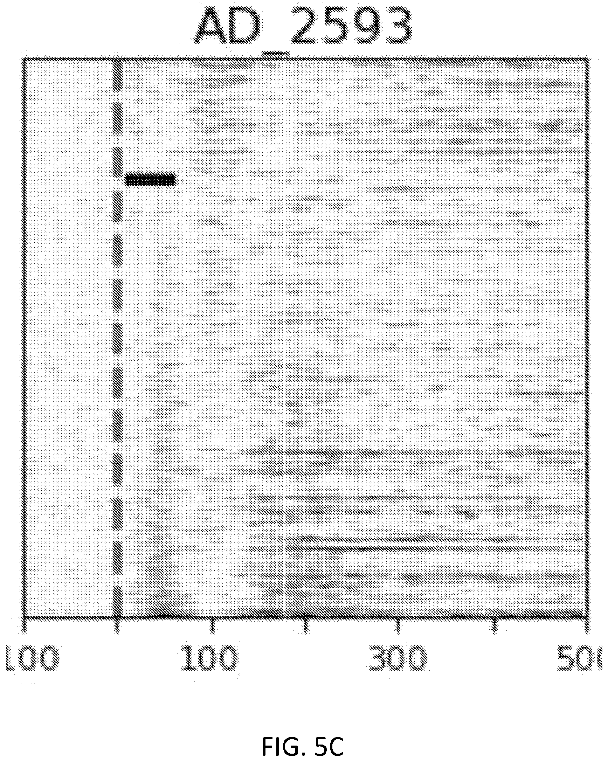

FIG. 5C is a conceptual diagram illustrating a heatmap, in accordance with some embodiments. A heatmap is a compilation of a plurality of epochs. The epochs can be MEG or EEG data. The epochs are sorted by a particular way based on values of one or more metrics that measure the features of the epochs. Each epoch is represented as a row in the heatmap and, in the example shown in FIG. 5C, the heatmap combines over 200 epochs (e.g., over 200 rows) and a distribution is illustrated as the heatmap. The heatmap may includes various colors of different degrees to represent positive values (e.g., positive peaks) and negative values (e.g., troughs) in each epoch. For example, blue color, from dark to light, can be used to represent the degree of negativity and red color, also from dark to light, can be used to represent the degree of positivity.
For example, the example heatmap in FIG. 5C has epochs on the y-axis and time with respect to the stimulus time on the x-axis. Each heatmap may represent one complete auditory stimulation test run for a subject. Each epoch represents a response to a single stimulus. In a heatmap, white refers to a neutral (close to baseline) magnetic or electrical field as measured by a sensor, while red arbitrarily refers to a positive magnetic or electrical field and blue arbitrarily refers to a negative magnetic or electrical field. For each epoch, the color scale is normalized from blue to red based on the data in the epoch. The relative intensity of the positive or negative field is indicated by the intensity of the red or blue color, respectively. The epochs in a heatmap are not ordered chronologically but rather by a metric of the signal. Any one of a number of different sorting metrics may be used. For example, the epochs in the heatmap may be sorted based on the duration of one of the three peaks, the maximum of one of the three peaks, or the latency of one of the three peaks, etc.
In some embodiments, due to the variation across epochs, valuable additional information may be obtained by analyzing the neuroimaging data in heatmaps. Visualizing the neuroimaging data in the form of a heatmap, such as the one shown in FIG. 5C, allows visual inspection of the set of raw epoch data to identify trends and parameters that are hidden or lost in averaged or otherwise collapsed or conflated neuroimaging data. In such a heatmap, each of the responses, or epochs, is plotted as a horizontal line with a color scale representing the strength of the measured magnetic field. These heatmaps allow visual interpretation of the set of raw epoch data that the computer processes in generating and using a model in predicting cognitive impairment (e.g., an ADD model). Although for convenience some of the following descriptions of the generation and use of the ADD model are described with respect to calculations that may be performed with respect to and on the data in these heatmaps, those of skill in the art will appreciate that in practice the computer performs calculations with respect to the data itself, without regard to how it would be visualized in a heatmap.
At least some of the candidate parameters for the ADD model were identified or are more easily explained by looking at the non-averaged epochs of neuroimaging data organized in heatmaps. Some of these candidate parameters include a percentage of epochs having a particular peak or combination of peaks. The determination of whether or not a given epoch has a given peak can be based on any one of a number of calculations.
Additional candidate parameters include identified subsets of epochs in a given set of scans from a single session for a given sensor. Specifically, two (or more) subsets may be identified for a given test patient dividing the epochs based on any one of the candidate parameters or some other aspects. For example, two subsets may be identified, based on a candidate parameter such as presence of one of the peaks where presence is a relative measure of magnetic field strength relative to the other epochs for that test patient. In this example, the subset with the peak being present may be divided into two further subsets of a "stronger" subset including some threshold proportion of the epochs (e.g., 50%) with the higher (or stronger, or strongest) relative presence of the peak, and also of a "weaker" subset including the remaining proportion of the epochs with the lower (or weaker, or weakest) relative presence of peak (or absence thereof). Other candidate parameters or aspects of the epoch data may also be used to generate subsets, such as strong and weak subsets, including, for example, peak timing and variability, and peak amplitude and variability.
Yet additional candidate parameters may be determined based on those identified subsets. For example, any given candidate parameter mentioned in this disclosure may be determined with respect to an identified subset of epochs. For example, if a strong peak A subset is identified, which may represent 50% of the epochs in the set of scans from a single session of a patient having the strongest relative presence of peak A compared to a weak peak A subset, another candidate parameter may be the mean or median amplitude (in terms of magnetic field strength) of the peak B in the strong subset. One of skill in the art will appreciate the wide variety of possible candidate parameters that may possibly be generated by dividing the epoch data from the set of scans from a single session of a patient and sensor according to one aspect/candidate parameter, and then calculating another candidate parameter based on an identified subset.

The generation of a heatmap may be carried by sorting the epochs in a particular manner based on values of a selected feature (e.g., a selected parameter). The epochs may be sorted by the ascending or descending order of the feature values. For example, the selected feature may be an amplitude of one of the peak A, peak B, or peak C. The epoch can be sorted by the amplitude. A computer may select additional stable features and sort the epochs based on the additionally selected features. Additional heatmaps that are sorted by different features can be generated. A feature may also be a compound feature that includes several sub-features, such as the number of B peaks in weak A peaks. The heatmaps may be displayed in a report.
Based on the report, whether the test patient is cognitively impaired may be determined by a computer or by a medical professional. For example, one or more heatmaps with sorted epochs are displayed. A medical professional may rely on the heatmaps to decide whether the heatmap shows any evidence of cognitive impairment. In one embodiment, a machine learning model may be trained. The detail of training a machine learning model is discussed in further detail. The computer inputs the data of the epochs to a machine learning model. The machine learning model provides an output such as a label or a score that corresponds to the likelihood of the test patient being cognitively impaired.
Throughout this disclosure, examples of data processing, feature selection, and visual representations of data such as heatmaps may be illustrated in by various MEG data or EEG data. While some of the examples are illustrated with one type of neuroimaging data (e.g., MEG data), the principles and processes described may be applied to another type of data (e.g., EEG data), and vice versa.

### III. Graphical Representation of Neuroimaging Data

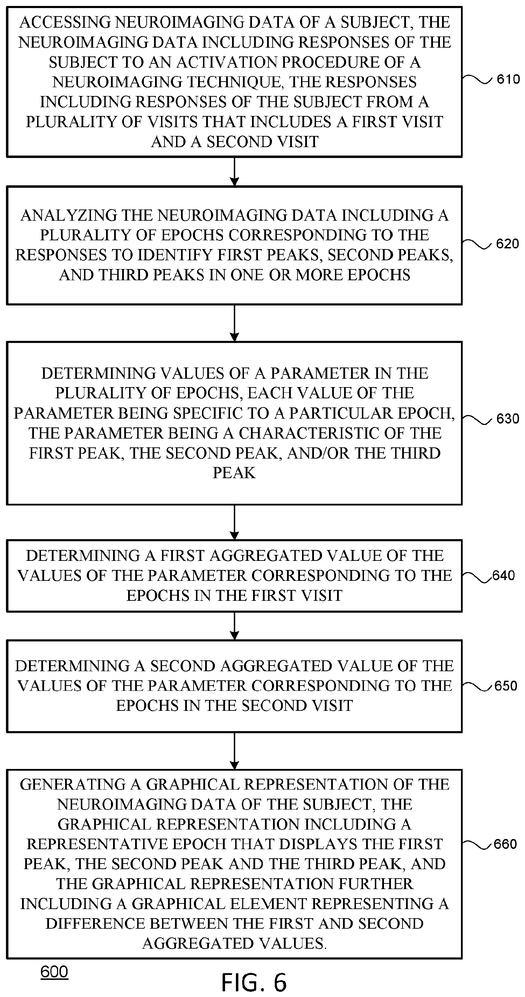

FIG. 6 is a flowchart depicting a process 600 for generating a graphical representation of neuroimaging data of a subject, in accordance with some embodiments. In step 610, neuroimaging data of a subject is accessed. For example, the neuroimaging data includes responses of the subject to an activation procedure of a neuroimaging technique. The neuroimaging technique may be EEG or MEG. The activation procedure may include sending the subject a series of auditory stimulus events. Examples of the activation procedure are in further detail with reference to FIGS. 1 and 2. The responses may include responses of the subject from multiple runs. For example, the neuroimaging data include data from a first run and a second run and the process 600 may be used to generate a graphical representation that may be used to illustrate a difference in the stimulus responses of the subject. In some embodiments, the first run and the second run may occur on different days such as occurred in different patient visits. In some embodiments, the first and second runs occur on the same day, such as corresponding to the morning and afternoon sessions of the visit.

In step 620, the neuroimaging data may be analyzed, such as by a computer. For example, the neuroimaging data, such as MEG data or EEG data includes a plurality of epochs corresponding to the responses of the subject. Each epoch may correspond to the measurement of sensor magnitude over time after a stimulus event. The computer identifies first peaks, second peaks, and third peaks in one or more epochs. For example, some epochs may include all three peaks. In other cases, some epochs may include one or more peaks. In some cases, a peak may be defined as the maximum value or minimum value of the sensor magnitude within a period of time after the stimulus event. For example, in some embodiments, Peak A P50 may be defined as the maximum positivity between 24 and 72 ms poststimulus. Peak B N100 may be defined as the maximum negativity between 70 and 130 ms. Peak C P200 may be defined as the maximum positivity between 180 and 235 ms.

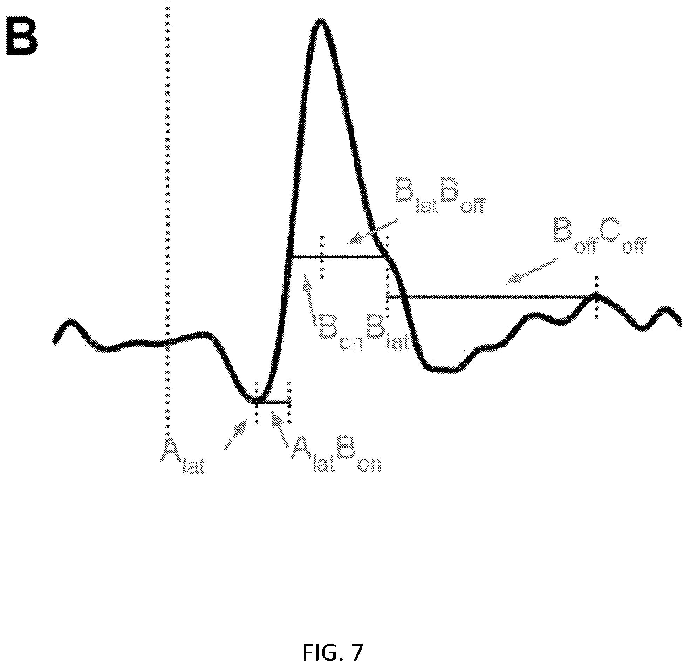

In step 630, values of a parameter in the plurality of epochs may be determined. The parameter may be a characteristic of the first peak, the second peak, and/or the third peak. For example, the characteristic can be whether a particular epoch has a type of peak. In another example, the characteristic may be the onset of a type of peak, the latency of a type peak, the amplitude of a type of peak, etc. In some embodiments, the parameter may also be a characteristic between two peaks. For example, the parameter may be a time between one of the peaks' latency and another peak's onset. Common examples of parameters may include Aₗₐₜ, the time between zero and peak A latency, AₗₐₜBₒₙ, the time between peak A latency and peak B onset, BₒₙBₗₐₜ, the time between peak B onset and peak B latency, BₗₐₜB_{off}, the time between peak B latency and peak B offset, B_{off}C_{off}, the time between peak B offset and peak C offset, A_{pct}, the percentage of epochs with A peak, B_{pct}, the percentage of epochs with B peak, and C_{pct}, the percentage of epochs with C peak. In some embodiments, latency corresponds to the time point in which the peak reaches its maximum absolute value. An onset is the time in which a peak surpasses 2 standard deviations of the baseline signal (time < 0). An offset is the time in which the signal returns to a value below that same threshold. Variability features measure the degree of dissimilarity across epochs within a time window. FIG. 7 is a graphical illustration of some of the features discussed. Additional examples of features are further discussed in Section VII.

In some embodiments, each value of the parameter is specific to a particular epoch. For example, the latency value of a peak in an epoch is different from the latency value of the same type of peak in another epoch. A computer may analyze different epochs in the neuroimaging data and measure values of a parameter in different epochs.

In step 640, a first aggregated value of the values of the parameter corresponding to the epochs in the first run may be determined. For example, during the first run, 250 rounds of stimulus events are applied to the subject, thereby generating about 250 epochs. In some cases, some of the epochs may be disregarded due to noise or other failures. For each epoch that is analyzed, the value of the parameter is determined. The collection of different values that correspond to the epochs in the first run may be aggregated to determine the first aggregated value. The aggregated value may be the percentage, average, variance, standard deviation, maximum, minimum, etc. For example, the variance of the set of values may be determined. In step 650, similarly, a second aggregated value of the values of the parameter corresponding to the epochs in the second run may be determined. The way to determine the aggregated value of the parameter for the second run may be the same as step 640.

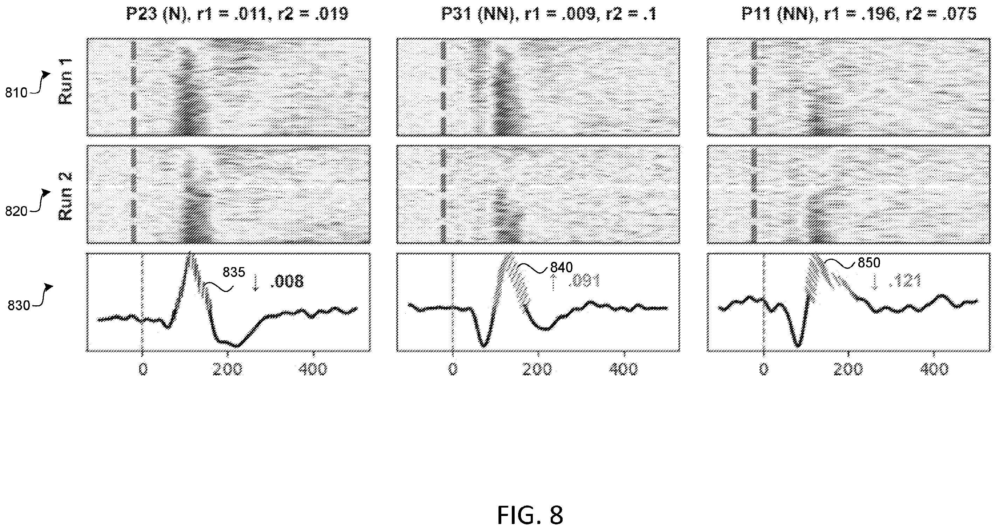

In step 660, a graphical representation of the neuroimaging data of the subject may be generated. The graphical representation may include a representative epoch that displays the first peak, the second peak and the third peak. The representative epoch may be the average of one or more epochs in the neuroimaging data. For example, a computer may take an average of multiple epochs in a run or in the entirety of the neuroimaging data. The graphical representation may further include a graphical element representing a difference between the first and second aggregated values. In some embodiment, the graphical element representing the difference between the first and second aggregated values may be displayed at a peak that corresponds to the parameter. For example, the graphical element includes a sloped line whose slope value is determined based on the difference between the first and second aggregated values. In this particular example, say the parameter is related to the variability in B peak signal. The sloped line may be displayed at the B peak to illustrate this is a parameter indicator related to B peak. Various examples of graphical representation of neuroimaging data are further illustrated in FIG. 8 through FIG. 38.

The neuroimaging data and the graphical representation of the data may have various applications, depending on embodiments. In some embodiments, the neuroimaging data may be input to a machine learning model. The machine learning model may determine a cognitive impairment score of the subject. Additional discussions of various machine learning models used are further discussed in Section VIII. In some embodiments, the graphical representation may be used by a medical practitioner to provide a diagnosis of a cognitive condition of the subject or to evaluate treatment of the cognitive condition of the subject. For example, a medical doctor may provide a diagnosis of a cognitive condition of the subject based on the graphical representation. In some embodiments, the graphical representation may be used by a medical practitioner to determine treatment of a cognitive condition, including administering a clinically approved dosage of an anti-cognitive impairment therapeutic agent to a patient diagnosed with a cognitive impairment condition based on the determination made from the graphical representation. In some embodiments, the graphical representation may be used by a medical practitioner for setting a dosage of an anti-cognitive impairment therapeutic agent in a subject. For treating and setting a dosage for a patient, a medical practitioner may review a series of graphical representations across multiple runs to determine whether a change in cognitive condition is observed from the neuroimaging data. For example, a first graphical representation may show a change between the first run and the second run, a second graphical representation may show a change between the second run and the third run, and so on. The medical practitioner can review the series of graphical representations and administer dosage accordingly.
Cognitive impairment conditions that can be diagnosed and/or treated using various processes described include Alzheimer's disease, Lewy Body Dementia, Frontotemporal Dementia, Vascular Dementia, Mixed Dementias that include Wernicke's Encephalopathy, Huntington's Disease, Parkinson's Disease, Creutzfeldt-Jakob Disease.
In various embodiments, a medical practitioner may administer Acetylcholinesterase inhibitors. Provocatively increases in acetylcholine are associated with increased engagement, which may be identified through the change in one or more parameters in the neuroimaging data. A medical practitioner may also administer 5-23 mg/day of Donepezil at, 4 mgX2/day of Galantamine, and/or 1.5-13.3mg/day of Rivastigmine. The range and precise dosage may be titrated based on the results of the neuroimaging data.
In some embodiments, a medical practitioner may also administer an NMDA antagonist. NMDA transmitters, such as glutamate, may be provocatively excitatory and fatigue generating. For example, Namenda at 7-28 mg/day may be administered to a subject. A medical practitioner may adjust the dosage of the NMDA antagonist based on the results of the neuroimaging data.
In some embodiments, a medical practitioner may also administer may also administer one or more antidepressants to a subject. The antidepressants may include 20-40 mg daily of Citalopram, 10-20 mg daily of Escitalopram, 10-80mg daily of Fluoxetine, 100-300mg daily of Fluvoxamine, 10-60mg daily of Paroxetine, 12.5-75mg daily of Paroxetine extended-release, 25-200mg daily of Sertraline, 50mg daily of Desvenlafaxine, 60mg daily of Duloxetine, 40-120mg daily of Levomilnacipran, 75-375mg daily of Venlafaxine, and 75-225mg daily of Venlafaxine extended-release. A medical practitioner may adjust the dosage of an antidepressant based on the results of the neuroimaging data.
In some embodiments, a medical practitioner may also administer anxiolytics and antioxidants to a subject and adjust the dosage based on the results of the neuroimaging data. In some embodiments, for Alzheimer's Disease, a medical practitioner may administer Aducanumab-binds aB monomers and oligomers at 1-10 mg/kg Q4weeks IV and adjust the dosage based on the results of the neuroimaging data.
In some embodiments, a medical practitioner may recommend a non-drug therapy such as Reminiscence Therapy, Cognitive Stimulation Therapy, and Reality Orienting Therapy based on the results of the neuroimaging data. A medical practitioner may recommend lifestyle changes such as staying active, staying organized, diet such as MIND diet and counseling based on the results of the neuroimaging data.

FIG. 8 illustrates examples of graphical representations of neuroimaging data of a subject, in accordance with some embodiments. FIG. 8 includes data illustrations of three subjects P23, P31, and P11. P23 is a normative (N) subject and P31 and P11 are non-normative (NN) subjects, who are diagnosed with one or more conditions of cognitive impairment. For each subject, FIG. 8 shows a first heatmap 810 that represents sorted epochs in a first run and a second heatmap 820 that represents sorted epochs in a second run. How a heatmap is generated and the epochs are sorted within a heatmap will be discussed in further detail with reference to Section V. FIG. 8 also shows a graphical representation 830 of an aggregated difference in a parameter between two runs. The parameter used in this particular example is B peak variability. The graphical representation 830 highlights the differences between two runs that are depicted in the two heatmaps 810 and 820.

The graphical representations 830 shows run to run differences of the parameter for the three subjects. In this example, the parameter value for each epoch is determined. The overall peak variability value is aggregated from the parameter values determined from the epochs in a run. The process is repeated for the first run and the second run. For each subject, the difference between the variability values of the two runs is determined and compared to a threshold. If the difference is smaller than a threshold, meaning the parameter values are not showing too much difference between two runs, a representative epoch is shown. The representative epoch may be the average of one or more epochs in the neuroimaging data of a subject. For example, for the subject P23, the peak variability value is determined as 0.011 for the first run and 0.019 for the second run. The difference is 0.008, which is a small number. As such, a representative epoch 835, which is aggregated from the epochs of the subject P23 is shown in graphical representation 830. The precise threshold level may be determined experimentally. For example, it can be a multiple of standard deviation (e.g., 1.5x STD, 2x STD, etc.) of differences in variability across multiple subjects that is statistically determined to separate most of the normal subjects and the cognitively impaired subjects.

In a second example, the subject P31 has a peak variability value of 0.009 in the first run and a value of 0.1 in the second run. The data shows that the subject P31 has much higher B peak variability in the second run compared to the first run, with a difference of 0.091, more than 10 folds of the difference determined in the normal subject P23. The increased variability in the second run may be a sign of fatigue, which may be an indication of cognitive impairment. In the graphical representation 830 for the second subject P31, a positively sloped line 840, which may be an example of a graphical element discussed in the process 600, is displayed to indicate that the peak variability increased in the second run. A representative epoch is displayed in the graphical representation 830 of the subject P31, but part of the B peak of the representative epoch is replaced with the positively sloped line 840. The placement and angle of the positively sloped line 840 may also be an indication that the parameter used (B peak variability) is related to B peak.

In a third example, the subject P11 has a peak variability value of 0.196 in the first run and a value of 0.075 in the second run. The subject has a lower variability in the second run, with a difference of -0.121. In the graphical representation 830 for the third subject P11, a negatively sloped line 850 is displayed to indicate that the peak variability decreased in the second run. In some embodiments, the slope of the line 840 or 850, whether positive or negative, may be commensurate with the value of the difference. For example, the higher the variability is in the second run, the more positively sloped line is displayed in the graphical representation 830. The sloped line may be repeated to provide a more visually appealing illustration. Forward slashes indicate a significant increase in variability between runs, backward slashes indicate a decrease.

While B peak variability is used as an example parameter for the illustration in FIG. 8, another parameter that is discussed in this disclosure may also be used to generate a graphical representation 830. For a parameter that is associated with a different peak, the placement of a graphical element illustrating the change in the parameter may be placed at a peak other than the B peak. Also, in some embodiments, more than one parameter may be used and illustrated in a graphical representation. Likewise, the sloped lines 840 and 850 are also illustrated as examples only. Other types of graphical symbols, marks, shading, letters, numbers, and other suitable illustrations may also be used.

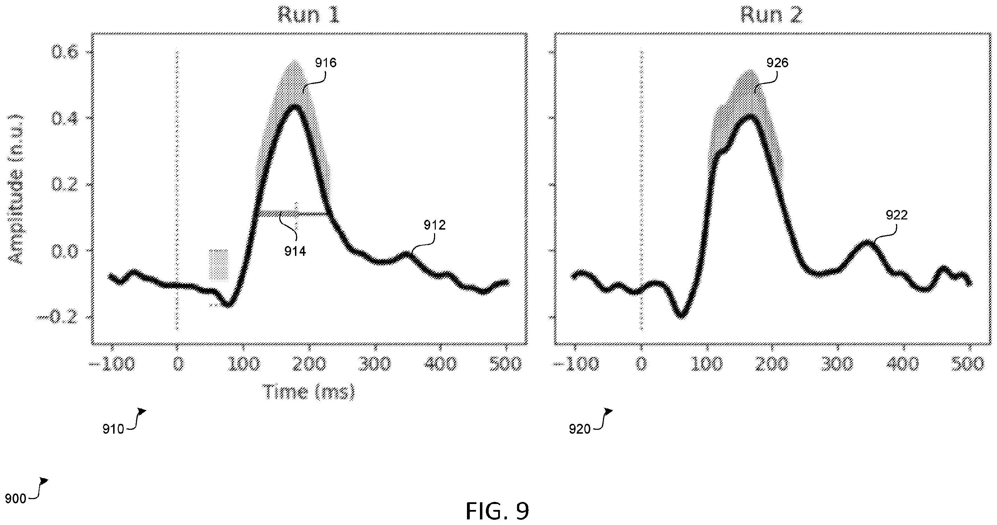

FIG. 9 is an example of graphical representation 900 that shows differences in neuroimaging data between two runs, in accordance with some embodiments. Instead of showing the difference in a single plot like the graphical representation 830, the graphical representation 900 includes two plots, one for each run. In the plot 910, a representative epoch 912 that displays the first peak, the second peak, and the third peak is shown. The representative epoch 912 may be the average of epochs in the first run. A first graphical element, which is in the form of a thick line 914 is shown within the region of B peak. The first graphical element 914 corresponds to a parameter that measures the time difference between B peak onset and B peak latency. If the average of the parameter among the epochs in the first run is outside a threshold range that may be determined based on samples of normal subjects, the thick line 914 is present to show that the parameter is outside the norm. In the plot 910, a second graphical element 916 takes the form of shading. The graphical element 916 is used to represent the parameter of the variability of B peak. In the second plot 920, a representative epoch 922 that displays the first peak, the second peak, and the third peak is shown. The representative epoch 922 may be the average of epochs in the second run.

While in FIG. 8 and FIG 9, the changes in one or more parameters between two runs are shown, in some embodiment, a graphical representation may include a series of plots that show the changes in one or more parameters in a series of runs throughout treatment of a cognitive impairment condition. In some embodiments, the parameter shown across the series of plots remains the same. In other embodiments, different plots and different graphical elements are shown in the series of plots to illustrate the changes of a variety of parameters. Hence, in such a series of plots, the parameters shown in different plots may be different. The selection of the parameters to be shown may depend on whether a particular parameter has a significant change between two runs. In some cases, a computer may store data of various parameters and allow a medical practitioner to select which parameter she wants to display in a graphical user interface.

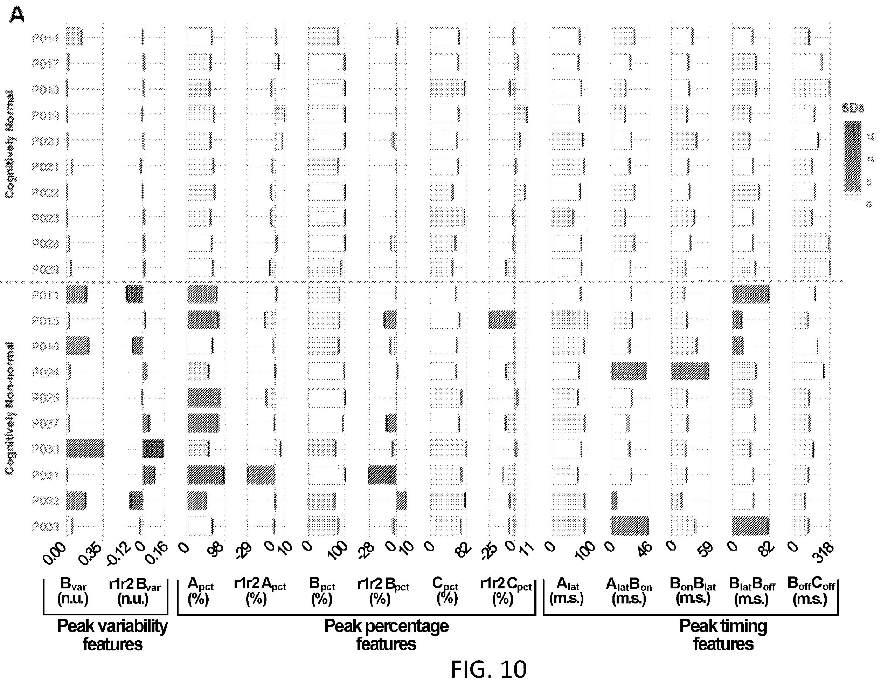

FIG. 10 is a graphical representation of neuroimaging data of multiple subjects and a number of parameters, in accordance with some embodiments. FIG. 10 includes bar plots showing subjects and parameters. Each column shows the subject values for that feature and the bar color indicates the outlier Grubb score (number of standard deviations from normal subjects). Subjects are presented in alphabetical order within a group, and features are organized based on the information they carry. The value of a parameter that is beyond a certain number of standard deviations is colored. The color becomes darker as the number of standard deviations increases. The graphical representation shows that subjects with cognitive impairment conditions can have a large number of parameters being out of the normal range. The graphical representation also shows that certain parameters can be good predictors of whether a subject is cognitively impaired.

Vast clinical experience with the EKG in the analysis of human heart function shows that the examination of timing and fidelity of individual depolarizations is useful in establishing normative (N) versus non-normative (NN) heart electrical function. We applied similar metrics and their correlates to brain function in an elderly cohort of N and NN using magnetocephalography (MEG), a clinically-established neurophysiologic technique that offers superior time and spatial resolution to comparable modalities.

### IV. HEATMAP GENERATION

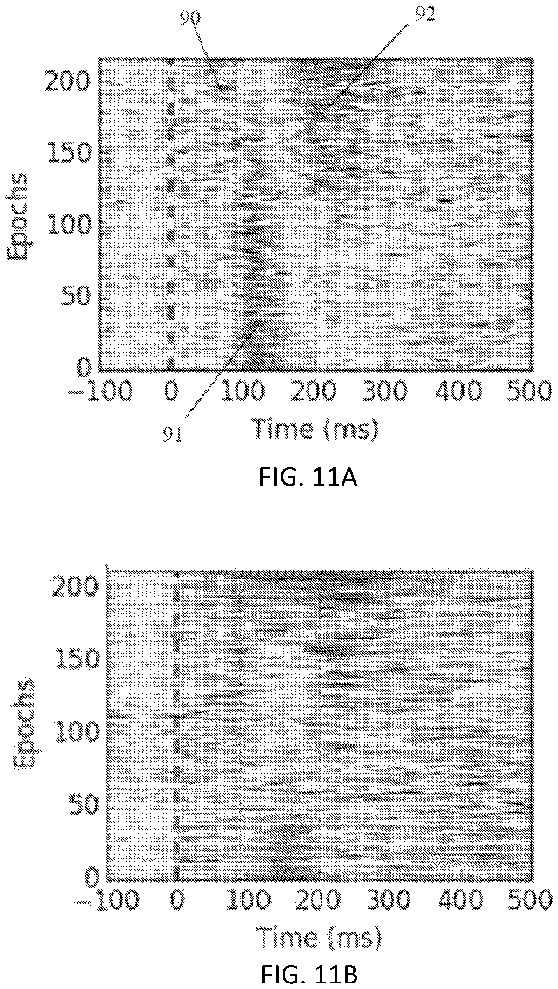

FIG. 11A through FIG. 11D illustrates several example heatmaps using MEG data, with epochs on the y-axis and time with respect to the stimulus time on the x-axis. Although the heatmap examples are illustrated with MEG data, similar processes may be applied to EEG data to generate similar heatmaps, and vice versa.

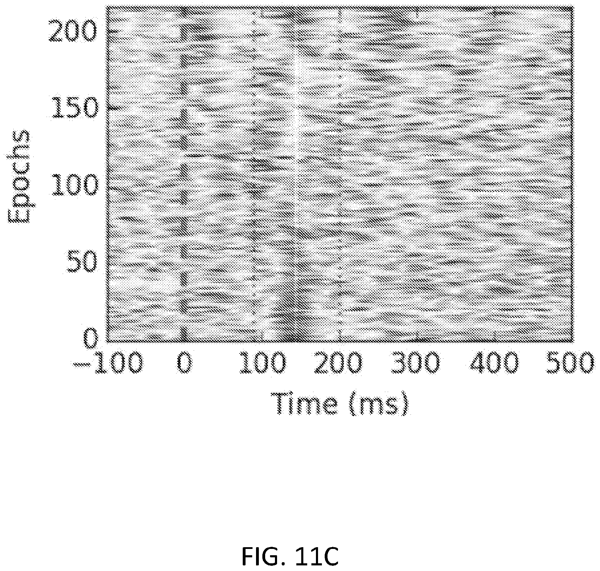

Each heatmap represents one complete auditory stimulation test run for one patient. Each epoch represents a response to a single stimulus. In these heatmaps, white refers to a neutral (close to baseline) magnetic or electrical field as measured by one of the sensors. For each epoch, the color scale is normalized from blue to red based on the data in the epoch. The relative intensity of the positive or negative field is indicated by the intensity of the red or blue color, respectively. The epochs in the heatmaps of FIG. 11A, FIG. 11B, and FIG. 11C are not ordered chronologically but rather by a similarity metric of the signal within the window of peak B 91. Any one of a number of different sorting metrics may be used. For example, the epochs in the heatmap may be sorted based on the duration of one of the three peaks 90, 91, 92, the maximum of one of the three peaks 90, 91, 92, or the latency of one of the three peaks 90, 91, 92. After the sorting of all epochs is done, for visual representation the highest peak B 91 is placed at the bottom in FIG. 11A through FIG. 11C.

FIG. 11A shows a heatmap of the MEG data from a normal patient. Peak B 91, represented in blue between about 90 and 200 msec, has a uniform, well-defined onset and leads to a strong peak C 92, represented in red and appearing after peak B 91. In contrast, FIG. 11B shows the MEG data for an AD patient having a peak B 91 with a less-uniform, less-defined onset. In this case, the peak B 91 is not particularly strong, and although the peak C 92 is not very uniform or well-defined, it is still clearly present. Not all AD patient MEG data, however, showed this same type of deviation. The MEG data (not shown) from one AD patient shows a stronger peak B 91 with a less-uniform, less-defined onset and a peak C 92 that is barely noticeable. MEG data (not shown) for two other AD patients shows a much stronger peak A 90 than for the MEG data of the normal patient shown in FIG. 11A. The onset of the peak B 91 was fairly uniform and well-defined for those AD patients but was delayed in comparison to peak B 91 of the normal patient, and peak C 92 was visible but weak. Finally, FIG. 11C shows MEG data for another normal patient, but the data is very atypical in comparison to the observed MEG data of the other normal patients. Peak A 90, peak B 91, and peak C 92 are fairly weak and poorly-defined in the MEG data in FIG. 11C, with peak B 91 starting later and ending earlier than for other normal patients. Collectively, these heatmaps illustrate that reliance on averaged or otherwise aggregated epoch data alone obscures the variety in stimulus responses that will occur in actual patients, and thus is likely to alone be insufficient to generate a model for discriminating between normal and AD patients.

At least some of the candidate parameters for the CI model were identified or are more easily explained by looking at the non-averaged epochs of MEG data organized in heatmaps. Some of these candidate parameters include a percentage of epochs having a particular peak or combination of peaks.

Additional candidate parameters include identified subsets of epochs in a given set of scans from a single session for a given SQUID sensor. Specifically, two (or more) subsets may be identified for a given test patient dividing the epochs based on any one of the candidate parameters or some other aspects. For example, two subsets may be identified, based on a candidate parameter such as the presence of one of the peaks where presence is a relative measure of magnetic field strength relative to the other epochs for that test patient. In this example, the subset with the peak being present may be divided into two further subsets of a "stronger" subset including some threshold proportion of the epochs (e.g., 50%) with the higher (or stronger, or strongest) relative presence of the peak, and also of a "weaker" subset including the remaining proportion of the epochs with the lower (or weaker, or weakest) relative presence of peak (or absence thereof). Other candidate parameters or aspects of the epoch data may also be used to generate subsets, such as strong and weak subsets, including, for example, peak timing and variability, and peak amplitude and variability.

Yet additional candidate parameters may be determined based on those identified subsets. For example, any given candidate parameter mentioned in Section VII may be determined with respect to an identified subset of epochs. For example, if a strong peak A 90 subset is identified, which may represent 50% of the epochs in the set of scans from a single session of a patient having the strongest relative presence of peak A 90 compared to a weak peak A 90 subset, another candidate parameter may be the mean or median amplitude (in terms of magnetic field strength) of the peak B 91 in the strong subset. One of skill in the art will appreciate the wide variety of possible candidate parameters that may possibly be generated by dividing the epoch data from the set of scans from a single session of a patient and sensor according to one aspect/candidate parameter, and then calculating another candidate parameter based on an identified subset.

Due to the variation across epochs, valuable additional information may be obtained in heatmaps. Visualizing this MEG data in the form of a heatmap allows visual inspection of the set of raw epoch data to identify trends and parameters that are hidden or lost in averaged or otherwise collapsed or conflated MEG data. In such a heatmap, each of the responses, or epochs, is plotted as a horizontal line with a color scale representing the strength of the measured magnetic field. These heatmaps allow visual interpretation of the set of raw epoch data that the computer processes in generating and using the CI model. Although for convenience some of the following descriptions of the generation and use of the CI model are described with respect to calculations that may be performed with respect to and on the data in these heatmaps, those of skill in the art will appreciate that in practice the computer 20 performs calculations with respect to the data itself, without regard to how it would be visualized in a heatmap.

Many candidate parameters were identified by observation of an apparent correlation between the candidate parameter and the Mini-Mental State Examination ("MMSE") score of the test patient. The apparent correlations were mostly initially identified by visual inspection of the heatmaps of model MEG data. For example, it was observed that the CI test patients (i.e., test patients with lower MMSE scores) tended to have more epochs with peak A 90 than normal test patients 50. It was also observed that normal test patients (i.e., with higher MMSE scores) tended to have more epochs with all three peaks. The weaker peak A 90 half of the epochs that have peak A 90 were observed to have a higher amplitude of peak B 91 in normal test patients than CI test patients. Finally, the number of epochs with peak C 92 in the weaker peak A 90 half of the epochs that have peak A 90 were observed to be within an intermediate range for normal test patients.

### V. FEATURE SELECTION AND SORTING

In some embodiments, the epochs of neuroimaging data are ordered and displayed as a two-dimensional heatmap with the positive and negative values being indicated by different colors and relative amplitude being indicated by color intensity. In some embodiments, a computer directs the ordering and display of the epochs of neuroimaging data. In some embodiments, the generated heatmap is displayed on an electronic screen. In some embodiments, the electronic screen is a computer screen of a computer monitor. The epochs of neuroimaging data may be ordered in any of a number of different protocols, depending on the desired parameters to be acquired. In some embodiments, the epochs are ordered based on the timing of maximum intensity of response (latency) for each of the major brain wave response peaks, the A peak, the B peak, and the C peak.

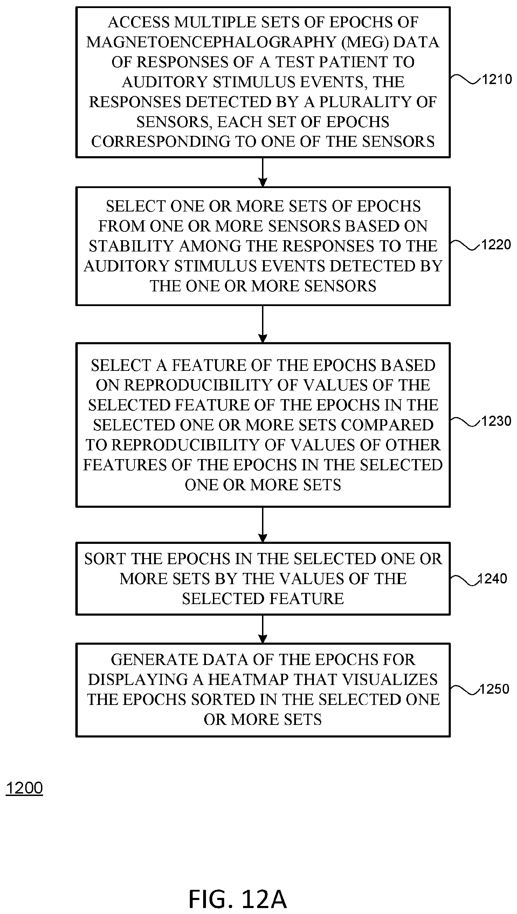

FIG. 12A is a flow diagram illustrating an example process 1200 of collection of neuroimaging data and processing data, according to an embodiment. The process 1200 may be a computer-implemented process. A computer may be a single operation unit in a conventional sense (e.g., a single personal computer), a virtual machine, or a set of computing devices that cooperate to execute the code instructions in a centralized or a distributed manner. For example, a computer may include a set of computing devices in a server room or computing nodes that communicate with a network in a distributed manner (e.g., cloud computing). A computer may include one or more processors and memory that store computer code including executable instructions. The instructions, when executed, cause the one or more processors to perform various processes described herein.

In one embodiment, a computer accesses 1210 multiple sets of epochs of neuroimaging data of responses of a test patient to auditory stimulus events. The test patient may participate in one or more auditory stimulation test sessions that are performed in one or more days across one or more clinical visits. In one example, two of the auditory stimulation test sessions are performed on the same day during a first visit and an additional auditory stimulation test session is performed on another day during a second visit that are days or weeks apart from the first visit. During each auditory stimulation test session, the test patient may be stimulated repeatedly under an activation procedure described in FIGS. 1 and 2. A sensor head that carries multiple sensors distributed at different locations around the test patient's brain may be used to detect the responses of the test patient. An example distribution of sensors is illustrated in FIG. 4A. Each sensor detects the response of the test patient at a specific location and generates a set of neuroimaging data of responses. The sensor signals are captured and may be converted to data of a suitable format such as digital data for storage. Each set of epochs of neuroimaging data corresponds to one of the sensors. For example, a set of epochs may include data values generated by a sensor in different test sessions. Multiple sets of epochs of neuroimaging data of responses may be transmitted to a data store. In one embodiment, the data may be uploaded to a Cloud data store that can be accessed by the computer.

The computer selects 1220 one or more sets of epochs from one or more sensors based on the stability among the responses to the auditory stimulus events detected by the selected one or more sensors. For example, the computer selects datasets from one or more stable sensors or from the most stable sensor. In some cases, the computer may focus on sensors that are located ipsilateral to the auditory stimulus events because, in some situations, ipsilateral responses to simple sound stimuli have been shown to display significant delays in different peaks of the neural response.

In selecting 1220 one or more sets of epochs that are relatively stable or a set that is the most stable, the computer may start with a pool of candidate sensors. The computer may select the sensor whose epoch data have the least variability across epochs or one or more sensors whose epoch data have low variability across epochs. The determination of variability across epochs may be evaluated based on various suitable statistical methods. For example, the selection 1220 may include a process in which the computer determines, for each of the candidate sensors, values of a metric of sensor stability among the epochs in the set corresponding to the candidate sensor. The metric of sensor stability may be defined in any suitable manner and, in some cases, may be specific to each epoch. In other words, in some cases, each epoch may include its value of the metric of sensor stability. For example, the metric may be defined as a range, the maximum value from a baseline reference epoch, a delay, or any model parameter described in this disclosure. The metric value may be specific to each epoch or may be calculated based on an average of a number of epochs. For a set of epochs corresponding to a candidate sensor, the computer determines a variance metric that is calculated from the values of the metric of sensor stability. The variance metric may be the statistical variance, standard deviation, or another suitable statistical metric. The computer repeats the determination of the variance metric for each of the candidate sensors. The computer selects one or more candidate sensors based on the variance metric corresponding to each of the selected candidate sensors. For example, the computer may select the most stable sensor or a few more stable sensors that are associated with a low variance. The selected one or more sets of epochs are corresponding to the one or more selected candidate sensors.

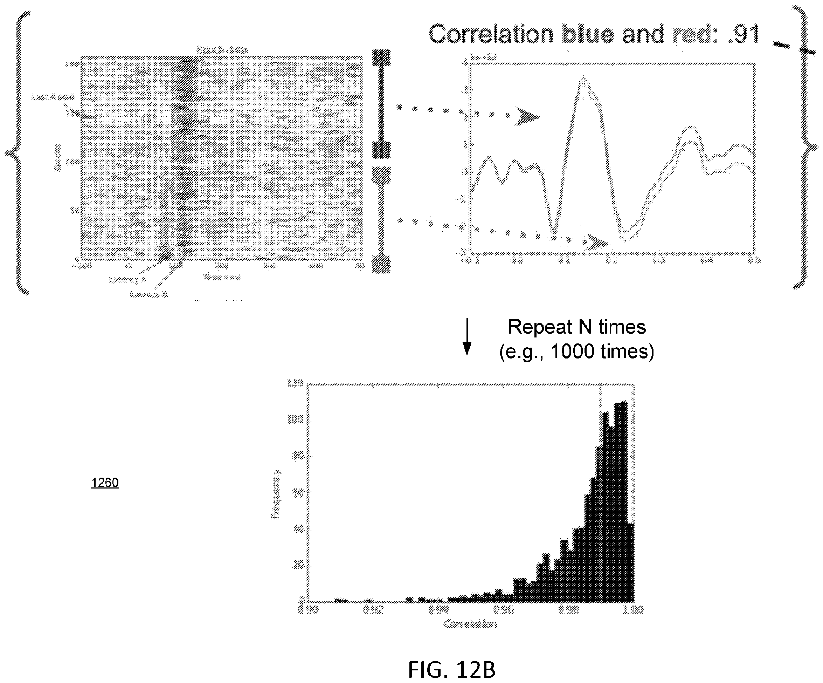

In one example of the selection process 1220, the computer uses an iteration process 1260 to select the stable sets of epochs. This example process 1260 is graphically illustrated in FIG. 12B. From a set of candidate sensors, the computer calculates two evoked responses (e.g., response averaged over epochs) after randomly splitting the epochs in a set into two subsets. The computer calculates the correlation between the two evoked responses. The computer may repeat this process many times (e.g., 1000 times) and sensor stability may be computed as the median correlation over all iterations. This sensor selection process may be referred to as stimulus response variability.

In other words, for each of the candidate sensors, the computer separates the set of epochs corresponding to the candidate sensor into two or more subsets. The computer averages the epochs in each of the two or more subsets to generate two or more averaged epochs. The computer determines a metric of sensor stability corresponding to a correlation among the two or more averaged epochs. The computer repeats the above step multiple times (e.g., 1000 times) to generate a plurality of values of the metric of sensor stability. The computer determines the statistical value (e.g., medium) of the plurality of values of the metric of sensor stability. The computer selects the most stable candidate sensor or one or more stable candidate sensors based on the statistical values corresponding to each of the selected candidate sensors. The sets of epochs that are selected 1220 correspond to the stable candidate sensors.

Continuing to refer to the process 1200 shown in FIG. 12A, the computer selects 1230 a feature of the epochs in the one or more sets selected in 1220. The selection 1230 may be based on stability such as reproducibility of values of the selected feature of the epochs in the selected one or more sets compared to the stability of values of other features of the epochs in the selected one or more sets. A feature may be selected from any model parameters that are discussed above in this disclosure. Reproducibility may be a special type of stability that evaluates a feature's values among epochs that are detected across different testing sessions. For example, in determining reproducibility, the computer may compare the epochs generated in different sessions of the same visit or across different visits that occurred on separate days to determine whether the epochs across different sessions show similar patterns.

The computer selects 1230 a feature that has high stability such as a high reproducibility. In one embodiment, the selection of a feature may be a two-step process that includes a first round of selection of relative stable features and a second round of selection to narrow the final result to a single feature. In various embodiments, one or more steps of the two-step process may be skipped, or additional steps may be added.

In the first round of selection 1230, the computer may narrow down a subset of features that are relatively stable or reproducible across visits. In one embodiment, feature stability may be defined as the Pearson correlation between the feature measured across days. For example, for each candidate feature, the computer constructs a first vector using a number of metrics (e.g., 20 metrics) for the candidate feature of a group of participants based on data obtained from a first visit. The metric may be any measures, such as statistical measures, of the feature, such as average, median, mode, range, variance, etc. of one or more participants in the group. The computer constructs a second vector using the same metrics for the same candidate feature of the group of participants based on data obtained from a second visit. The computer measures the correlation between two vectors that represent two different visits. The computer repeats the construction of vectors and the measurement of correlations for other candidate features. Relatively stable candidate features are selected for the second round. For example, features that have a significant correlation between the two vectors (p < .05, corrected using False Discovery Rate at q < .05) may be selected.

In other words, the first round of selection may include dividing the one or more sets of epochs selected in step 1220 into two or more subsets of epochs. Each subset corresponds to the responses generated in a different visit of the test patient. The computer generates, for each candidate feature, two or more metric vectors. Each metric vector includes one or more metric values of the candidate feature measured from a group of participants that includes the test patient. Each metric vector corresponds to each subset of epochs that are generated in a different visit of those participants. For each candidate feature, the computer determines a correlation between the two or more metric vectors. The computer repeats the correlation determination for different candidate features. The computer selects one or more candidate features whose correlation among the two or more metric vectors is above a threshold. The ultimately selected feature may be selected from this pool of relatively stable candidate features.

In a second round of selection 1230, a feature may be selected using one or more criteria that will each be discussed in further detail below. The criteria may include how well the ultimately selected feature distinguishes between normal volunteers and cognitively-impaired individuals through a machine learning model such as a decision-tree classifier. Another criterion may be how many cognitively-impaired individuals are outside the normal volunteer range. Yet another criterion may be how many cognitive tests with which the feature is significantly correlated.

For each of the criteria above, the computer may establish an acceptable threshold by conducting nonparametric permutation tests. The computer stores the best possible outcome when running the approach using data shuffled among participants. For example, taking into consideration of the first criterion that involves the use of a machine learning model, by using permutation tests, the computer may find that it is extremely unlikely (p<0.05) that one of the candidate features would perform a classification between normal volunteers and cognitively-impaired individuals with more than 70% accuracy when using shuffled data. Therefore, the computer may conclude that the candidate feature performs better than 70% in that criterion. Candidate features performed better than a threshold determined in one or more of the criteria may be kept for final selection.

To elaborate, the permutation tests include shuffling data across participants. For example, the computer may test how well a candidate feature pctA can distinguish between normal volunteers and cognitively-impaired individuals. The computer may set a threshold of accuracy at a certain level (e.g., 85%). The computer shuffles the data across all participants so that there is no relationship between a participant's number for pctA and their diagnosis. The computer tries the criteria again. The computer should get the result from shuffled data close to 50%, as there is no relationship between data and labels if the number of normal volunteers and cognitively-impaired individuals in the participant pool is close to 50-50. The computer may continue this shuffling routine many times to come up with a null distribution. The computer computes a number of how often the computer can find the true value (e.g., set at a threshold of 85%) when there is no real relationship between data and labels.

The framework of the permutation test may be expanded for candidate features being considered at the same time. For example, if there are 10 candidate features, the chances that one of them would get above the threshold level (e.g., 85%) just by accident would be higher. For even more candidate features such as 1000 features, even with shuffled data, the chance of locating one or more apparently stable features by accident would still be higher. Thus, the computer may correct for all those tests at the same time (i.e. the number of candidate features that are being considered at the same time). The computer shuffles the data for all candidate features at the same time, and observes that it was unlikely (less than 5% probability) that any of the stable features would go above 70% accuracy just by chance. Then, the threshold may be set at 70% or a similar number.

The permutation tests in the second round of selection 1230 may be repeated for one or more criteria in order to select a final feature that passes each permutation test for each criterion. The first criterion may be how well the feature distinguishes between normal volunteers and cognitively-impaired individuals through a machine learning model. The machine learning model may be a decision tree classifier, a support vector machine, a neural network, etc. Training and the execution of the machine learning model are discussed with reference to Section VIII. For a candidate feature, the computer inputs the data of the candidate feature into the machine learning model. The computer uses the machine learning model to select the feature. The machine learning model outputs a determination of whether a participant is cognitively impaired. The output of the machine learning model may be compared to the actual label of the participant (whether the participant is known to be cognitively impaired) to determine how well the feature performs. The determination using the machine learning model may be repeated for shuffled data (e.g., shuffling the participant's label on whether he/she is cognitively impaired) in a permutation test.

The second criterion may be how many cognitively-impaired individuals are outside the normal volunteer range. For each candidate feature, the computer determines a range of values of the candidate feature among normal volunteers. The computer determines, for the candidate feature, the number of cognitively-impaired individuals whose values of the candidate feature are outside the range of the values among normal volunteers. The computer selects the feature based on the number of cognitively-impaired individuals whose values of the selected feature are outside the range of values among normal volunteers. The second criterion can also be used as another round of permutation test. Based on the range of values among normal volunteers, shuffled data of the values may be compared to the range to determine a participant with the shuffled data is cognitively impaired. The determination is compared to the actual label of the participant. This can be repeated for many participants to generate a null distribution.

The third criterion may be how many cognitive tests with which the feature is significantly correlated. For each candidate feature, the computer determines a correlation of the candidate feature with a set of cognitive tests. This may include using one or more different cognitive tests (e.g., 20 cognitive tests). The computer may select a feature based on the correlations of the candidate features with the set of cognitive tests. The third criterion may also be used in an additional round of permutation test by using the correlations as prediction criteria of whether participants with shuffled data are cognitively impaired.

The various sub-processes discussed above with reference to the selection process 1230 may be used together or separately to select a feature. In various embodiments, one or more sub-processes may be skipped and additional suitable sub-processes or selection criteria that are not explicitly discussed may also be added.

Continuing with the process 1200 shown in FIG. 12A, the computer sorts 1240 the epochs in the one or more sets selected in step 1220 by the values of the feature selected in step 1230. For example, each epoch may include peak A, peak B, and peak C. A set of epochs may be graphically represented as a heatmap as shown in, for example, FIGS. 11A and 11B. The heatmap graphically presents a first color of different scales to represent a positive polarity of the epochs and a second color of different scales to represent a negative polarity of the epochs. The heatmap arranges the epochs in a set in a first axis and displays changes in values of the epochs over time in a second axis. In the first axis, the computer sorts the epochs based on the value of the feature associated with each epoch. The epochs may be sorted by the ascending or descending order of the feature values. For example, the selected feature may be an amplitude of one of the peak A, peak B, or peak C. The epoch can be sorted by the amplitude.

The computer generates 1250 data for displaying a heatmap that visualizes the epochs sorted in the one or more sets selected in step 1220. The data may be in a format that is suitable for graphical visualization. As a result, a heatmap with sorted epochs can be presented at the display device to illustrate the cognitive condition of the test patient. The computer may repeat 1260 step 1230 through step 1250 to select additional stable features and sort the epochs based on the additionally selected features. Additional heatmaps that are sorted by different features can be generated. A feature may also be a compound feature that includes several sub-features, such as the number of B peaks in weak A peaks. The heatmaps may be displayed in a report.

Based on the report, whether the test patient is cognitively impaired is determined 1270. For example, one or more heatmaps with sorted epochs are displayed. A medical professional may rely on the heatmaps to decide whether the test patient is cognitively impaired. In one embodiment, a machine learning model may be trained. The detail of training a machine learning model is discussed with reference to Section VIII. The computer inputs the data of the epochs to a machine learning model. The machine learning model provides an output such as a label or a score that corresponds to the likelihood of the test patient being cognitively impaired.

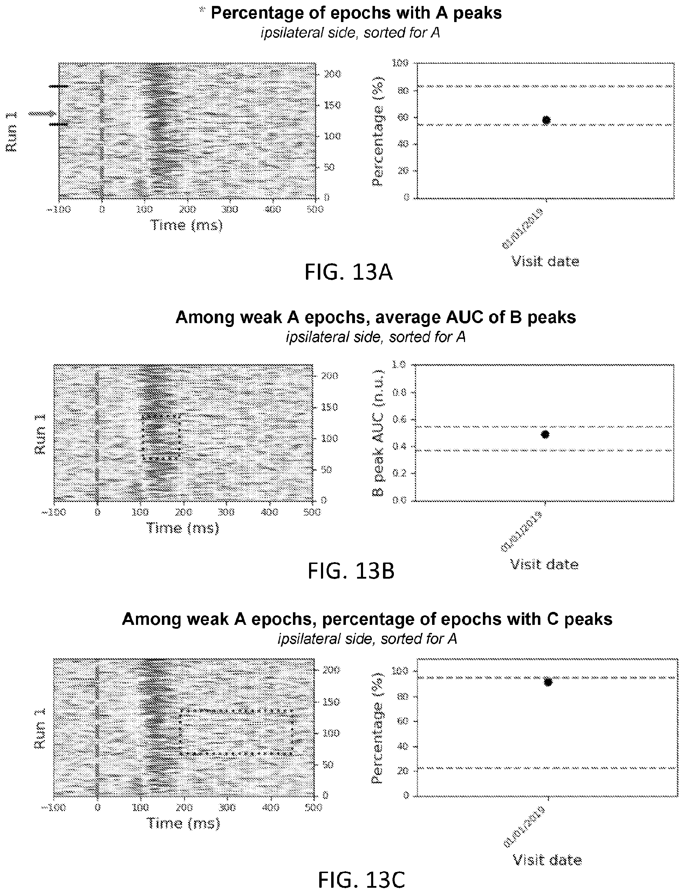

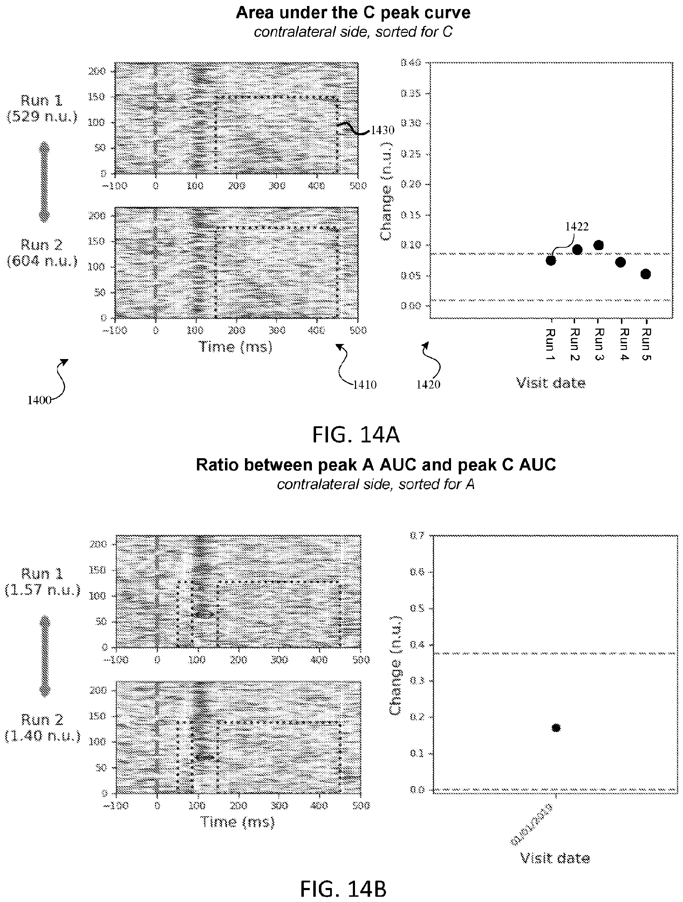

Referring to FIG. 13A through FIG. 14B, a clinical display that may take the form of a GUI may allow an operator to select different versions of heatmaps sorted by a type of peak and also displays a particular feature. For example, a GUI may have one or more buttons that allow an operator to select the sorting option of the epochs (e.g., sorted by peak A, peak B, or peak C), the data source (e.g., whether ipsilateral responses or contralateral responses), and the feature to display. The feature may be a feature that is used in a CI model to generate the cumulative score or may be another relevant feature but is not directly used in the CI model. Referring specifically to FIG. 14A, the GUI 1400 may include a first display area 1410 for displaying a heatmap selected by the operator and a second display area 1420 for displaying a change in the selected feature value across different visits.

In the first display area 1410, the GUI 1400 displays one or more graphical elements 1430 at the heatmap in a location that corresponds to the feature selected. The graphical element 1430 represents an area of the heatmap that corresponds to a measurement for the feature in the heatmap. The feature selected may be related to one type of peak and may represent a measurement (e.g., amplitude, AUC, latency, etc.) of the type of peak. The graphical element 1430 may point to or otherwise emphasize an area in the heatmap that is related to the type of the peak associated with the selected feature and to the measurement. For example, in FIG. 14A, the feature selected is the area under the C peak curve. The graphical element 1430 is a dash lined rectangle that encloses an area in the heatmap that represents the area used to calculate the feature. In FIG. 14B, the feature selected is the ratio between peak A AUC and peak C AUC, the graphical elements may be two dash lined rectangles that respectively enclose the peak A location and the C peak location. For different features selected, different types of graphical elements may be used. For example, in FIGS. 13A, the graphical element is an arrow. In some embodiments, the graphical element is two parallel dashed lines.

In the second display area 1420, the GUI may display a plot of feature values across different runs that generate the epoch data (e.g., each run may correspond to a patient visit that captures MEG data or a patient visit may generate multiple runs). The second display area 1420 may also be referred to as a timeline of values over different runs. The second display area 1420 may include two dashed lines that indicate a normal range of values of the selected feature for NVs. A plurality of points 1422 each indicate the value of the selected feature of a particular run. In one embodiment, the GUI, by default, displays in the first display area 1410 the heatmap of the last run that is plotted at the second display area 1420. An operator of the GUI may select a different point in the second display area 1420 to change the heatmap. The heatmap displayed is generated based on the MEG epoch data that is collected during the particular run selected in the second display area 1420. In one embodiment, the GUI may include a button for selecting more than one run in the second display area 1420. Based on the selection, the GUI displays a plurality of heatmaps in the first display area 1410 to allow users of the GUI to compare heatmaps generated based on MEG data collected at different times.

The heatmaps shown in the GUI 1400 may be sorted by different options. The GUI 1400 may include a button for selecting a sorting of the plurality of epochs by peaks A, peaks B, or peaks C. For some selected features, the location of the graphical element 1430 may change based on the sorting option to represent different aspects of the measurement of the feature under different sorting. The GUI may also include another button for selecting ipsilateral data or contralateral data in displaying the heatmap.

Other sorting examples and GUI examples are discussed in U.S. Patent Application Publication No. 2020/0321124, published on October 8, 2020, entitled "Methods and magnetic imaging devices to inventory human brain cortical function," which is incorporated by reference herein for all purposes.

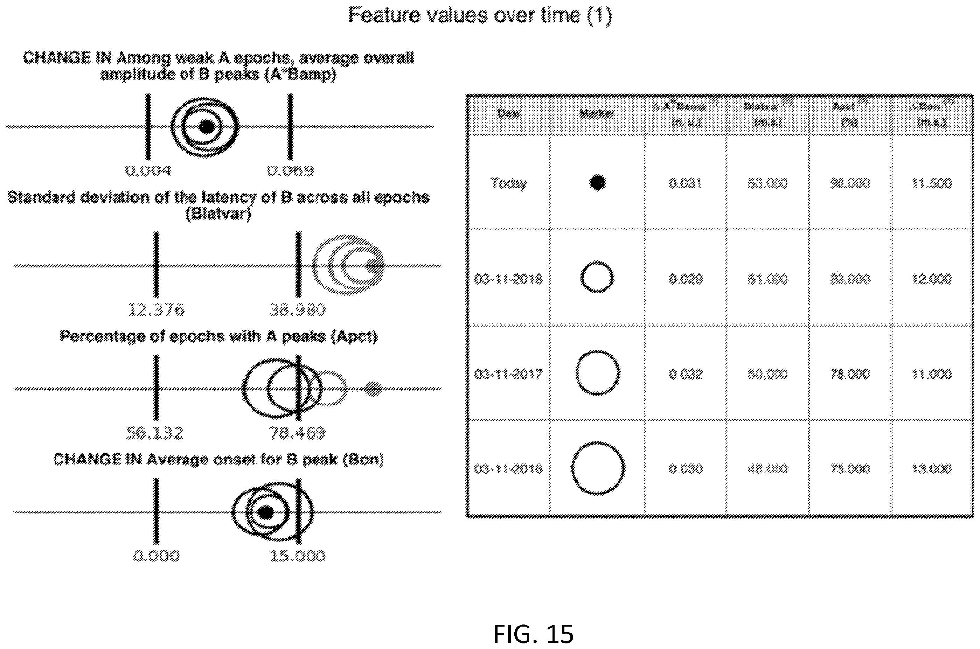

FIG. 15 shows a portion of an exemplary report of results from the analysis of the neuroimaging data of a test subject. The report displays a longitudinal view of feature values, across the many visits for the given patient. The images on the left side display the normal range (vertical bars) of each feature (or feature run-to-run change), and the center of each circle marks the feature value. The greater the circle diameter, the longer it has been since the measurement was taken. The current measurement result is marked with a filled dot.

In exemplary embodiments, a circle becomes red when it is outside the normal range. These plots make it natural to observe the evolution of a specific feature for a test subject over time, whether the value trends towards the abnormal range, or it becomes closer to normal values, such as, for example, as a result of an intervention. Finally, the feature values over time are also shown in the table to the right of the display. For the longitudinal display, individual features are shown in columns, and the multiple measurements over time are the rows.

The top and bottom features on the left of FIG. 15 show stable normal values. The second feature from the top shows a consistently-abnormal value, and the one below displays a significant worsening over time. As noted above, the oldest measurement is represented by the biggest circle, and all other (more recent) measurements are marked by smaller circles with radius decreasing linearly with time, i.e., a circle for a measurement acquired 2 years ago is twice the size of the circle for a measurement from 1 year ago. The current measurement is represented by a filled circle.

### VI. EXAMPLE EVOKED POTENTIAL SUMMARY PLOTS

In some embodiments, a computer may provide a graphical representation of a summary plot of an aggregated epoch of a test patient in the background of a normal range of evoked potential to provide a quick summary on certain features of the test patient that derivate from the normal range.

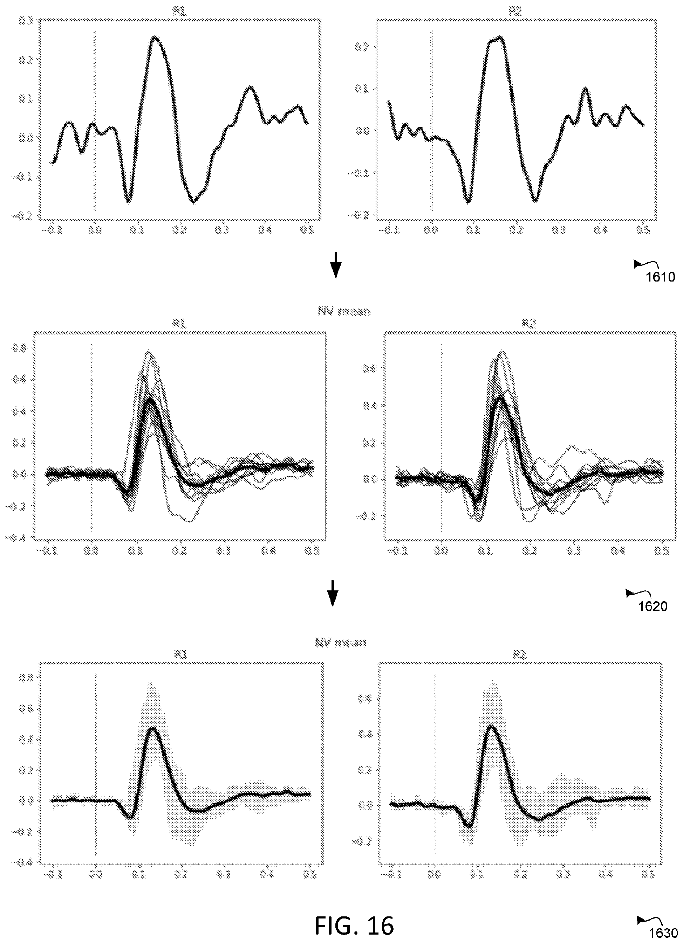

FIG. 16 is a conceptual diagram illustrating a computer-implemented process of generating a background of the normal range of evoked potential of normal volunteers, according to an embodiment. A computer may access datasets of epochs of normal volunteers. For each normal volunteer, the computer may aggregate the epochs to generate an averaged line. The plots 1610 are the aggregated plot of a normal volunteer respectively in two different runs, R1 and R2. The computer may repeat the aggregation process for other normal volunteers to generate multiple aggregated plots for the runs R1 and R2. The plot1620 shows the aggregated plots of multiple normal volunteers. Based on the aggregated plots of multiple normal volunteers, the computer may determine a range of epochs of normal volunteers and turn the range into a grey background, as shown in the plots 1630. In the plots 1630, the middle line in each run shows an average among the normal volunteers. The data of the normal range and the average may be saved by the computer and be retrieved for future use.

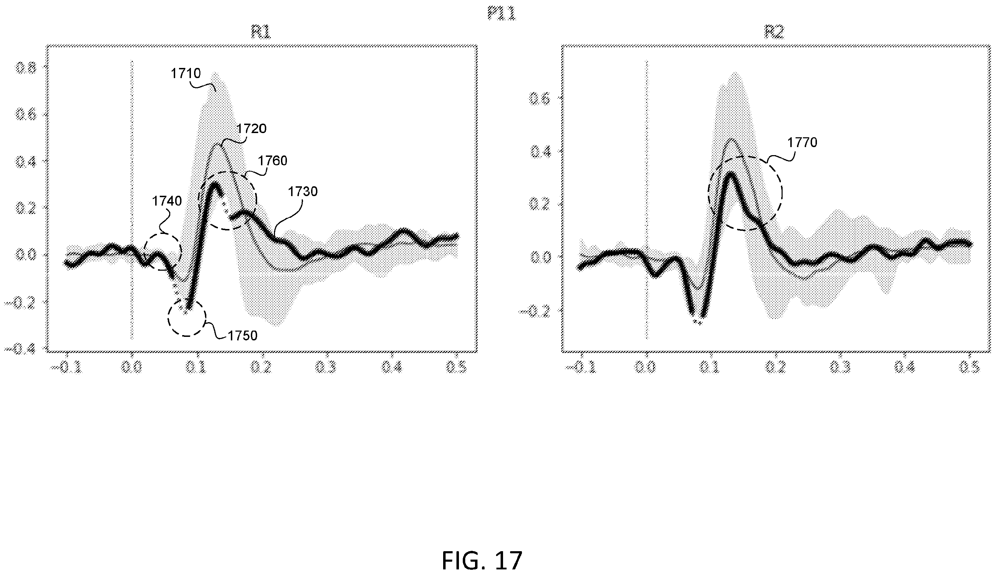

For test patients, a computer may also aggregate the epochs of a test patient and put the aggregated plot onto the grey background that shows the range of normal volunteers. The plot may serve as a summary plot of a test patient. The summary plot may be presented in a graphical user interface as part of the cognitive capacity report of the test patient. FIG. 17 shows two example summary plots of a test patient P11 for the first run and the second run. For the first run R1, the grey area 1710 shows the normal range. The thinner middle line 1720 shows an average plot of normal volunteers. The thicker line 1730 with dotted portions shows the aggregated plot of the test patient P11. The dotted portions indicate the part of the aggregated plot that is out of the normal range.

In one embodiment, the summary plots highlight the features that are out of the normal range so that a computer or a medical professional can make a determination on selecting a feature that can be used to sort the epochs to generate a heatmap. For example, in FIG. 17, the left plot for the first run R1 indicates that one or more features of the test patient P11 may be out of range. Region 1740 indicates that there might be a fixed timing delay for the epochs of the test patient P11. Region 1750 indicates that the feature of A peak amplitudes of the test patient P11 is out of range and the feature of B peak onset variability is also abnormal. Region 1760 shows two peaks at the B peak region, indicating that the test patient P11 might have an abnormally large value of B peak latency variability because the epochs aggregated do not form a single B peak. Likewise, in the region 1770 of the second run R2, the presence of two peaks at the B peak region indicates that the test patient P11 might have an abnormally large value of B peak latency variability. Based on the summary plots, a computer or a medical professional may select a feature for further investigation. For example, the epochs of the test patient P11 may be sorted by the selected feature to generate a heatmap for further evaluation. In one embodiment, the selection of the features and the generation of the heatmaps may be performed automatically by a computer. In another embodiment, a graphical user interface may present the summary plot and allow a user to click on various regions on the plot, such as a region with a dotted line that shows an out-of-range section of the aggregated plot. In response to the selection by the user, the graphical user interface may provide suggestions of features to investigate. Based on a selection of the user, a computer may generate a heatmap and cause the graphical user interface to display the heatmap.

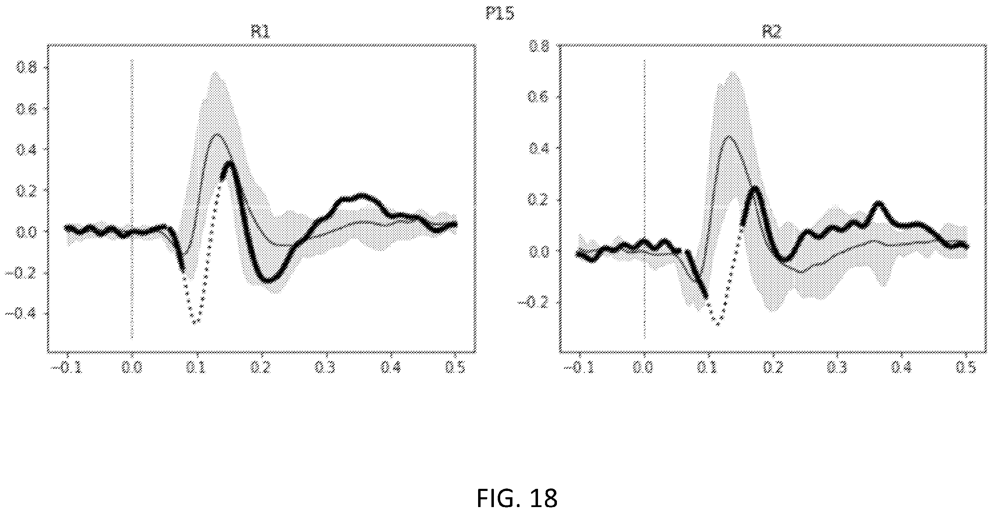

FIG. 18 shows two example summary plots of a test patient P15 for the first run and the second run. The plots show that the amplitude of A peaks of the test patient P15 is abnormally high. Also, the latency value of A peaks and the latency value of B peaks are larger than normal.

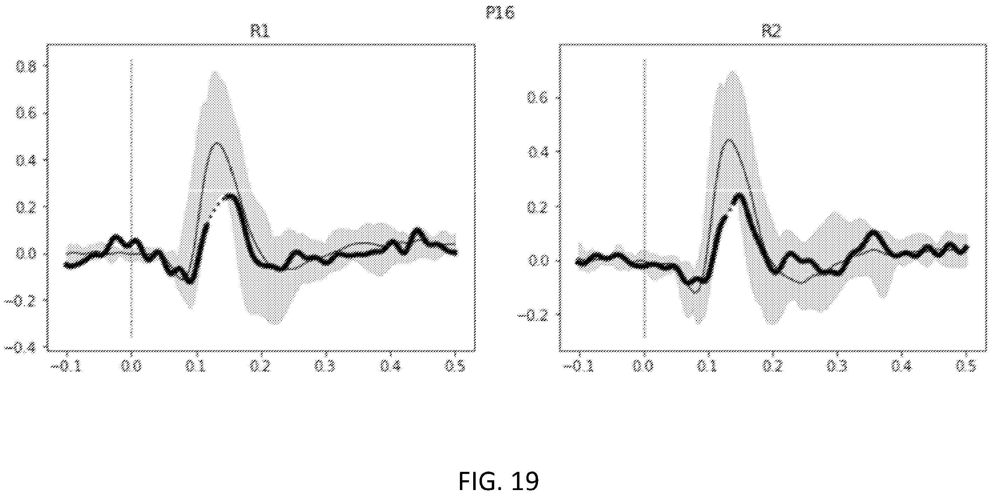

FIG. 19 shows two example summary plots of a test patient P16 for the first run and the second run. The plots show that A peak latency variability may be abnormal so that the A peaks are not aggregated in the summary plots to an easily identifiable peak in each run. The plots also show that the B peak amplitude may be lower than normal and the number of epochs that have B peaks may also be lower than normal so that the aggregated plots show that the amplitude of the B peak is below the normal range. The abnormal features may be confirmed based on heatmaps that are generated by sorting the epochs by the potentially abnormal features.

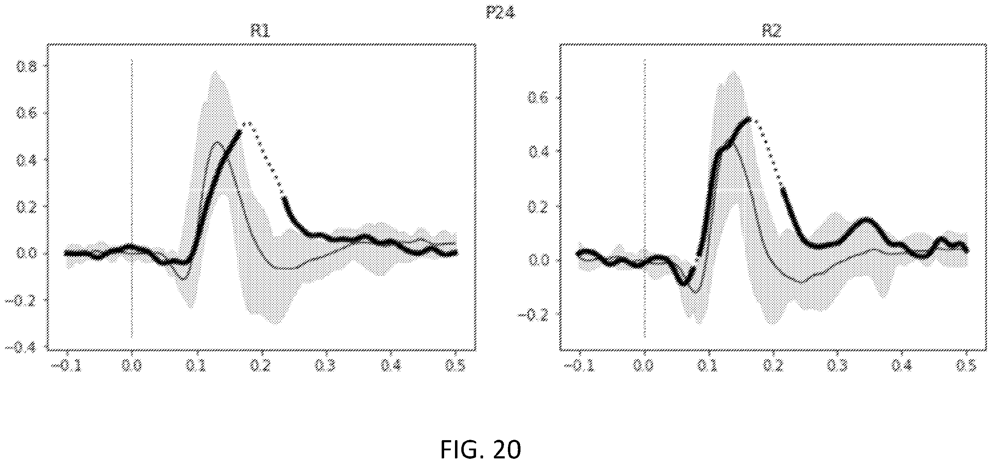

FIG. 20 shows two example summary plots of a test patient P24 for the first run and the second run. For the first run, the C peak in the aggregated plot of the test patient P24 is hardly identifiable. This might be due to the variability of the latency of C peaks in various epochs. The B peaks are also delayed in both runs, indicating that the feature B peak latency might be out of range for the patient P24. The variability of the latency of A peaks may also be larger than normal in the first run R1 so that A peak in the aggregated plot is also hardly identifiable.

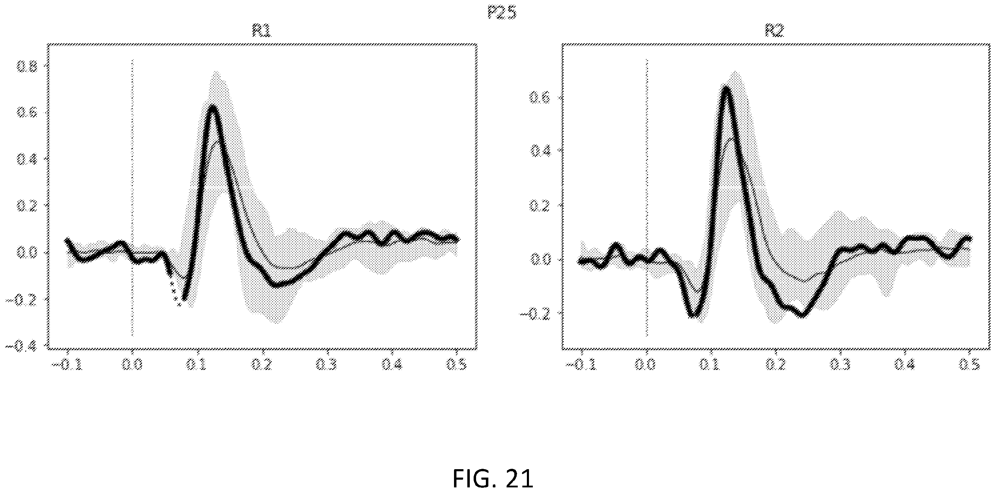

FIG. 21 shows two example summary plots of a test patient P24 for the first run and the second run. Based on the summary plots, the test patient P24 might have a cognitive condition that is closer to normal volunteers because the aggregated plots are mostly within the normal range. The amplitude of the A peak in the first run R1 is slight out of range.

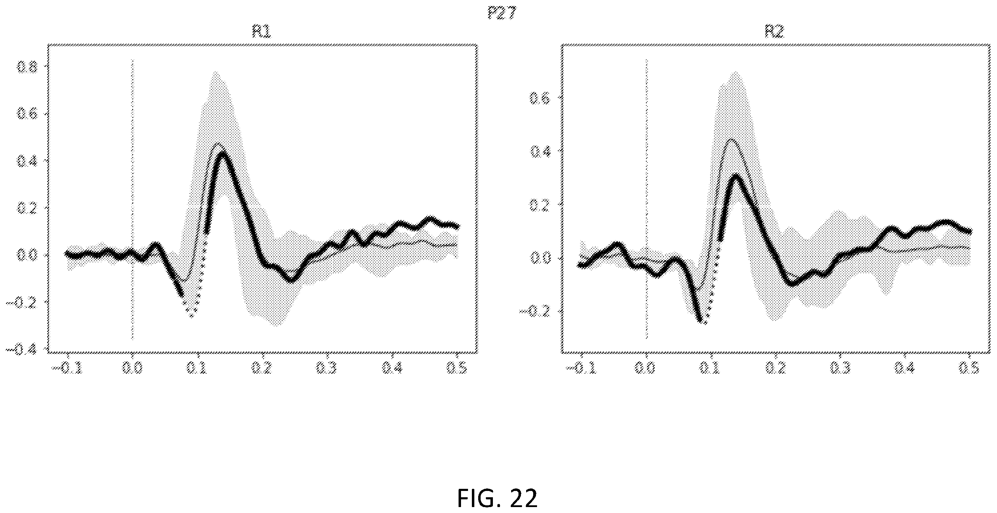

FIG. 22 shows two example summary plots of a test patient P27 for the first run and the second run. Based on the summary plots, the test patient P27 might have a higherthan-normal number of A peaks present in the epochs and the B peak onset may also be delayed, leading to the dotted portion of the plots being out of the normal range. The amplitude of A peaks may also be larger than normal. The precise features that are abnormal may be confirmed by generating the heatmaps that are sorted by the features.

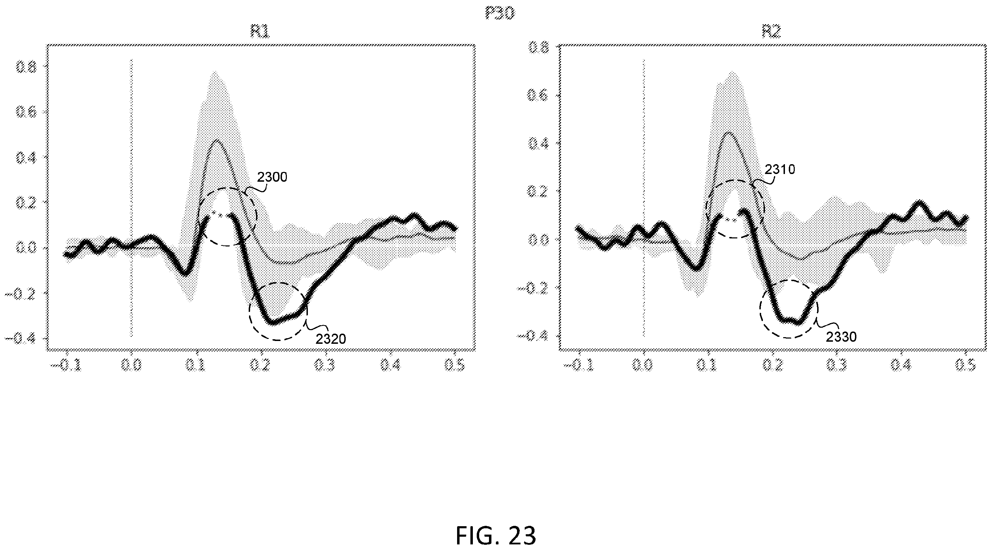

FIG. 23 shows two example summary plots of a test patient P30 for the first run and the second run. The regions 2300 and 2310 show that the B peak region does not form a clear peak. This might indicate that the B peak latency and onset variability are high so that the aggregated plots do not form a clear B peak. The high latency variability of B peak might also be shown by regions 2320 and 2330, which show large negative values in the C peak region because the negative values may indicate that the offset of a large number of B peaks are delayed.

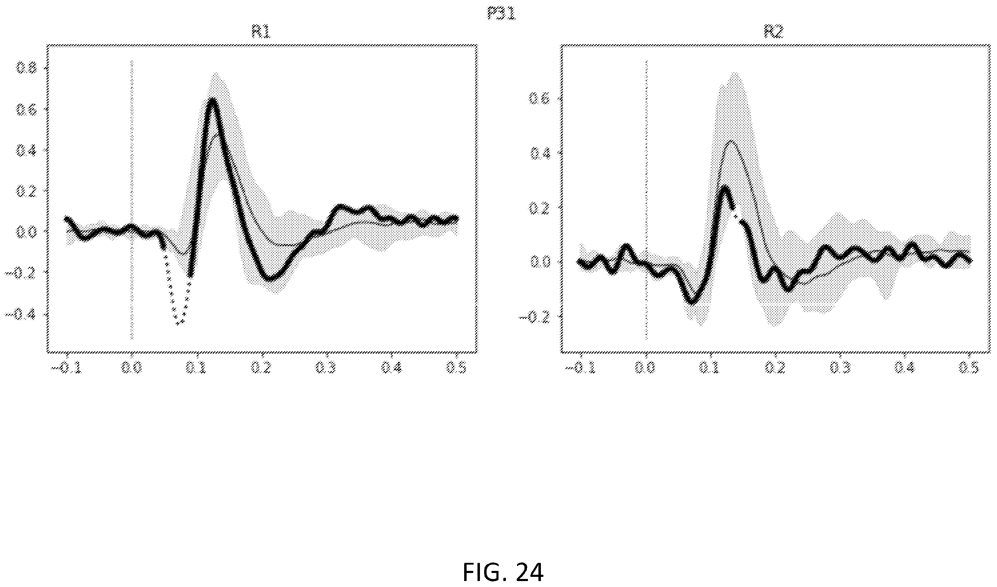

FIG. 24 shows two example summary plots of a test patient P31 for the first run and the second run. The first run R1 may indicate that the test patient P31 has a higher-than-normal number of A peaks and the amplitude of A peak is abnormally high. FIG. 17 through FIG. 23 show that most test patients' features and abnormality are consistent across the first run R1 and second run R2. In contrast, for test patient P31, the aggregated plots in the first run R1 and the second run R2 are quite different, particularly in the amplitude of B peaks. This might indicate that the test patient P31 experienced fatigue in the second run.

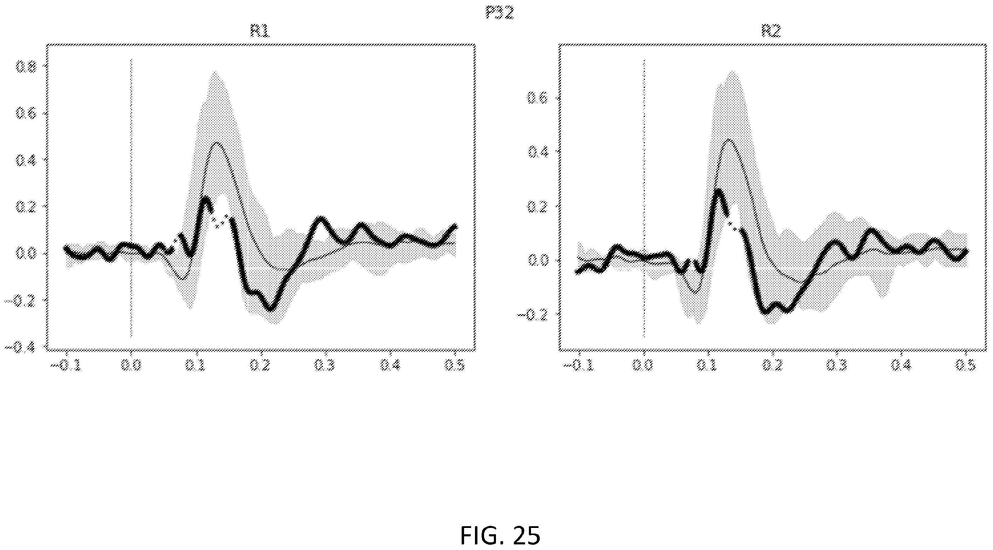

FIG. 25 shows two example summary plots of a test patient P32 for the first run and the second run. Both runs show that the test patient P32 does not have clear A peak, B peak, or C peak. This might indicate that the test patient P32 has abnormally large variability in the latency and onset of A peaks, B peaks, and C peaks.

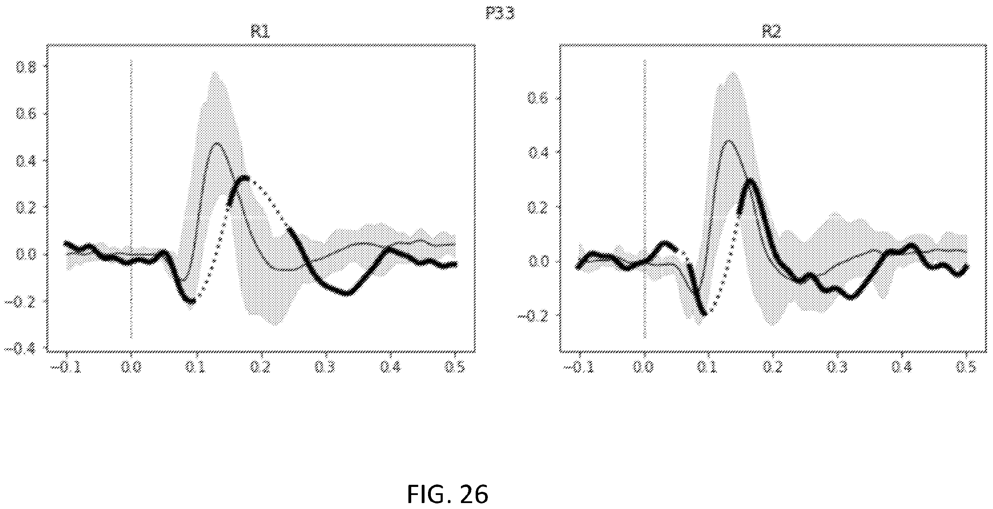

FIG. 26 shows two example summary plots of a test patient P33 for the first run and the second run. The plots show that the onset of B peak is delayed so that the rising of B peak is out of the normal range in both first run R1 and second run R2. The plots also show that the B peaks are consistently delayed so that the test patient P33 has sharp aggregated B peaks in both runs but the aggregated B peaks are delayed compared to the normal range.

A computer may identify the features that are outside the normal range and use the data to determine whether a test patient is cognitively impaired. The computer may train one or more machine learning models to determine whether a test patient is cognitively impaired. The computer may also use the summary plots to lead to further presentations of various heatmaps that are used to determine whether a test patient is cognitively impaired.

### VII.a Parameter (Feature) Identification

There is a great deal of information that can be obtained from the recorded epochs of neuroimaging signal data. On an individual epoch level or after averaging many epochs, the following pieces of information may be determined for use as candidate parameters themselves, or as precursor information towards the determination of other candidate parameters. A computer may determine maximum 80 (or maximum "strength") of peak A 90, the maximum 81 of peak B 91, and the maximum 82 of peak C 92, in either absolute units of magnetic field strength, electrical activity, in some other units, or on a relative scale such as % of largest recorded epoch for that subject or relative to some baseline. The computer may also determine an associated time of occurrence of each peak after stimulation, which are referred to hereafter as latency A, latency B, and latency C, respectively. Latencies may also be computed in other forms, for example the latency of peak B 91 may be calculated relative to the average peak A 90 latency, for that subject or for a population, and so on. A computer may also determine an area under the curve with respect to a baseline, relative to that subject or relative to a population, for peak A 90, peak B 91, and peak C 92. The onset and offset of each peak 90, 91, 92, calculated, for example, as mean (baseline) +/- 2 standard deviations, may also be useful in candidate parameter identification.

There can be various candidate parameters (features). Some of the features are peak latency, which may be length of time between stimulus application and the brain signal achieving its maximum absolute value, and Peak B onset and offset, which may be the time point after stimulus application when the absolute value of the signal became more than twice the standard deviation of the baseline (time < 0), within a 100 to 190 ms window after stimulus application. Another parameter may be the percentage of epochs with one of the three peaks, which may be the percentage of the total number of standard epochs showing any of the 3 peaks. After computing which epochs have each of the 3 peaks, the percentage of epochs with a combination of the peaks captures how many epochs have a combination of 2 or 3 peaks. Area of A and C may be related to looking at the heatmap as a regular image. The area of A and C may be the amount of blue (negative polarity) in the trials detected to contain A and C peaks, respectively. Strong and weak A peaks with B may be the number of B peaks in the first half (strong) epochs with A peaks, and then the second half (weak) of epochs with A peaks. Strong and weak A peaks with C may be similar to the one above, but the number of C peaks in epochs with strong and weak A peaks. Peak B amplitude in strong and weak A epochs may be similar to the one above, but it is based on the average peak B amplitude (e.g., amount of red) in epochs with strong A and also in epochs with weak A peaks. In other words, the amount of red (positive polarity) within the B peak time window, for the first and seconds halves of epochs with A peaks.

### VII.b Candidate Timing Parameters

Some of the candidate parameters may be generally categorized as peak timing parameters, including peak latency parameters, peak onset parameters, peak offset parameters, and peak duration parameters. Each of these candidate parameters may be calculated for each of peak A 90, peak B 91, and peak C 92. For these candidate parameters, the values of the candidate parameters for the CI model are determined based on epochs from test subject training data that are determined to include all three peaks 90, 91, 92, herein referred to as the tri-peak subset. Thus, instead of using all epochs from the scan session of a subject of a sensor to calculate the value of the timing parameter for each peak, it was first determined which epochs had each peak, and then the value for the timing parameter for each peak was calculated. The average and variability of the value of each timing parameter was calculated through bootstrapping, and these averages and variabilities are additional possible CI model candidate parameters. Additional parameters may also include the values of the timing parameters (and their averages and variabilities) as instead calculated from averaged response MEG or EEG data (i.e., the average of all epochs together per sensor per subject).

Each of various peak latency parameters may be estimated in accordance with the length of time between stimulus application and an epoch achieving its maximum (or minimum) absolute value. For example, the latency of peak B 91 may be estimated as a time point in each epoch at which the signal displayed its maximum absolute value. The values of the peak B 91 latency average ["latencyB (mean)"] and variability ["latencyB (var)"] candidate parameters for a particular model subject may be calculated based on the data set of the individual peak B 91 latency points for the epochs under consideration (e.g., those having all three peaks) for that particular model subject in the training set. The resulting candidate parameter values may then be fed into the CI model for training.

The latency of peak A 90 may be estimated based on the time point in each epoch at which the first time derivative of the signal became zero, counting backwards from the latency of peak B 91. The values of the peak A 90 latency average ["latencyA (mean)"] and variability ["latencyA (var)"] candidate parameters may be determined based on the time points for these epochs under consideration for each subject in the training set.

Again, starting at the latency of peak B 91 and going backwards, the onset of peak B 91 may be estimated based on the time point in each epoch at which the absolute value of the signal became more than a predetermined number of the standard deviation (e.g., twice the standard deviation) of the baseline signal (for time < 0). The values of the peak B 91 onset average ["onsetB (mean)"] and variability ["onsetB (var)"] candidate parameters may be determined based on the time points for these epochs under consideration for each subject in the training set.

Similar to the onset of peak B 91, the time point in each epoch for the offset of peak B 91 may be estimated using the same criteria but counting forward from the latency of peak B 91. The values of the peak B 91 offset average ["offsetB (mean)"] and variability ["offsetB (var)"] candidate parameters may be determined based on these time points for the epochs under consideration for each subject in the training set.

Starting at the latency of peak A 90 and going backwards in time, the onset of peak A 90 may be estimated as the time point in each epoch at which the first time derivative of the signal changes sign. The values of the peak A 90 onset average ["onsetA (mean)"] and variability ["onsetA (var)"] candidate parameters may be determined based on these time points for the epochs under consideration for each subject in the training set. Note that the onset of peak B 91, as defined herein, may be the same as the offset of peak A 90. Similarly, the offset of peak B 91, as defined herein, may be the same as the onset of peak C 92.

The offset of peak C 92 was calculated as the first time point in each epoch when the signal returns to the same value as in the offset of peak B 91, or some threshold time (e.g., 450 msec post stimulation), whichever occurs sooner. The value of the peak C 92 offset average ["offsetC (mean)"] and variability ["offsetC (var)"] candidate parameters may be determined based on these time points for the epochs under consideration for each subject in the training set.

The duration of peak B 91 in each epoch is the offset of peak B 91 minus the onset of peak B 91. The values of the peak B 91 duration average ["duration (mean)"] and variability ["duration (var)"] candidate parameters may be determined based on these time points for the epochs under consideration for each subject in the training set.

For each of these timing parameters, a particular process for calculating the value of the candidate parameter is provided above, however those of skill in the art will appreciate alternative mechanisms of calculating these quantities may be established.

### VII.c Candidate Subset Parameters

The determinations of the values of other candidate parameters for the test subjects in the training set involves further processing of the epochs of the neuroimaging data. As above, illustration by heatmap is useful in conceptualizing these candidate parameters. One type of processing includes determining which epochs include one or more of the peaks. This calculation can be used for determining a number of candidate parameters, including those based on strong/weak subsets of epoch.

In one embodiment, to perform this processing and/or identify candidate parameters, the epochs in the heatmap are sorted based on similarity within specific time windows. Often, though not necessarily, the sorting is with respect to a particular "sorting" peak. For example, the epochs may be sorted based on the time window of sorting peak B 91, such that epochs at the bottom of the plot look more similar, and are more likely to have a peak B 91, than epochs at the top. To do the sorting, initial peak boundaries are first estimated using all epochs for a test subject, and those initial estimates are used to sort the heatmap and count the epochs that displayed each peak. In one embodiment, sorting is performed using spectral embedding that transforms the data to a single dimension, after applying a radial basis function ("RBF") kernel with a gamma value such as gamma = 0.1.

After the epochs are sorted based on their similarity within a time window related to peak A 90, peak B 91, or peak C 92, a cutoff epoch for delineating between which epochs are determined to have and to not have the sorting peak is selected that maximizes the correlation of the sorted area within the time window. In one embodiment, an ideal linear signal decay function is used to determine the maximum of the correlation within the time window. For example, assume peak A 90 is the sorting peak and there are a total of 200 epochs. When visually examining the heatmap sorted in the initial guess for peak A 90, only about the bottom 30% of the epochs had peak A 90 in one case. Computationally, to determine the cutoff epoch, a computer may create 200 different images where the signal in the time window for peak A 90 linearly decays from the "bottom" of the heatmap to one of the 200 epochs, and remains zero after it ends its decay. The image that has the highest correlation with the actual heatmap is considered the image where the zero is around the 30% mark.

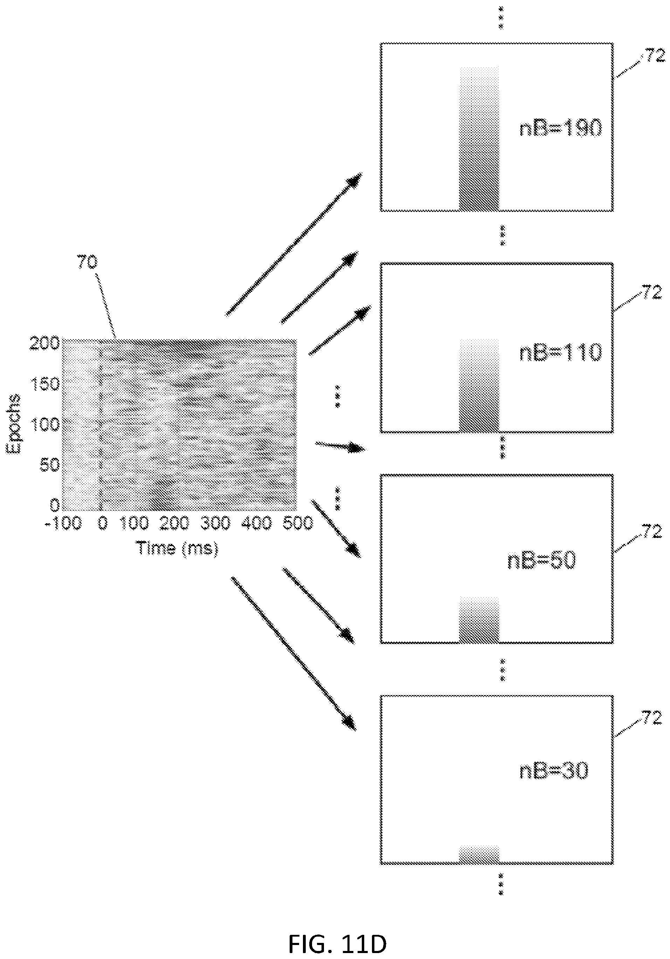

FIG. 11D schematically shows the determination of the nB value for a sample set of scans from a single session. The real heatmap 70 is spatially correlated with every possible ideal heatmap 72 from no epochs having peak B 91 up to all of the epochs having peak B 91. Each epoch is assigned a normalized maximum value based on the maximum value of the strongest peak B 91. For a given sample set, the peak latencies, onsets, and offsets are determined using bootstrapping. Those three timing variables are then used in determining nB (or nA or nC). The sorting of the heatmap is done using only the data within the onset-to-offset time window of the peak being analyzed. After nB (or nA or nC) is determined, all of the epochs from 1 to nB (or nA or nC) are classified as having peak B 91 (or peak A 90 or peak C 92).

The ideal heatmaps 72 for nB=30, nB=50, nB=110, and nB=190 are shown in FIG. 3D for the real heatmap 70 having about 200 epochs. Each ideal heatmap 72 has a linear gradient within the peak B 91 window, where epoch one has a value of one (e.g., dark blue) and epoch nB has a value of zero (e.g., white). The nB value for the ideal heatmap 72 with the highest correlation to the real heatmap 70 is assigned as the nB value for the real heatmap 70. A similar approach is used to assign the values for nA and nC.

Using these approaches, it can be determined which specific epochs have (or lack) each of the three peaks 90, 91, 92, and the number of epochs with each peak can be calculated, as well as how many epochs have every possible combination of the three peaks 90, 91, 92. Said differently, the tri-peak subset of epochs can be determined. Additionally, the values for a number of the candidate parameters for each subject in the training set can be determined, including the candidate parameter regarding the number of epochs with peak A 90 [nA], the candidate parameter regarding the number of epochs with peak B 91 [nB], the candidate parameter regarding the number of epochs with peak C 92 [nC], the candidate parameter regarding the number of epochs with peak A 90 and peak B 91 [A*B], the candidate parameter regarding the number of epochs with peak A 90 and peak C 92 [A*C], the candidate parameter regarding the number of epochs with peak B 91 and peak C 92 [B*C], and the candidate parameter regarding the number of epochs with peak A 90, peak B 91, and peak C 92 [A*B*C]. The values for these candidate parameters may be determined as a number count, or as a fraction of the total number of epochs for that test subject. The candidate parameters may also be determined using percentage of epochs with one or more of the three peaks. For example, the percentage of epochs having peak A [pctA] may be expressed as 73%. The candidate parameters may also be expressed as the percentage of epochs having peaks A and B, having peaks A, B, and C, having peaks A or C, having peak A but not C, etc.

The values of other candidate parameters may also be determined for each test subject 50 in the training set. The values of the area of peak A and C [area A and C], area of peak B [areaB] are respectively the aggregated area under the heatmap (e.g., heatmap shown in FIG. 3A) that is blue (i.e., with positive magnetic field signal) and the aggregated area under the heatmap that is red (i.e., with negative magnetic field signal). The value of an area ratio candidate parameter (e.g., [areaAandC/areaB]) is the ratio of these two numbers.

The values of other candidate parameters may be determined by creating strong and weak subsets, as introduced above. The value of the candidate parameter for the strong peak A 90 epochs containing peak B 91 is based on the number of epochs having a peak B 91 in the strong peak A 90 subset (e.g., half/50% cutoff) of epochs ["strongA_Bnum"]. Similarly the value of the candidate parameter for the weak peak A 90 epochs containing peak B 91 is based on the number of epochs having a peak B 91 in the weak peak A 90 subset ["weakA_Bnum"]. The value of the candidate parameter for the amplitude of peak B 91 in the strong peak A 90 epochs is based on the average amplitude (e.g., amount of red) of peak B 91 in the epochs in the strong peak A 90 ["strongA_Bamp"] subset. The value of the candidate parameter for the amplitude of peak B 91 in the weak peak A 90 epochs are based on the average amplitude (e.g., amount of red) of peak B 91 in the epochs in the weak peak A 90 ["weakA_Bamp"] subset. In other embodiments, these candidate parameters measuring amplitude may be based on another factor other than average, such as median and generally, any measure of amplitude may be used.

Values for other similar candidate parameters may also be calculated for the reverse situation of subsets including peak B 91, with values based on peak A 90 amplitude or number ["strongB_Anum", "weakB_Anum", "strongB_Aamp", "weakB_Aamp"]. Further values for candidate parameters may also be calculated based on any permutation of a given subset of epochs (e.g., strong or weak) containing a peak (e.g., A, B, or C), and some measure of a quantity of the epochs in that subset (e.g., amplitude or count of another one of peak A 90, peak B 91, or peak C 92).

### VII.d Other Candidate Parameters

The feature ratio area under the curve ["rAUC"] is calculated as the ratio of the area under the curve ("AUC") of peak C 92 to the AUC of peak A 90 from the averaged MEG or EEG data. The boundaries of peaks A and C are defined manually for each run, based on when each peak started and finished with respect to the horizontal baseline. Boundaries are straight vertical lines crossing the time chosen for the beginning and end of each peak. The area is then calculated by creating a straight baseline from the starting point of the boundary to the ending point of the boundary and summing the magnitude of the signal with respect to this baseline. Finally, the ratio between the two areas under the curves is calculated. In exemplary experiments, rAUC tended to be greater in normal test subjects than cognitively-impaired test subjects.

For the ratio latency ["rLat"], the latency of each peak from the averaged MEG or EEG data is determined by finding the time of the highest absolute magnitude of the signal within the three sets of pre-determined boundaries. Then, the difference between the latency of peak C 92 and latency of peak B 91 is calculated, and similarly, the difference between latency of peak B 91 and latency of peak A 90. The ratio of these differences is the value for rLat. In exemplary experiments, rLat tended to be lower for the cognitively-impaired test subjects and was particularly low for one such test subject.

After an initial identification of the rAUC and rLat candidate parameters and investigation of their potential as model parameters, a more thorough identification and investigation was performed. As discussed previously, this included not just looking at averaged neuroimaging data from numerous scans but also investigating the distribution of the activation over epochs in the heatmaps of the model neuroimaging data.

Other candidate parameters based on evaluating the heatmaps included ["areaA_ratio"], which is the ratio of the area of peak A 90 in the weak peak A 90 epochs to the area of peak A 90 in the strong peak A 90 epochs; ["Bamp_ratio"], which is the ratio of the overall amplitude of peak B 91 in the stronger half of peak A 90 epochs to the overall amplitude of peak B 91 in the weaker half of peak A 90 epochs (a similar parameter can be determined and used for the C peaks ["Camp_ratio"], and similarly for any permutation of the peaks used to determine the weak and strong subsets, and the peak used to determine the ratio); ["Bnum sA/wA"], which is the ratio of the number of epochs having peak B 91 in the stronger half of peak A 90 epochs to the number of epochs having peak B 91 in the weaker half of peak A 90 epochs; ["Camp_ratio"], which is the ratio of the overall amplitude of peak C 92 in the stronger half of peak A 90 epochs to the overall amplitude of peak C 92 in the weaker half of peak A 90 epochs (a similar parameter can be used for the B peak ["Bamp_ratio"], and similarly for any permutation of the peaks used to determine the weak and strong subsets, and the peak used to determine the ratio); and ["Cnum sA/wA"], which is the ratio of the number of epochs having peak C 92 in the stronger half of peak A 90 epochs to the number of epochs having peak C 92 in the weaker half of peak A 90 epochs. Generally, further permutations of the above parameters are also possible. For example, any parameter including a ratio can also be calculated by inverting the values described above as making up the ratio.

Another candidate parameter, [badInPool], that can be added is a summation of how many candidate parameters in the pool were outside the range for normal test subjects. For example, if the pool includes 17 candidate parameters, the value of [badInPool] is in the range of 0 to 17, depending on how many of the 17 candidate parameters a given CI test subject has a value outside the Gaussian distribution fitted to the normal test subject values. In other words, for each of the 17 candidate parameters, the normal values are gathered and fit to a Gaussian distribution. For each candidate parameter, if the value of the candidate parameter for an CI test subject has a probability of being in that distribution that is smaller than the smallest normal test subject probability, then a value of one is added to the [badInPool] candidate parameter. In other words, the less likely the excluded CI test subject was to be part of the normal distribution, the higher the value of the [badInPool] parameter.

To determine the [badInPool] candidate parameter, a separate calculation is made for each of the candidate parameters already in the CI model. For a given candidate parameter, the neuroimaging data for all normal test subjects according to an alreadydetermined cutoff for that model parameter (based on whether the MEG or EEG data comes from a normal test subject) is fit to a distribution, such as a normal (Gaussian) distribution. That distribution is used to estimate the smallest probability among normal test subjects to be part of the normal test subjects, where that value is used as a cutoff to mark the value of a given parameter as "bad" or not. In a leave-one-out cross-validation framework, the left-out subject is not used when estimating the normal distribution (although if the left-out subject were an AD subject, the value would not be used anyway).

The value of the [badInPool] candidate parameter for each subject is a simple summation of how many other candidate parameters for that test subject had smaller probabilities of being in the distribution for normal test subjects than the smallest normal test subject probability. In an example CI model having six other candidate parameters aside from [badInPool], [badInPool] can go from 0 to 6.

Another possible, similar candidate parameter is [weightInPool], which is a more detailed version of [badInPool]. The weight for [weightInPool] is a summation of the absolute differences between the smallest normal test subject probabilities and that test subject's corresponding probability of being in the distribution for normal test subjects, summed over the set of candidate parameters in the model (other than [badInPool]). [badInPool] and [weightInPool] are both posthoc parameters.

### VII.e Specific Examples of Parameters

In certain embodiments, various exemplary parameters represent different measurements of one or more peaks in the epochs. One example parameter includes area under the curve of peak X, where peak X may be peak A, B, or C. This parameter measures the amount of blue or red signals between onset and offset of peak A, B, or C. Another example parameter includes the percentage of epochs with peaks X. This parameter measures the number of epochs identified to have peaks X as a percentage of the total number of epochs.

Example parameters further include the percentage of epochs with both peaks X and peaks Y, where a peak Y is another peak A, B, or C different from peak X. This parameter measures the number of epochs identified to have both peaks X and Y as a percentage of the total number of epochs. Example parameters further include the percentage of epochs with X or peaks Y. This parameter measures the number of epochs identified to have either X, Y, or both peaks as a percentage of the total number of epochs. Example parameters further include the percentage of epochs with peaks X among epochs with strong peaks A. By way of example, epochs with peaks A may be sorted from strongest to weakest peak A, and the number of epochs with peaks X among the stronger half of the epochs with peaks A is counted. Example parameters further include the percentage of epochs with peaks X among epochs with weak peaks A. By way of example, epochs with peaks A are sorted from strongest to weakest peak A, and the number of epochs with peaks X among the weaker half of the epochs with peaks A is counted.

Example parameters further include the average normalized AUC of peaks X among epochs with weak peaks A. By way of example, epochs with peaks A are sorted from strongest to weakest peak A, and the average amplitude of the peak X is computed among the weaker half of the epochs with peaks A that also have peaks X. Example parameters further include the average normalized AUC of peaks X among epochs with strong peaks A. By way of example, epochs with peaks A are sorted from strongest to weakest peak A, and the average amplitude of the peak X is computed among the stronger half of the epochs with peaks A that also have peaks X.

Example parameters further include the average latency in peak X. This parameter measures the time in which the peak X reaches its maximal absolute amplitude. Example parameters further include the variability in the latency of the peak X. This parameter measures the variability in the time in which the peak X reaches its maximal absolute amplitude. Example parameters further include the average duration of the peak X. This parameter is the average difference between peak X offset and onset. Example parameters further include the variability of the duration of the peak X. This parameter measures the variability of the difference between peak X offset and onset. Example parameters further include the average onset for peak X. This parameter measures the average time in which the peak X surpasses a certain number of standard deviations (e.g., 2 standard deviations) of the baseline signal. Example parameters further include the variability of the onset for peak X. This parameter measures the variability of the time in which the peak X surpasses a certain number of standard deviations (e.g., 2 standard deviations) of the baseline signal. Example parameters further include the standard deviation of the latency of X across all epochs. The time point in which peak X reaches its maximum absolute value is calculated in each epoch. The standard deviation over epochs is reported.

Example parameters further include the average amplitude of the peak X. This parameter measures the average of the maximum absolute value reached by the peak X across epochs. Example parameters further include the variability in the maximum absolute amplitude of the peak X. This parameter measures the variability of the maximum absolute value reached by the peak X across epochs. Example parameters further include the average offset for peak X. This parameter measures the average time in which the peak X returns to a value below a certain number of standard deviations (e.g., 2 standard deviations) of the baseline signal. Example parameters further include the variability of the offset for peak X. This parameter measures the variability of the time in which the peak X returns to a value below a certain number of standard deviations of the baseline signal (e.g., 2 standard deviations). Example parameters further include a change in peak X time shift. This parameter computes how many time points peak X went above a certain number of standard deviation of baseline (e.g., 1 standard deviation), and divides it by the total number of time points between onset and offset (0 to 1, closer to one means less variable). This parameter may serve as a proxy to how "diagonal" the peak is, from the bottom of the heatmap to the top. The more consistent in time across epochs (i.e., the less diagonal), the closer the parameter is to 1.

Example parameters further include peak X amplitude ratio between epochs with strong and weak peaks A. Epochs with peaks A are sorted from strongest to weakest peak A, the average amplitude of the peak X is computed among the epochs that also have peaks X. The ratio of that amplitude between strong and weak A epochs is calculated. Example parameters further include the rate of increase of the peak X. This parameter measures the slope of the line that goes from peak X onset to peak X latency time points. Example parameters further include the rate of decrease of the peak X. This parameter measures the slope of the line that goes from peak X latency to peak X offset time points. Example parameters further include the ratio between peak A AUC in strong over weak peaks A. The amount of blue signal is calculated for weak and strong A epochs, and the ratio is calculated. Example parameters further include the ratio between peak A AUC and peak C AUC. This parameter measures the amount of positive polarity signal in peak A epochs over the amount of positive polarity signal in C peak epochs Example parameters further include the ratio of number of epochs with peaks X between strong and weak peak A epochs. By way of example, epochs are split into weak and strong peaks A, and the number of epochs with peak X in each group is compared against each other.

### VII.f Model Parameter Selection

The candidate parameters were evaluated based on whether they were reproducible within and across test subject visits (each visit generating a set of epochs) for reliability and stability, respectively. Bland-Altman plots may be used to measure those characteristics. In other embodiments, other criteria and methods may be used to evaluate the reliability and stability of candidate parameters, including, but not limited to, intraclass correlation coefficient ("ICC") and regression analysis.

Among the wide variety of possible candidate parameters that may be used to build the CI model, thirty-seven candidate parameters were identified from visual analysis of MEG data to build one implementation of a CI model. The subtle differences between the MEG scans of CI test subjects and "normal" test subjects described above were identified by careful manual visual review and observation and not by a computer algorithm. The 37 candidate parameters, include (as ordered from best to worst in terms of excluding CI test subjects from the distribution for normal test subjects) as weakA_Bamp, strongA_Bnum, nA, weakA_Camp, A*B*C, strongA_Bamp, B*C, areaC, duration (var), Cnum sA/wA, areaA, A*C, weakA_Cnum, nC, areaA_ratios, latencyA (var), onsetA (var), A*B, nB, offsetB (mean), strongA_Cnum, offsetB (var), Bnum sA/wA, Bamp_ratio, areaA/areaC, latencyB (mean), areaA/areaC, latencyB (var), offsetC (var), latencyA (mean), Camp_ratio, onsetA (mean), onsetB (mean), onsetB (var), duration (mean), strongA_Camp, and offsetC (mean).

Some of these candidate parameters were selected for further analysis based on being reliable and stable candidate parameters. Further analysis included determining the correlation between the candidate parameter and the MMSE score of the test subject 50. The selection of which reliable and stable candidate parameters became model parameters was based, at least in part, on the weights the linear and non-linear models assigned to the model parameters.

It is important to note that two subjects with very similar MMSE scores were found to have very different peak C 92 amplitudes, which highlights how these candidate parameters may offer new insights into the disease that were hidden by just looking at MMSE scores.

In some embodiments, a certain number of parameters (e.g., 100 parameters) are generated. In one embodiment, roughly half of the parameters are selected from each side of the head. For example, in some embodiments, 50 of the parameters are contralateral features while other 50 of the parameters are ipsilateral features. To select the parameters, the stability of the parameters across different subject visits are determined for those 100 features. The stability is measured based on correlation. By way of example, for each of the features, a scatter plot may be created among multiple subjects. In the scatter plot, the X axis is the parameter value at the first run of a subject and the Y axis is the parameter value at the second run of the subject. The runs may be generated during the same or different subject visits. Multiple points can be plotted based on the two-run plots of different subjects. The more stable the feature is, the closer to a diagonal line the plot will be. In other words, using techniques such as linear regression, a diagonal line of slope 1 may be fit through a scatter plot using data among different subjects for a stable feature. For the scatter plots, additional dimensions (e.g., additional subject visits or additional runs) may be added if the stability across more than two runs is determined. The most stable features may be selected given a preset threshold (e.g., p < 0.05, false discovery rate q < 0.05). The selection process may reduce the set of features to 35 out of the initial 100. In turn, the within-day variability in absolute value for each of the selected features (e.g., the 35 selected features) may be determined. A total of 70 features are may be selected (e.g., 35 selected features and 35 variability values determined from the selected features) for further analysis such as training and testing of the CI model.

### VIII. Model Training

A CI model may be trained to classify subjects based on their neuroimaging data. A wide variety of machine learning techniques can be used to create the CI model, examples of which include Random Forest Classifiers ("RFC"), Random Classifier Regressors, Gradient Boosting, Support Vectors (also known as Support Vector Machine or "SVM"), Linear SVM, Radial basis function kernel SVM ("RBF SVM"), Linear Regression, Logistic Regression, and other forms of regressions, such as partially least square regression. This list is not exhaustive, and one of skill in the art will appreciate that other machine learning techniques may also be used, including techniques in the field of deep learning such as Neural Networks.

Generally, training these models generates a set of coefficients, which may also be referred to as weights, that represent directly or indirectly how the values for the various model parameters correspond to either a cumulative score that correlates (positively or negatively) with CI or a classification of CI. For example, in one embodiment, the cumulative score may measure a value that is negatively correlated with the chance of a subject having some form of CI. Put differently, the lower the cumulative score, the more likely that the subject having the cumulative score is detected with one or more forms of CI. A set of model test subjects were selected to include a subset having no known cognitive dysfunction and a subset showing a range of severity of symptoms of cognitive dysfunction, specifically cognitive dysfunction associated with CI. However, in practice the principles described herein may also be applicable to a variety of other diseases and conditions, including, but not limited to, mild cognitive disorder. In the case of a CI example model generated using RFC with one-step classification, the coefficients may also be referred to as "critical values", as used in the literature regarding RFC models, in this case for categorizing the values of particular model parameters for a given subject as being normal or CI-indicative.

What the model is trained to detect may vary by implementation. Examples include a two-step classification and a one-step classification. In a two-step classification, a first model is used to predict the cumulative score for a subject, and then a second model is used to categorize or quantify a subject with respect to a particular disease or CI based on the predicted cumulative score. In a one-step classification, a single model categorizes or quantifies a subject with respect to CI directly.

For two step classifications, the first step uses a linear/non-linear model, generally a linear or non-linear regression, although in alternate implementations more complicated algorithms may be used. After the cumulative score has been predicted, the second step includes using a simple cutoff to classify whether the test subject is a normal test subject or an CI test subject. For example, a set of predicted cumulative scores of test subjects is fit to a linear model and one or more weights is determined that correlates the predicted cumulative scores with a categorization.

The CI model may be a static model or a living model. In a static model, the model parameters and their weights are not changed as the model is used to evaluate and assess new subjects. For example, in the RFC example, the normal value limits are calculated by fitting a Gaussian distribution to the set of normal subjects minus whatever subject is left out in the cross validation. In a living model, new neuroimaging data that has been collected from some or all new subjects becomes additional model neuroimaging data used to further train the weights of the candidate parameters or to add, delete, or change candidate parameters and thereby update the model. For a progressive disease, such as AD, the CI model may also be fine-tuned by monitoring the subjects and collecting model neuroimaging data over time and re-evaluating the earlier CI model neuroimaging data, such as if a particular normal test subject begins to show symptoms of the progressive disease, to add, delete, or change candidate parameters and/or retrain the CI model to re-determine the model weights, and thereby update the model.

In some embodiments, both the selection of features for use in training the CI model and the training of the CI model may be conducted through a cross-validation process. For example, in one embodiment, a random set of 5 features out of 70 features pre-selected are used in training the CI model. In an example cross-validation process, a random set of 5 features are selected out of the 70 features. The test subjects are divided into a training set and a testing set. For example, in a collection of 20 test subjects, 19 out of the 20 test subjects may be classified as the training set and the last test subject is held out and used as the testing set. Other combinations of numbers in the training set and testing set are also possible. The testing set is used to train the weights of the CI model for the random set of 5 features for a weighted combination of features to predict the cumulative score. The CI model may be a linear or non-linear model. In one embodiment, the CI model is a linear model. After it is trained, the CI model is used to predict the cumulative score of the testing set and compute the error of the testing set. For example, the error may be computed by determining the difference between the actual cumulative score and the predicted score.

The cross-validation process may be repeated for additional rounds by using different training and testing sets. Other combinations of training and testing sets are repeated to train the CI model and determine the error computed by the CI model. For example, in each round, a different test subject is held out as the testing set and the training is conducted using the rest of the subjects. After the error values for different test subjects are determined, an error metric such as a mean-square error is computed across all rounds. The error metric may represent the mean error of the 5 features selected for the CI model.

In addition to using different training sets, the cross-validation process may be repeated for additional sessions for using different features. In another session of training, a different set of 5 features may be selected and the cross-validation process is repeated to determine the error metrics for this particular set of 5 features. The training and cross-validation processes may further be repeated until other possible combinations of 5 features are tested. In some embodiments, a combination of 2, 3, 4, 6 features, or other suitable numbers, may also be tested. In some embodiments, a limited number of features are used to train the CI model to achieve a balance between having sufficient features to describe an accurate story with a satisfactory error and avoiding an excessive number of features that make the model narrative become difficult to understand and that could overfit the data.

In some embodiments, the cross-validation process that includes leaving one test subject out as a testing set may be referred to as leave one out cross validation ("LOOCV").

In some embodiments, in addition to features used in the training the CI model and predicting the cumulative score, additional features that best correlate (either individually or collectively) with the cumulative score may also be reported in a clinical display that is to be. The additional features may be reported even though they are not included in training the CI model or the prediction of the cumulative score.

In various embodiments, the training techniques for training a machine learning model may be supervised, semi-supervised, or unsupervised. In supervised training, the machine learning algorithms may be trained with a set of training samples that are labeled. The labels for each training sample may be a binary value, a multi-class value, or a continuous variable. In some cases, an unsupervised learning technique may be used. The samples used in training are not labeled. Various unsupervised learning techniques such as clustering may be used. In some cases, the training may be semi-supervised with the training set having a mix of labeled samples and unlabeled samples.

The parameters may be transformed into one or more latent space. For example, using a partial least squares method, the parameters are transformed into a lower dimensional latent space before the values are converted into a machine learning model.

A machine learning model may be associated with an objective function, which generates a metric value that describes the objective goal of the training process. For example, the training intends to reduce the error rate of the model in determining the MMSE score estimate. In such a case, the objective function may monitor the error rate of the machine learning model compared to an actual score. Such an objective function may be called a loss function. Other forms of objective functions may also be used, particularly for unsupervised learning models whose error rates are not easily determined due to the lack of labels. In various embodiments, the error rate may be measured as cross-entropy loss, L1 loss (e.g., the absolute distance between the predicted value and the actual value), L2 loss (e.g., root mean square distance).

A machine learning model may take various suitable structures. For example, in a neural network, the neural network may receive an input and generate an output. The neural network may include different kinds of layers, such as convolutional layers, pooling layers, recurrent layers, fully connected layers, and custom layers. A convolutional layer convolves the input of the layer with one or more kernels to generate convolved features. Each convolution result may be associated with an activation function. A convolutional layer may be followed by a pooling layer that selects the maximum value (max pooling) or average value (average pooling) from the portion of the input covered by the kernel size. The pooling layer reduces the spatial size of the extracted features. In some embodiments, a pair of convolutional layers and pooling layer 540 may be followed by a recurrent layer that includes one or more feedback loops. The recurrent layer may be gated in the case of an LSTM. The feedback may be used to account for position relationships among methylation variants. The neural network may also include multiple fully connected layers that have nodes connected to each other. The fully connected layers may be used for classification and regression. In some embodiments, one or more custom layers may also be presented for the generation of a specific format of output. The order of layers and the number of layers in the neural network may vary. In some embodiments, a neural network includes one or more convolutional layers but may or may not include any pooling layer or recurrent layer. If a pooling layer is present, not all convolutional layers need to be followed by a pooling layer. A recurrent layer may also be positioned differently at other locations of the neural network. For each convolutional layer, the sizes of kernels (e.g., 3x3, 5x5, 7x7, etc.) and the numbers of kernels allowed to be learned may be different from other convolutional layers.

A machine learning model includes certain layers, nodes, kernels, and/or coefficients. Training of a machine learning model includes iterations of forward propagation and backpropagation. Each layer in a neural network may include one or more nodes, which may be fully or partially connected to other nodes in adjacent layers. In forward propagation, the neural network performs the computation in the forward direction based on outputs of a preceding layer. The operation of a node may be defined by one or more functions. The functions that define the operation of a node may include various computation operations such as convolution of data with one or more kernels, pooling, recurrent loop in RNN, various gates in LSTM, etc. The functions may also include an activation function that adjusts the weight of the output of the node. Nodes in different layers may be associated with different functions.

Each of the functions in the neural network may be associated with different coefficients (e.g. weights and kernel coefficients) that are adjustable during training. In addition, some of the nodes in a neural network each may also be associated with an activation function that decides the weight of the output of the node in forward propagation. Common activation functions may include step functions, linear functions, sigmoid functions, hyperbolic tangent functions (tanh), and rectified linear unit functions (ReLU). After input is provided into the neural network and passes through a neural network in the forward direction, the results may be compared to the training labels or other values in the training set to determine the neural network's performance. The process of prediction may be repeated for other transactions in the training sets to compute the value of the objective function in a particular training round. In turn, the neural network performs backpropagation by using gradient descent such as stochastic gradient descent (SGD) to adjust the coefficients in various functions to improve the value of the objective function.

Multiple iterations of forward propagation and backpropagation may be performed. Training may be completed when the objective function has become sufficiently stable (e.g., the machine learning model has converged) or after a predetermined number of rounds for a particular set of training samples.

### IX. Experimental Result of MEG Data

Participants were 10 cognitively normal (N) and 10 age-matched non-normal patients (NN) relative to their cognitive function (mean age 75.1 +- 6.4 SD). Subjects underwent two MEG scans approximately 45min apart (r1 and r2), as well as a battery of neuropsychological assessments. In the scanner, subjects were presented with a random series of standard and deviant tones (50 msec tone duration, every 2.5 seconds, 5:1 proportion) for a total of 250 tones. Data collected from 100 ms prior to stimulus presentation to 500 ms after presentation represented one epoch, and only responses to the standard tone were analyzed. Data from the ipsilateral sensor were analyzed that showed the most stable response to the tones across epochs, as the ipsilateral response to simple sound stimuli has been shown to display significant delays in different peaks of the neural response.

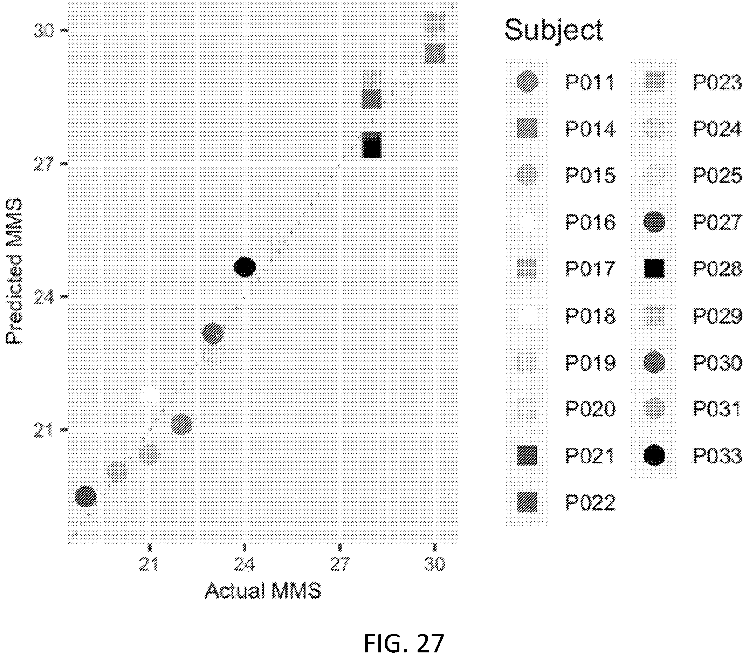

A single, simple MEG-derived metric that correctly distinguishes between the subject group was found. Three distinct peaks (A, B, and C) in the neural response to the tone were found. Thirteen features describing peak variability, percentage, and timing were used to quantify the response. Features were computed using the average signal over epochs and also the heatmap, a visual representation of all individual epochs. The heatmap is organized such that epochs are arranged on the vertical axis based on a specific feature, such as signal similarity or peak latency. Single features showed NNs as significant outliers when compared to Ns (outlier Grubb score > 3). A combination of a minimal feature set achieved perfect separability between the two groups (leave-one-out cross validation, Gaussian Naive Bayes classifier, features AₗₐₜBₒₙ, BₗₐₜB_{off}, A_{pct}, and *r1r2* Bᵥₐᵣ). Using a partial least squares model to reduce dimensionality, the set of features was also able to predict subjects' Mini-Mental tests scores (r = .99, p < 2.23e⁻¹⁶, MAE = 0.413). FIG. 27 shows the result of a regression model in predicting MMSE score of a number of subjects.

The strongest feature in separating N and NN was the variability of B signal shape between the 2 runs. N showed very little change between runs. For NN, there was a subgroup with more variability in r2 than r1 consistent with fatigue as noted above. There was a second subgroup with decreased variability in r2 suggesting increased cognitive engagement from the rest period for these NN.

Other features of NN subjects might also have readily identifiable behavioral correlates, such as a heightened startle response to the stimulus with increased A_{pct}, and consequent neural fatigue evident in loss of *r1r2* A_{pct} and *r1r2* B_{pct} in these NN subjects.

It was found that the information in peak timing features in NN highly reminiscent of EKG results. For example, the only 2 NN subjects with a prolonged AₗₐₜBₒₙ were among the 4 NN subjects with normal % A, a result consistent with a possible protective effect from the delay on cognitive fatigue.

Similar to how EKG is used to assess heart function, the combination of features identified here provides an objective and subject-specific clinical tool for clinicians to monitor an elderly subject's cognitive status. Such a screening device may prove useful in assessing response to therapies as well as cognitive function in at-risk individuals.

### X. Analysis and Graphical Representations of EEG Data

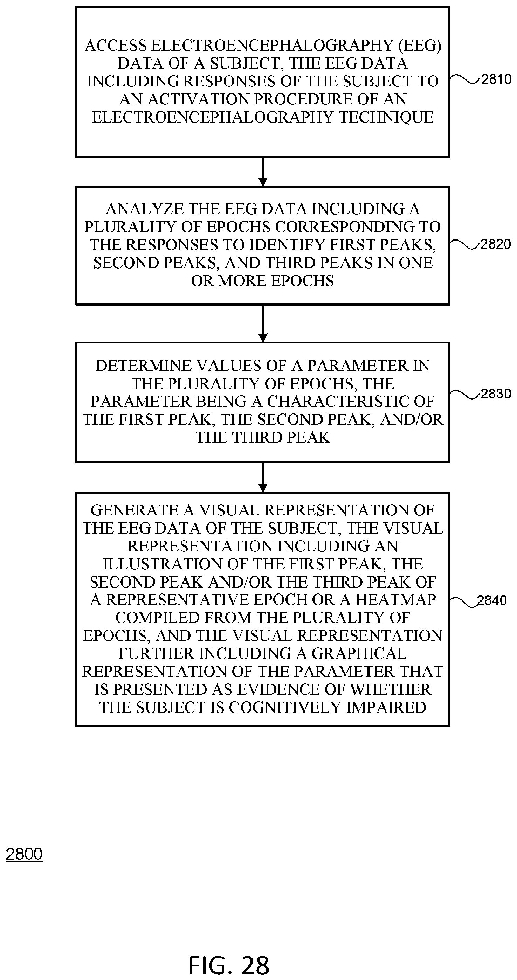

FIG. 28 is a flowchart depicting an example process 2800 for analyzing and graphically representing EEG data, in accordance with some embodiments. The data processed and the graphical representations of data may allow a machine learning model or a medical professional to determine whether there is evidence of cognitive impairment in a subject.

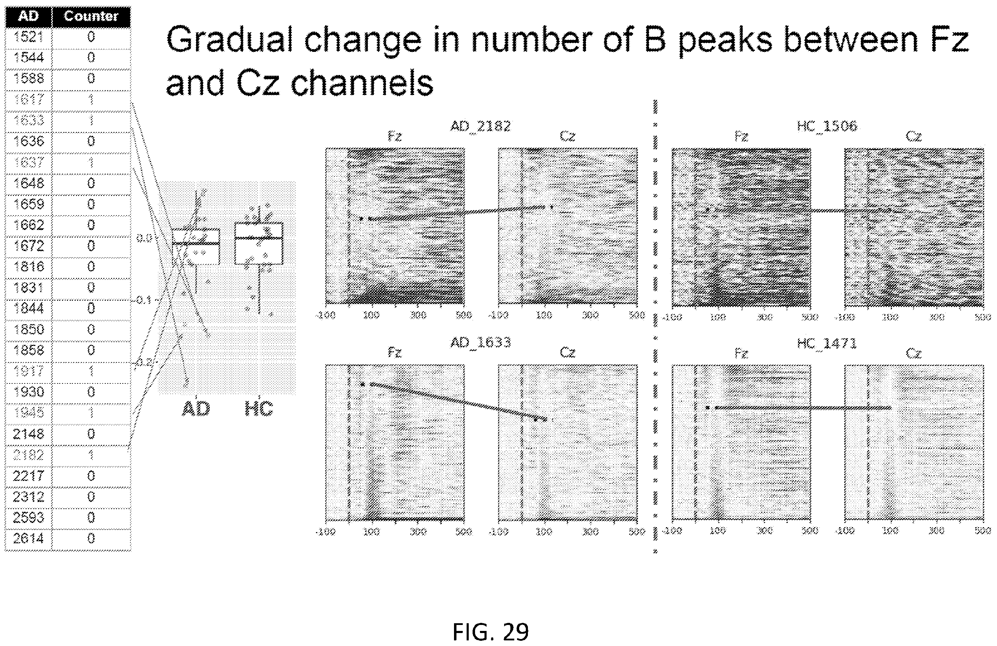

In step 2810, electroencephalography (EEG) data of a subject is accessed. The EEG data includes responses of the subject to an activation procedure of an electroencephalography technique, such as those discussed in FIG. 1 through FIG. 5B. In step 2820, the EEG data is analyzed. The EEG data includes a plurality of epochs corresponding to the responses to identify first peaks, second peaks, and third peaks in one or more epochs. Examples of EEG peaks are discussed in FIG. 5B and throughout this disclosure. In step 2830, values of a parameter in the plurality of epochs are determined. The parameter is a characteristic of the first peak, the second peak, and/or the third peak. The selection of a parameter is discussed throughout the disclosure on the discussion of parameter (feature) selection. FIG. 29 through FIG. 36 provides additional examples of parameters may be used.

For example, features were created to capture signal characteristics in the evoked responses and heatmaps that differentiated healthy subjects from subjects with AD. Selection criteria may include sorting AD subjects based on the number of features in which they are outside the normal distribution. In increasing order (e.g., subjects with fewest number of features first), the feature may also be included in the set if it provides new information (e.g., if PZ_aucB is already in the feature set, include PZ_aucC, but not P3_aucB). If two features with the same information are candidates, the one that classifies the most AD subjects overall may be selected.

In step 2840, a visual representation of the EEG data of the subject is generated. The visual representation may include an illustration of the first peak, the second peak and/or the third peak of a representative epoch or a heatmap compiled from the plurality of epochs. The visual representation further may further include a graphical representation of the parameter that is presented as evidence of whether the subject is cognitively impaired. FIG. 29 through FIG. 46 shows various examples of the graphical representation. Each of the graphical representations in FIG. 29 through FIG. 46 show that certain parameters can be used as evidence of cognitive impairments. The parameter may be graphically represented for a medical professional to review. Alternatively or additionally, one or more parameters may be used as features in a machine learning model to predict cognitive impairment.

FIG. 29 is a graphical illustration of a parameter for the gradual change in number of B peaks between two channels, in accordance with some embodiments. The example uses the Fz and Cz channels for EEG data, but other electrode sites may also be used. The parameter measures the increase or decrease in the number of B peaks between Fz and Cz channels. The calculation may include sorting the Fz heatmap based on the average signal in each epoch within the B peak window (B onset to B offset), such that epochs with lower AUC (area under the curve, e.g., more blue) are at the bottom of the heatmap. The sorting may be repeated for the Cz heatmap. In turn, the number of epochs that have AUC < 0 in each channel Fz or Cz may be counted. A line from the number of B peaks in Fz to the number of B peaks in Cz may be fit. Negative slopes mean the number of B peaks decreases between Fz and Cz. FIG. 29 shows that AD subjects often are associated with a slope between the channel Fz and the channel Cz. Normal subjects usually have a flat line, meaning the number of epochs that have B peak remain the same in the Fz channel and the Cz channel.

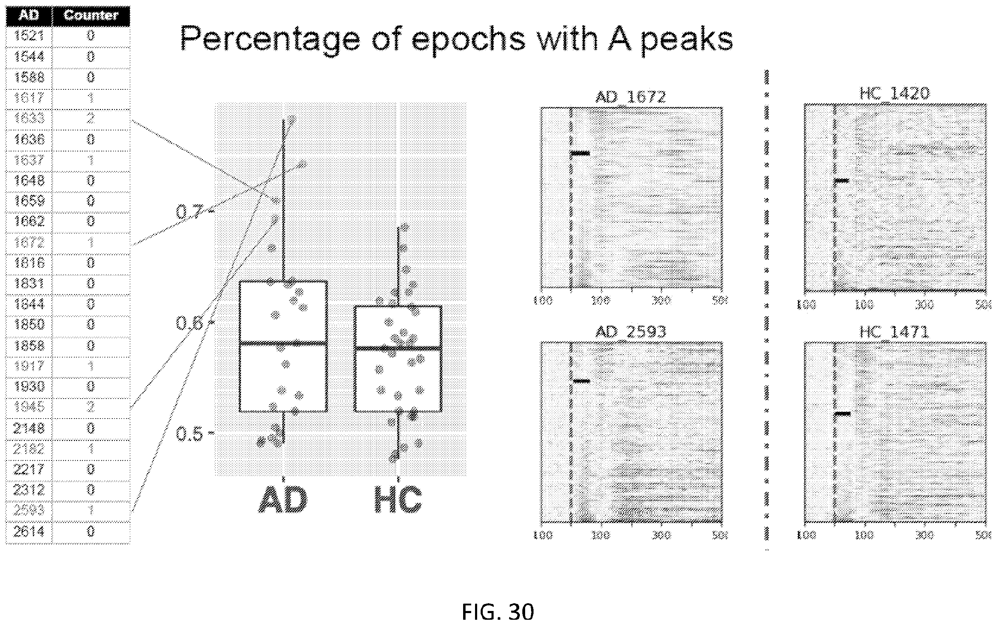

FIG. 30 is a graphical illustration of a parameter for the percentage of epochs with A peaks, in accordance with some embodiments. The parameter measures the percentage of all epochs that have an A peak (average AUC > 0). The calculation may include sorting the heatmap based on the average signal in each epoch within the A peak window (A onset to B onset), such that epochs with higher AUC (area under the curve, e.g., more red) are at the bottom of the heatmap. In turn, the number of epochs that have AUC > 0 is counted and is divided by the total number of epochs. FIG. 30 shows that subjects with AD often have a higher percentage of epochs with A peaks.

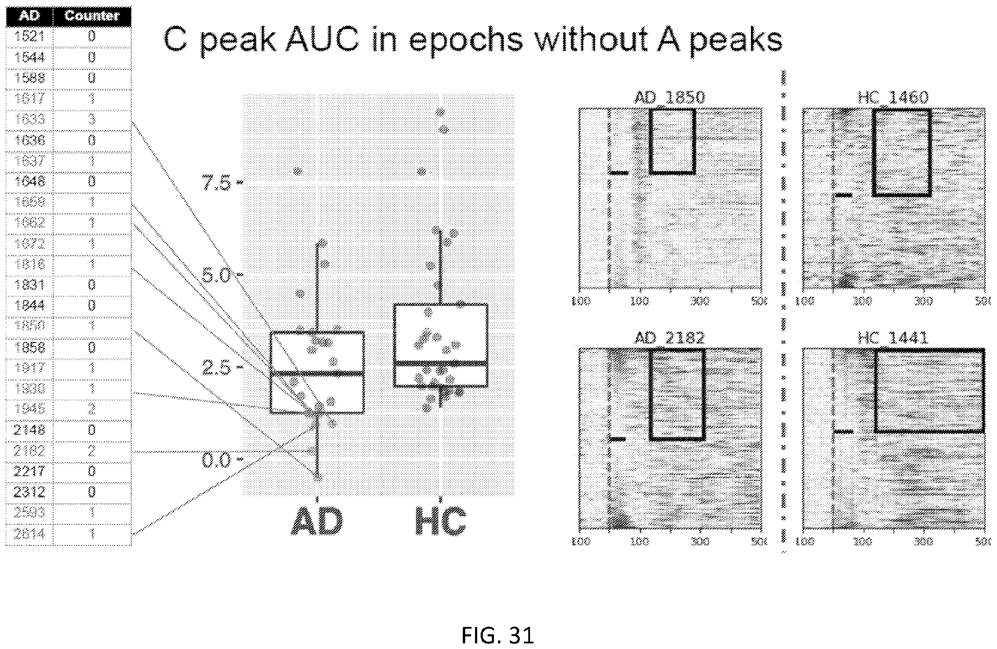

FIG. 31 is a graphical illustration of a parameter for C peak AUC in epochs without A peaks. The parameter measures the amount of red in the C peak time window B offset to C offset) averaged over epochs without A peaks. The calculation may include sorting the heatmap based on the average signal in each epoch within the A peak window (A onset to B onset), such that epochs with higher AUC (e.g., more red) are at the bottom of the heatmap. The average AUC in the C peak time window (B offset to C offset) for epochs without A peak (epochs above the A peak mark in the heatmap) is then calculated. The rectangular box in each heatmap in FIG. 31 shows the AUC in the C peaks for epochs that have no A peak. The little horizontal line in each heatmap is the A peak cutoff mark. FIG. 31 shows that subjects with AD tend to have a lower C peak AUC in epochs without A peaks.

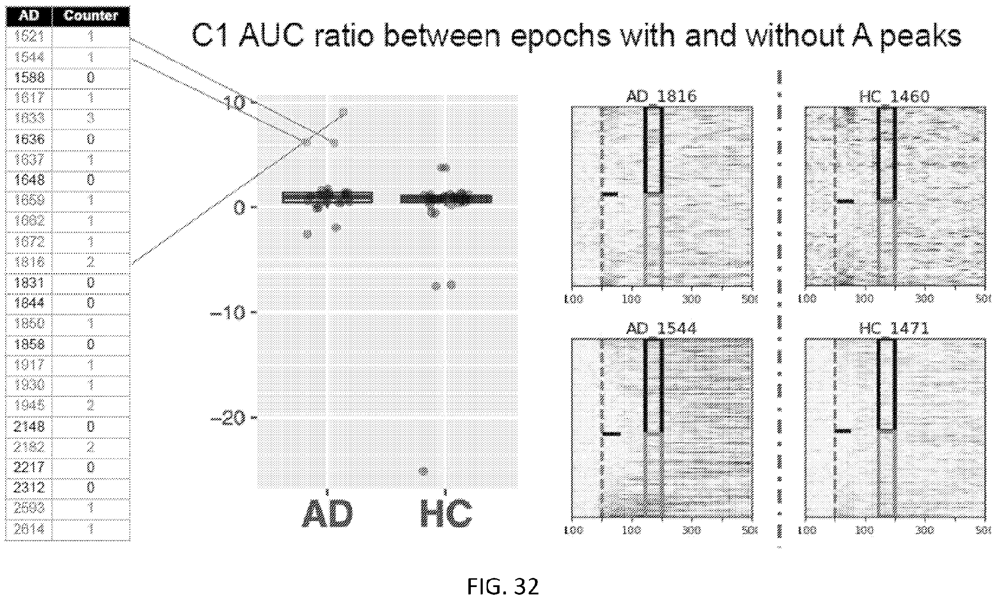

FIG. 32 is a graphical illustration of a parameter for C1 AUC ratio between epochs with and without A peaks. The parameter measures the ratio of average area under the curve in the time interval 145-200 ms (C1), between epochs with A peaks and epochs without A peaks. On broader terms, the parameter measures, on average, how much more red is the C1 region for epochs below the A peak mark compared to the epochs above the A peak mark. The calculation may include sorting the heatmap based on the average signal in each epoch within the A peak window (A onset to B onset), such that epochs with higher AUC (e.g., more red) are at the bottom of the heatmap. In turn, the average AUC in the 145-200ms time window for all epochs with A peak (below the A peak mark in the heatmap) is calculated. The average AUC in the 145-200ms time window for all epochs without A peak (above the A peak mark in the heatmap) is also calculated. The ratio is computed based on the two average AUC numbers. The more red C1 is in the A peak region, compared to the no-A peak regions, the higher the ratio. In FIG. 32, two rectangular boxes in each heatmap illustrate the AUC for the epochs with A peak and the epochs without A peak. FIG. 32 shows that some subjects with AD may present a much higher ratio and this parameter may be evidence of cognitive impairment.

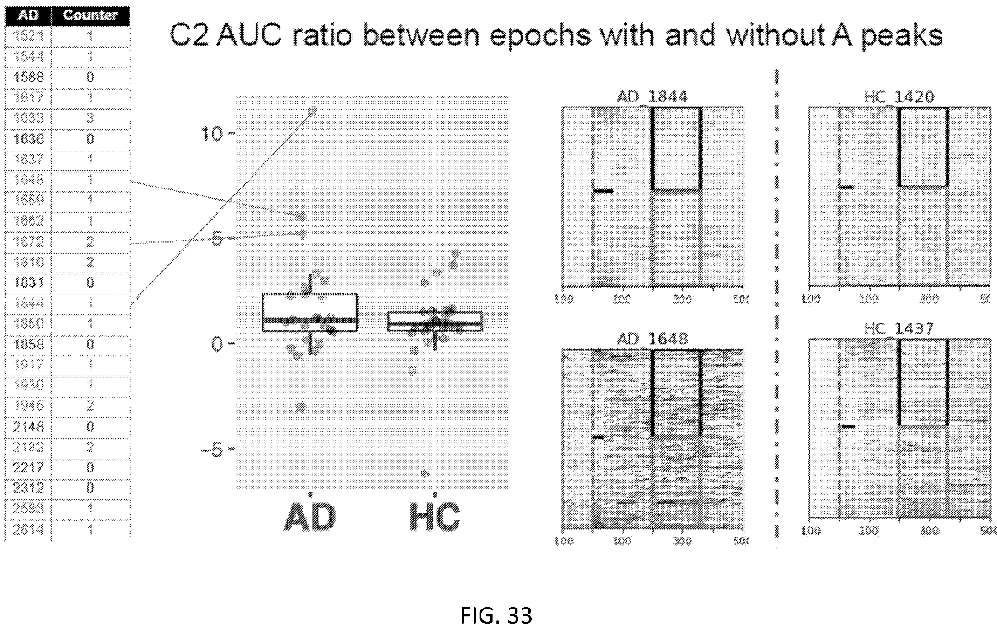

FIG. 33 is a graphical illustration of a parameter for C2 AUC ratio between epochs with and without A peaks. The parameter measures the ratio of average area under the curve in the time interval 200-360 ms (C2), between epochs with A peaks and epochs without A peaks. On broader terms, the parameter measures, on average, how much more red is the C2 region for epochs below the A peak mark compared to the epochs above the A peak mark. The calculation may include sorting the heatmap based on the average signal in each epoch within the A peak window (A onset to B onset), such that epochs with higher AUC (e.g., more red) are at the bottom of the heatmap. In turn, the average AUC in the 200-360ms (C2) time window for all epochs with A peak (below the A peak mark in the heatmap) is calculated. The average AUC in the 200-360ms (C2) time window for all epochs without A peak (above the A peak mark in the heatmap) is also calculated. The ratio is computed based on the two average AUC numbers. The more red C2 is in the A peak region, compared to the no-A peak regions, the higher the ratio. In FIG. 33, two rectangular boxes in each heatmap illustrate the AUC for the epochs with A peak and the epochs without A peak. FIG. 33 shows that some subjects with AD may present a much higher ratio and this parameter may be evidence of cognitive impairment.

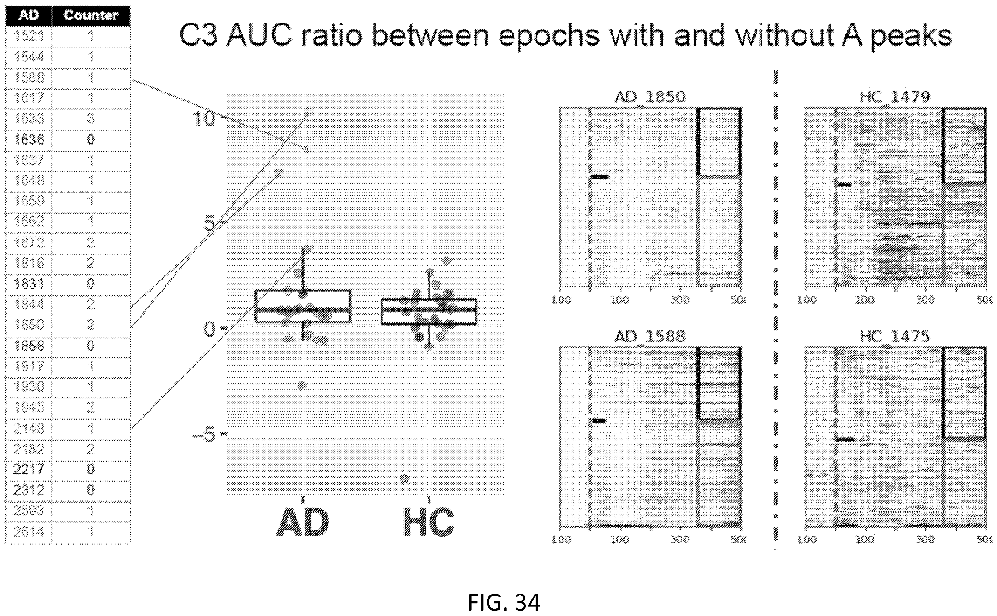

FIG. 34 is a graphical illustration of a parameter for C3 AUC ratio between epochs with and without A peaks. The parameter measures ratio of average area under the curve in the time interval 360-500 ms (C3), between epochs with A peaks between epochs with A peaks and epochs without A peaks. On broader terms, the parameter measures, on average, how much more red is the C3 region for epochs below the A peak mark compared to the epochs above the A peak mark. The calculation may include sorting the heatmap based on the average signal in each epoch within the A peak window (A onset to B onset), such that epochs with higher AUC (e.g., more red) are at the bottom of the heatmap. In turn, the average AUC in the 200-360ms (C2) time window for all epochs with A peak (below the A peak mark in the heatmap) is calculated. The average AUC in the 360-500 ms (C3) time window for all epochs without A peak (above the A peak mark in the heatmap) is also calculated. The ratio is computed based on the two average AUC numbers. The more red C3 is in the A peak region, compared to the no-A peak regions, the higher the ratio. In FIG. 34, two rectangular boxes in each heatmap illustrate the AUC for the epochs with A peak and the epochs without A peak. FIG. 34 shows that some subjects with AD may present a much higher ratio and this parameter may be evidence of cognitive impairment.

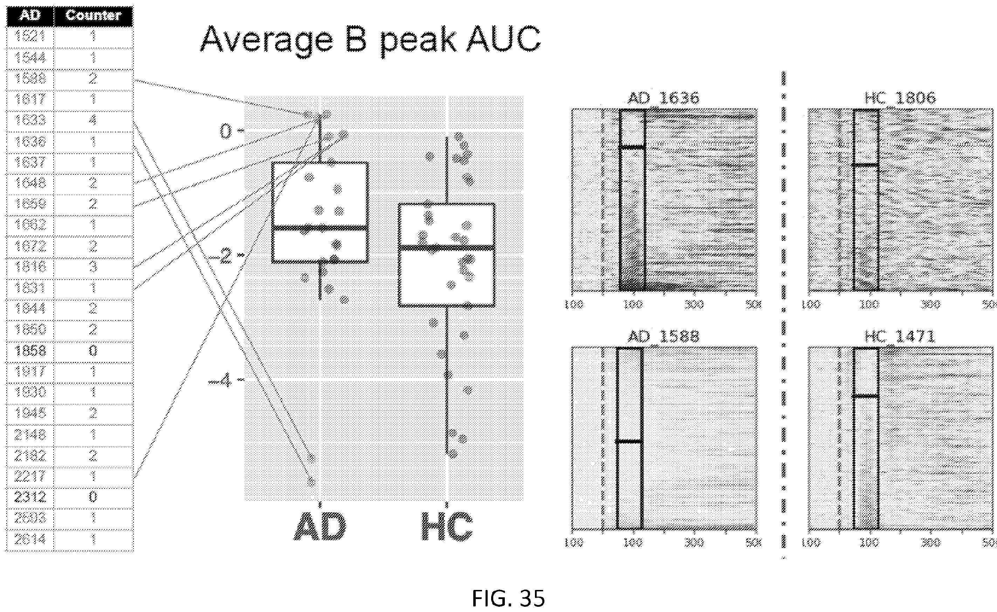

FIG. 35 is a graphical illustration of a parameter for average B peak AUC. The parameter measures the average amount of signal in the B peak time window, modulated by the number of epochs with B peaks. The calculation may include sorting the heatmap based on the average signal in each epoch within the B peak window (B onset to B offset), such that epochs with lower AUC (e.g., more blue) are at the bottom of the heatmap. In turn, the average AUC in the B peak time window is calculated. The number epochs that have AUC < 0 is counted and is divided by the total number of epochs. An average is determined based on the calculation. FIG. 35 shows that some subjects with AD may have a more extreme average B peak AUC.

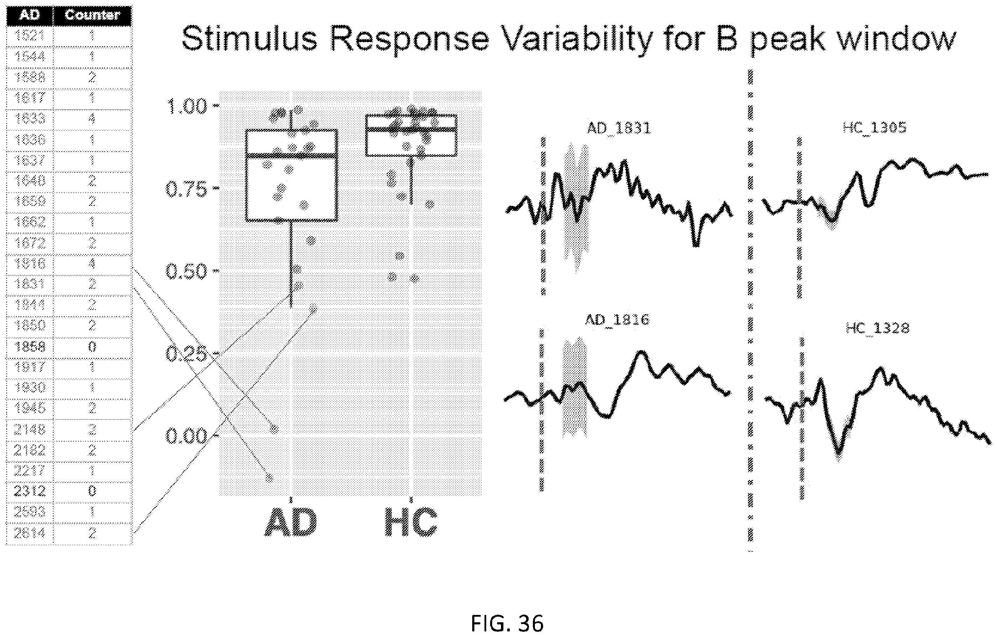

FIG. 36 is a graphical illustration of a parameter for stimulus response variability for B peak window. The parameter measures signal similarity among all epochs within B peak time window. The calculation may include splitting the epochs into 2 halves and calculate the average response over epochs in each half. The Pearson correlation between the 2 signals within the B peak time window (B onset to B offset) is calculated. This generates a single number between -1 and 1, with 1 being perfectly correlated signals, 0 meaning no association between the 2 signals. The procedure may be repeated for N times, such as one hundred times, where the epochs in each half change randomly. This generates a distributions of correlation values. The median correlation of the distribution is computed. Higher value means that signal across epochs is similar within the B peak window. FIG. 36 shows that subjects with AD have a higher variability than normal subjects.

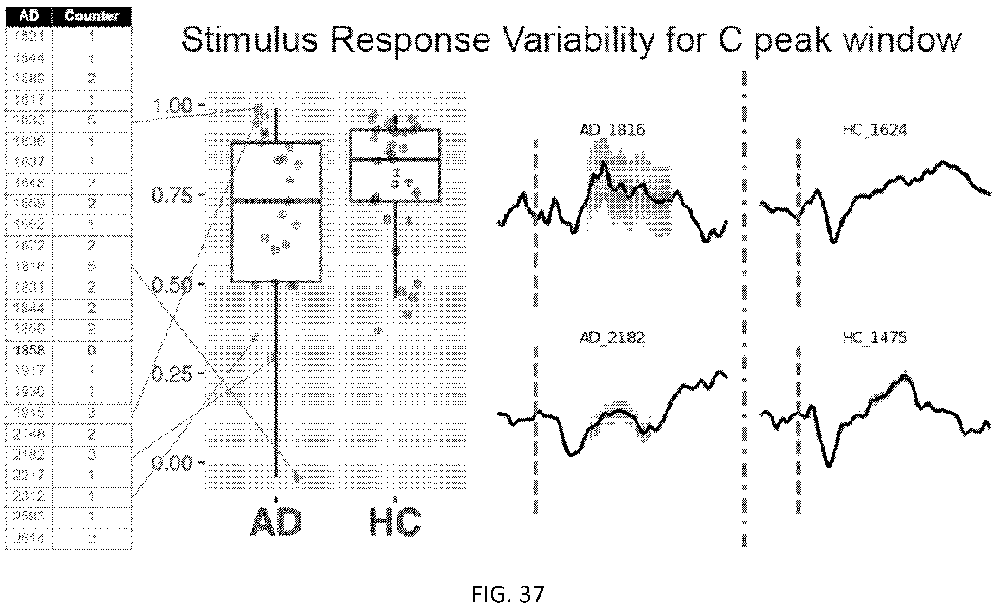

FIG. 37 is a graphical illustration of a parameter for stimulus response variability for C peak window. The parameter measures signal similarity among all epochs within C peak time window. The calculation may include splitting the epochs into 2 halves and calculate the average response over epochs in each half. The Pearson correlation between the 2 signals within the C peak time window (B offset to C offset) is calculated. This generates a single number between -1 and 1, with 1 being perfectly correlated signals, 0 meaning no association between the 2 signals. The procedure may be repeated for N times, such as one hundred times, where the epochs in each half change randomly. This generates a distributions of correlation values. The median correlation of the distribution is computed. Higher value means that signal across epochs is similar within the C peak window. FIG. 37 shows that subjects with AD have a higher variability than normal subjects.

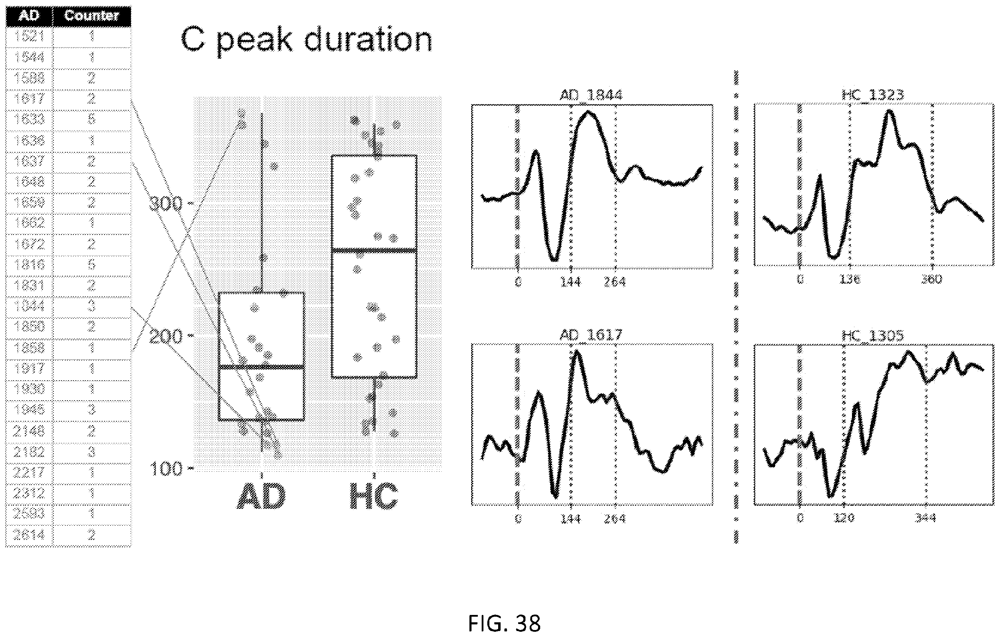

FIG. 38 is a graphical illustration of a parameter for C peak duration. The parameter measures the duration of the C peak in the averaged response. The calculation may include computing the time difference between B offset and C offset. This generates a distributions of correlation values. The median correlation of the distribution is computed. Higher value means that signal across epochs is similar within the C peak window. FIG. 38 shows that the distribution of the C peak duration for subjects with Ads and normal subjects. The C peak duration is a useful parameter as prolonged sensor processing might be expected to be problematic.

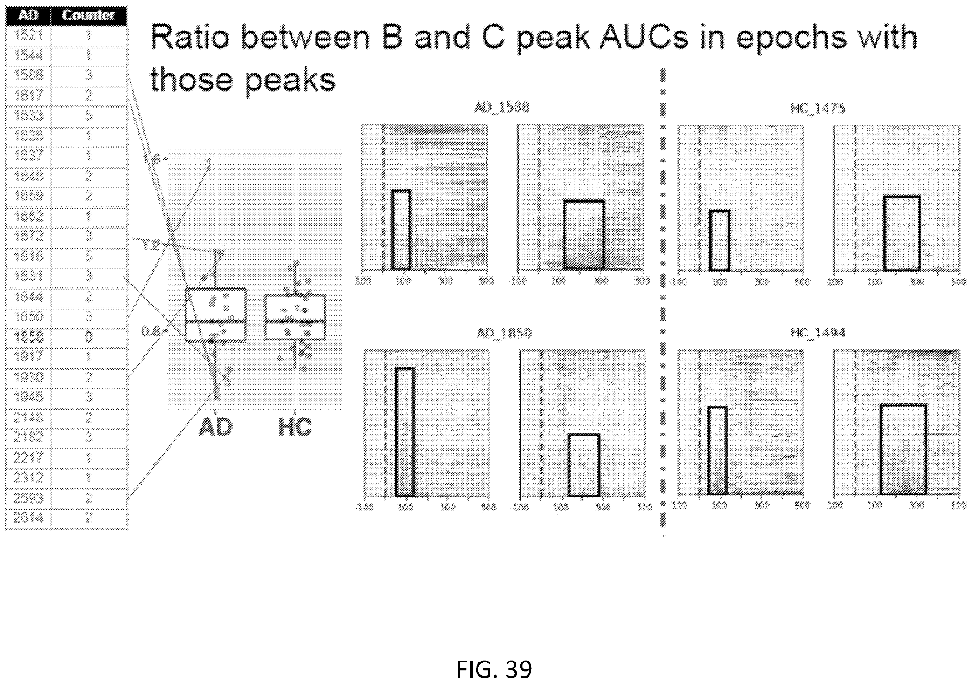

FIG. 39 is a graphical illustration of a parameter for the ratio between B and C peak AUCs in epochs with those peaks. The parameter measures the relationship between the average amount of signal in the B and C peak time windows, only within the epochs that contain those peaks. The calculation may include determining the average AUC in the time window between B onset and offset for all epochs with B peaks (below the last B peak in the heatmap). 2. The average AUC in the time window between B and C offset for all epochs with C peaks (below the last C peak in the heatmap) is calculated. The absolute ratio between the results of the last two steps is calculated. FIG. 39 shows that the parameter is a ratio between the average AUC in the two rectangles for each subject. FIG. 39 shows that distribution of the values of parameters for AD subjects and healthy controls (HC).

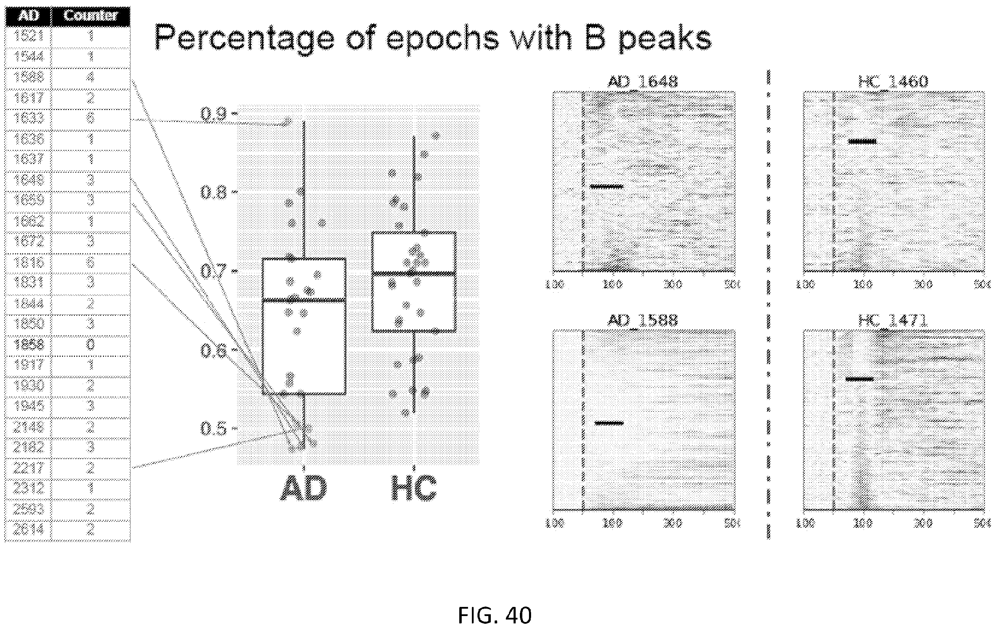

FIG. 40 is a graphical illustration of a parameter for percentage of epochs with B peaks. The parameter measures percentage of all epochs that have an B peak (average AUC < 0). The calculation may include sorting the heatmap based on the average signal in each epoch within the B peak window (B onset to offset), such that epochs with lower AUC (area under the curve, more blue), are at the bottom of the heatmap. In turn, the number of epochs that have AUC < 0 is counted and is divided by the total number of epochs. FIG. 40 shows that AD subjects on average have fewer B peaks compared to healthy controls and a low B peak average can be an evidence of cognitive impairment such as AD.

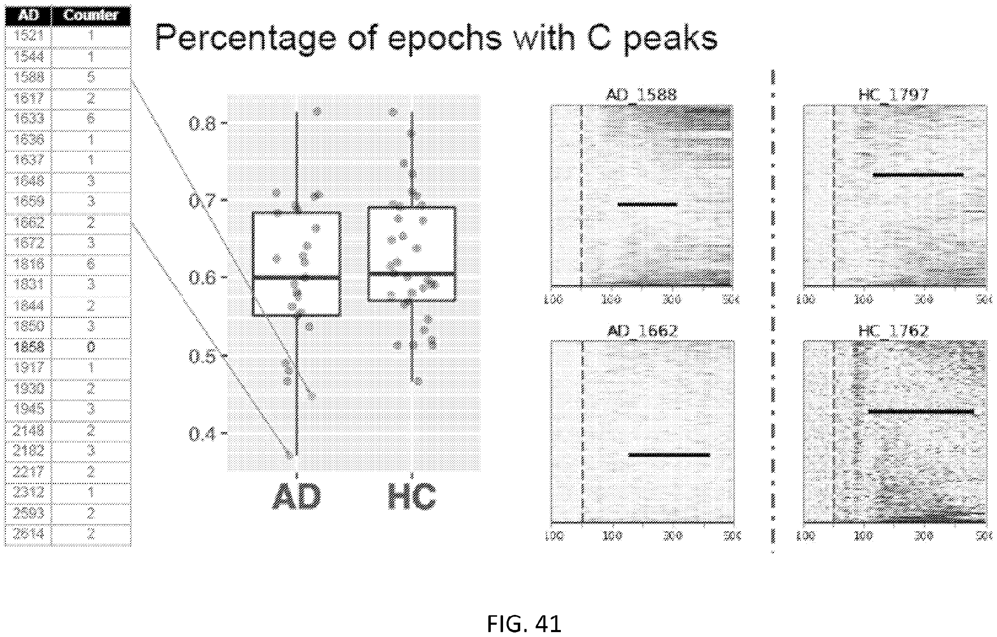

FIG. 41 is a graphical illustration of a parameter for percentage of epochs with C peaks. The parameter measures percentage of all epochs that have an C peak (average AUC > 0). The calculation may include sorting the heatmap based on the average signal in each epoch within the C peak window (B offset to C offset), such that epochs with higher AUC (area under the curve, more red), are at the bottom of the heatmap. In turn, the number of epochs that have AUC > 0 is counted and is divided by the total number of epochs. FIG. 41 shows that AD subjects on average have fewer C peaks compared to healthy controls and a low C peak average can be an evidence of cognitive impairment such as AD.

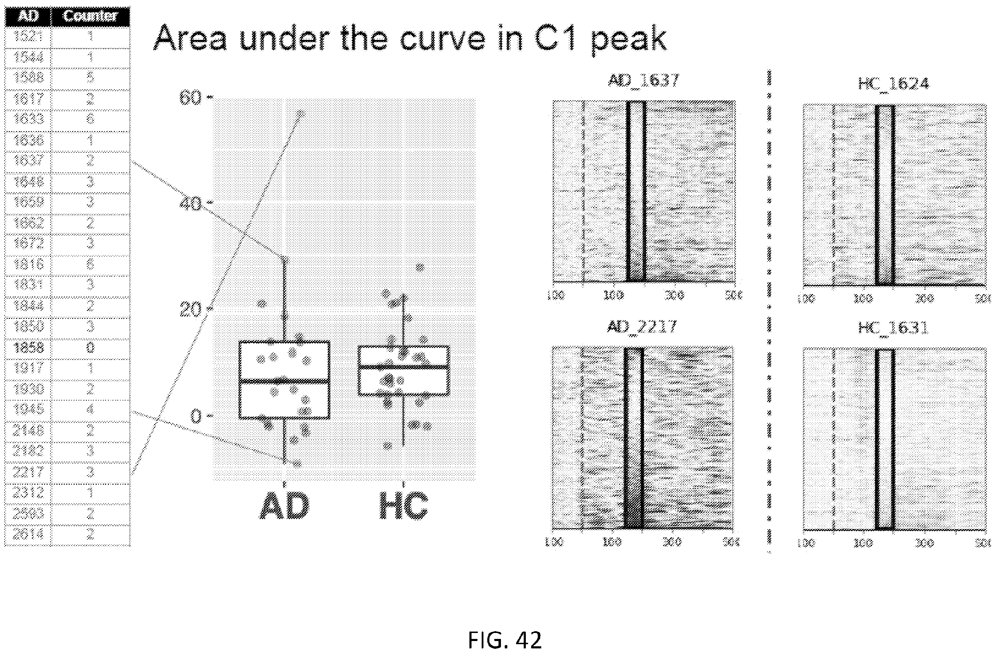

FIG. 42 is a graphical illustration of a parameter for area under the curve in C1 peak. The parameter measures amount of red in the C1 peak time window (145 to 200 ms), averaged over all epochs. The calculation may include sorting the heatmap based on the average signal in each epoch within the C peak window (B offset to C offset), such that epochs with higher AUC (area under the curve, more red), are at the bottom of the heatmap. The average AUC in the C1 peak time window (145 to 200ms) is calculated over all epochs. FIG. 42 shows that the more red in the rectangular region for a subject, the higher is the value of the parameter. FIG. 42 shows that an abnormal value of this parameter can be evidence of cognitive impairment such as AD.

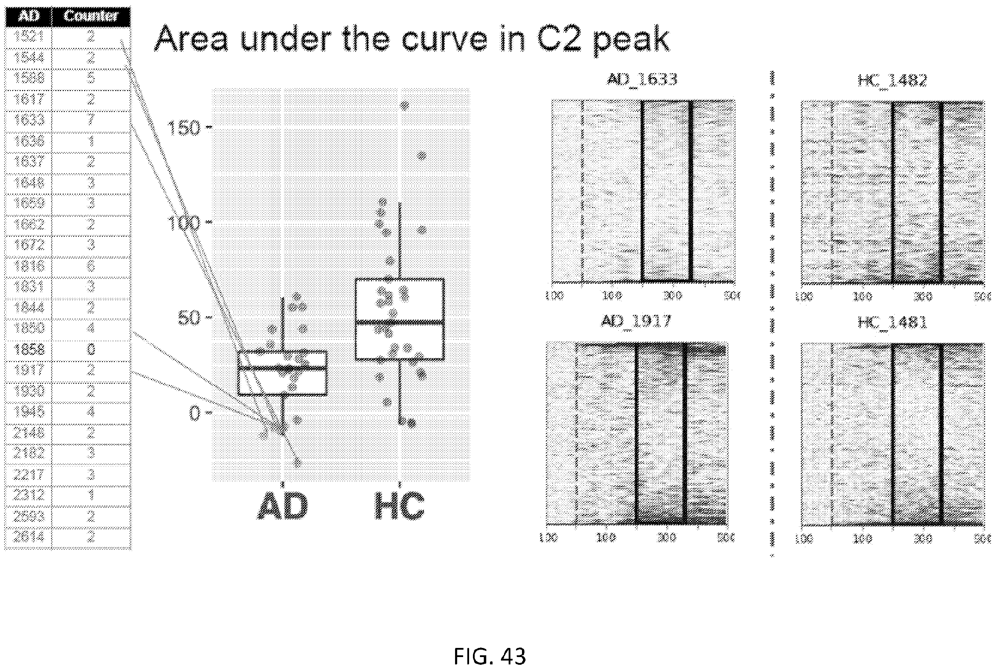

FIG. 43 is a graphical illustration of a parameter for area under the curve in C2 peak. The parameter measures amount of red in the C2 peak time window (200 to 360 ms), averaged over all epochs. The calculation may include sorting the heatmap based on the average signal in each epoch within the C peak window (B offset to C offset), such that epochs with higher AUC (area under the curve, more red), are at the bottom of the heatmap. The average AUC in the C2 peak time window (200 to 360 ms) is calculated over all epochs. FIG. 43 shows that the more red in the rectangular region for a subject, the higher is the value of the parameter. FIG. 43 shows that an abnormally low value of this parameter can be evidence of cognitive impairment such as AD.

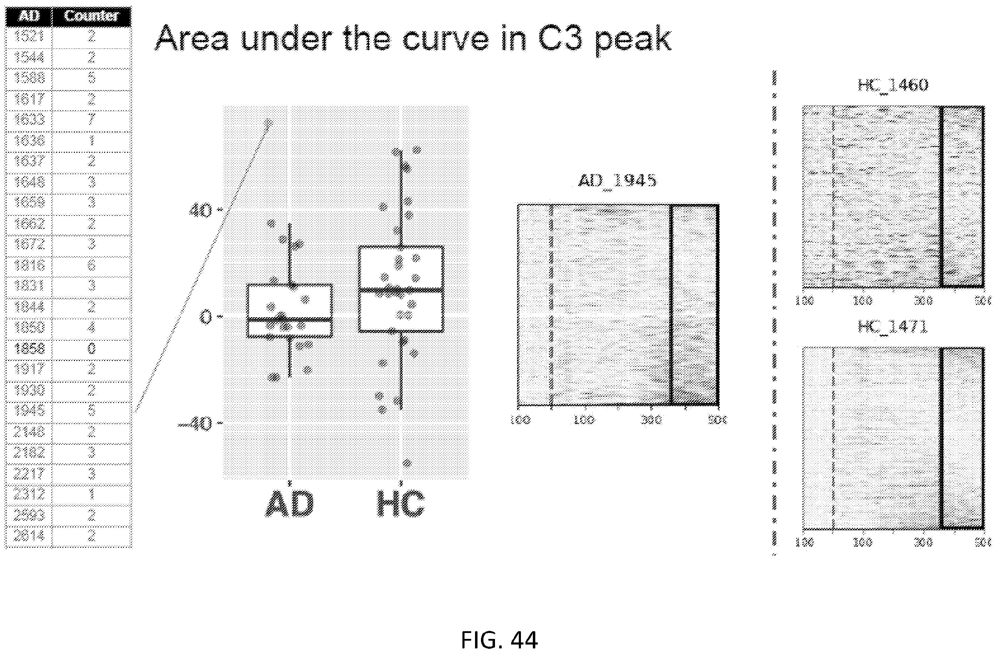

FIG. 44 is a graphical illustration of a parameter for area under the curve in C3 peak. The parameter measures amount of red in the C3 peak time window (360 to 500 ms), averaged over all epochs. The calculation may include sorting the heatmap based on the average signal in each epoch within the C peak window (B offset to C offset), such that epochs with higher AUC (area under the curve, more red), are at the bottom of the heatmap. The average AUC in the C3 peak time window (360 to 500 ms) is calculated over all epochs. FIG. 44 shows that the more red in the rectangular region for a subject, the higher is the value of the parameter. FIG. 44 shows that an abnormally high value of this parameter can be evidence of cognitive impairment such as AD.

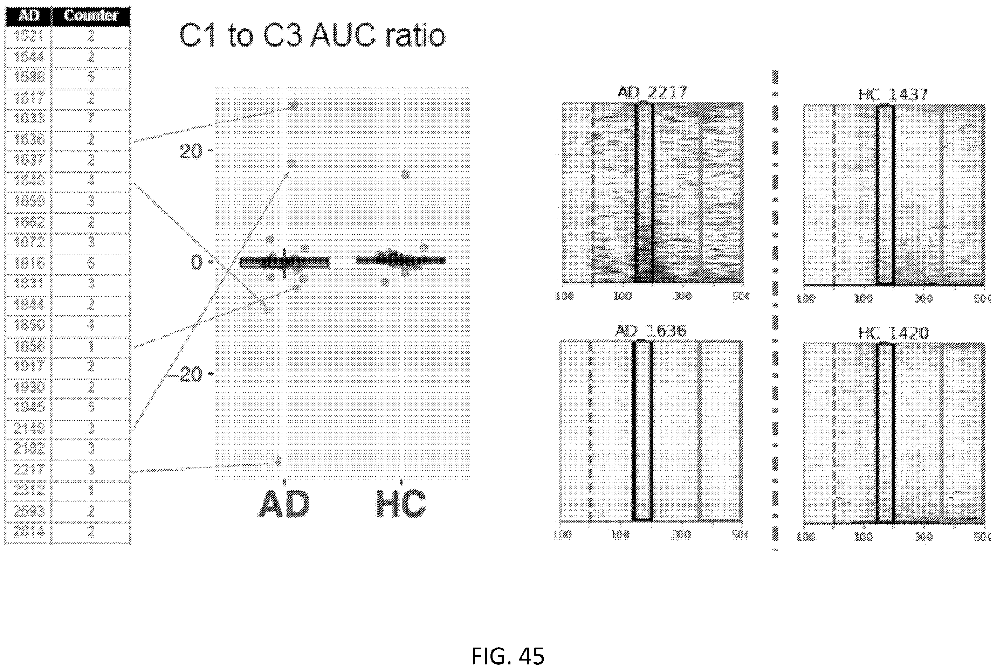

FIG. 45 is a graphical illustration of a parameter for C1 to C3 AUC ratio. The parameter measures ratio of average area under the curve in the time interval C1 (145-200 ms) and C3 (360-500 ms) over all epochs. In broader terms, the parameters determine on average how much more red is the C1 region compared to the C3 region. The calculation may include sorting the heatmap based on the average signal in each epoch within the C peak window (B offset to C offset), such that epochs with higher AUC (area under the curve, more red), are at the bottom of the heatmap. The average AUC in the 145-200 ms time window for all epochs is calculated. The average AUC in the 360-500 ms time window for all epochs is calculated. The ratio between the last two steps is computed. FIG. 45 shows that an abnormal ratio for this parameter can be evidence of cognitive impairment such as AD.

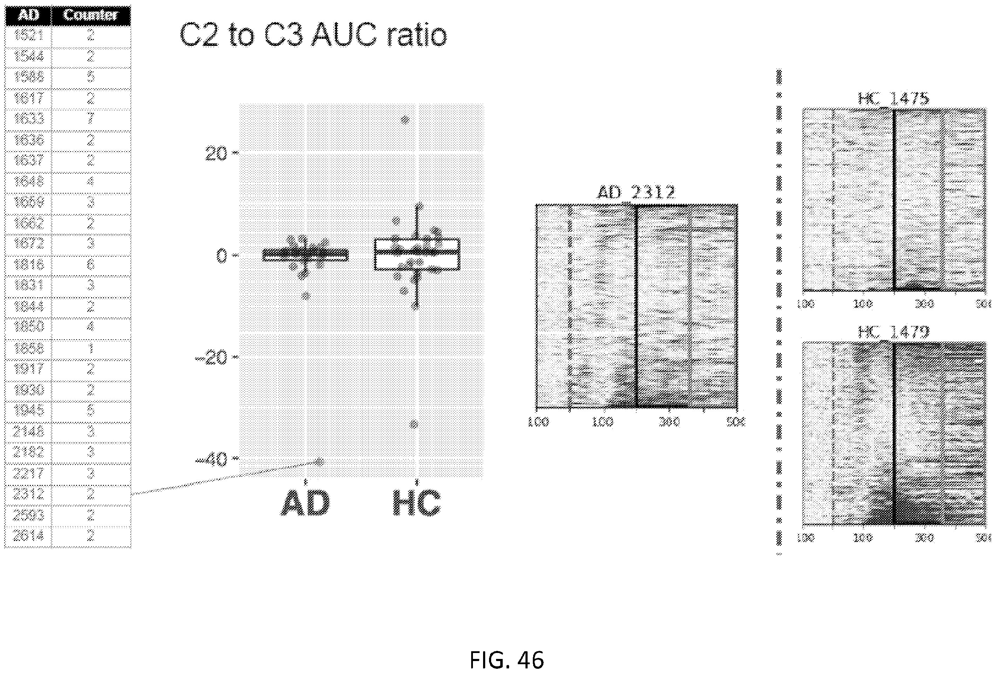

FIG. 46 is a graphical illustration of a parameter for C2 to C3 AUC ratio. The parameter measures ratio of average area under the curve in the time interval C2 (200-360 ms) and C3 (360-500 ms) over all epochs. In broader terms, the parameters determine on average how much more red is the C2 region compared to the C3 region. The calculation may include sorting the heatmap based on the average signal in each epoch within the C peak window (B offset to C offset), such that epochs with higher AUC (area under the curve, more red), are at the bottom of the heatmap. The average AUC in the 200-360 ms time window for all epochs is calculated. The average AUC in the 360-500 ms time window for all epochs is calculated. The ratio between the last two steps is computed. FIG. 46 shows that an abnormal ratio for this parameter can be evidence of cognitive impairment such as AD.

The following feature set, exemplified in FIG. 29 through FIG. 46, to be informative in characterizing the heatmaps and potentially related to a subject's cognitive status. This set is able to perfectly separate AD subjects form health controls in the EEG dataset.

| **Feature name** | **ADs outside normal range** | **Channel** |
|---|---|---|
| Gradual change in number of B peaks between Fz and Cz channels | 6 | Fz / Cz |
| Percentage of epochs with A peaks | 4 | Cz |
| C peak AUC in epochs without A peaks | 8 | Cz |
| C1 AUC ratio between epochs with and without A peaks | 3 | P3 |
| C2 AUC ratio between epochs with and without A peaks | 3 | P4 |
| C3 AUC ratio between epochs with and without A peaks | 4 | F4 |
| Average B peak AUC | 8 | F3 |
| Stimulus Response Variability for B peak window | 4 | P4 |
| Stimulus Response Variability for C peak window | 5 | F4 |
| Ratio between B and C peak AUCs in epochs with those peaks | 7 | P4 |
| Percentage of epochs with B peaks | 6 | F3 |
| Percentage of epochs with C peaks | 2 | F4 |
| Area under the curve in C1 peak | 3 | P3 |
| Area under the curve in C2 peak | 5 | Pz |
| Area under the curve in C3 peak | 1 | P3 |
| C1 to C3 AUC ratio | 5 | P3 |
| C2 to C3 AUC ratio | 1 | F3 |

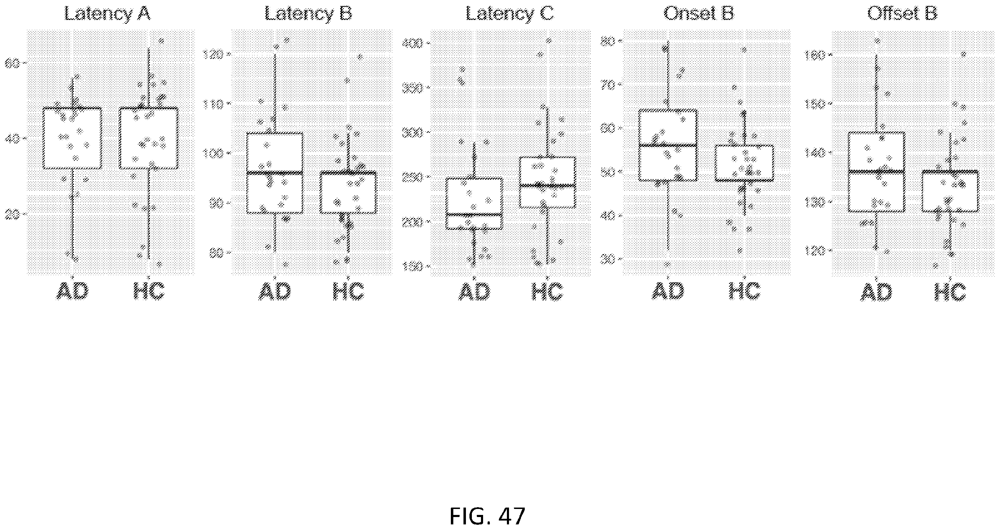

FIG. 47 is a graphical illustration of various timing features, in accordance with some embodiments. Various timing features are useful in characterizing the signal. Note that latencyB in particular could be a quite useful feature due to the delayed latency in a few ADs. However, the single delayed HC (1715) has a very unstable signal (low SRV, the lowest HC in the slide for Stimulus Response Variability for B). If SRV is employed as a signal quality metric, the dataset for 1715 would be removed, and latency would prove to be a good feature as well.

In some embodiments, peak timing (latency, onset, and offset for A, B, and C peaks) is identified first in Cz, then Pz, then Fz. In some embodiments, the analysis only moves on to the next channel in the list if the system cannot find onset and offset of B. Peak timing is computed in the averaged response over all epochs. For example, the system may start by making the B search zone (75 to 120 ms) to be positive (B peak will be a positive peak), so the system can have a standard for signal increase and decrease. The system may also identify B peak latency (signal maxima) within that time window. If signal amplitude at latency is less than 2 times the standard deviation of the baseline period (-500 to 0 ms) + the mean of the baseline, the system fails to identify B peak in that channel. If the system identifies B peak latency, the system go backwards in time from the latency to look for B peak onset. B onset is found if (signal goes below the threshold AND 35ms from latency have passed) or the signal changes direction (starts getting higher) AND 35ms from latency have passed.

If the system reaches stimulus onset (t=0) and have not found B onset, the system fails to find B peak. The system may go back to B peak latency and go forward in time to find B peak offset. B offset is found if (signal goes below the threshold AND 35ms from latency have passed) or the signal changes direction (starts getting higher) AND 35ms from latency have passed or the signal reaches same amplitude as B onset AND 35ms from latency have passed. If the system reaches the end of the signal (t=500ms) and have not found B offset, the system fails to find the B peak.

If the system identifies B peak onset, latency, and offset, the system may move on to identify A onset and C offset (A offset = B onset, C onset = B offset). The system may identify A peak latency (signal minimum) between t=0 and B onset. The system may go backwards in time from the latency to look for A peak onset. A onset is found if (signal goes above 0 AND 25ms from latency have passed) or signal goes above negative threshold AND 25ms from latency have passed or the signal changes direction (starts getting lower) and 25ms from latency have passed. If the system reaches stimulus onset (t=0) and have not found A onset, the system fails to find the A peak. Because A onset is trickier to find due to signal noise, for subject where A onset is not found in any of the 3 channels, the system uses the heuristic of A onset = 8ms to later sort heatmaps and compute features.

The system may move on to identify C offset regardless of A onset outcome. The system may identify C peak latency (signal minimum) between B offset and 400ms. The system may go forwards in time from the latency to look for C peak offset. C offset is found if (signal goes above signal value at B offset - 2 times the signal variability at baseline. If the system reaches the end of the signal, C offset is the highest voltage the signal got to before the end.

### XI. ADDITIONAL CONSIDERATIONS

Similar methodologies may be developed that may be useful in monitoring for other specific medical conditions or generally monitoring human brain function. The model described herein analyzes the neuroimaging data collected after an auditory stimulus, including the relative extent of brain activation/excitation and subsequent response to the activation. The neuroimaging data for the model may come from only a small number of the SQUID or EEG sensors generally from as few as a single sensor up to about six, although a full set of sensors (e.g., 306 sensors) may also be used.

While the invention has been described with reference to one or more embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims. In addition, all numerical values identified in the detailed description shall be interpreted as though the precise and approximate values are both expressly identified.

## Claims

1. A computer-implemented method, comprising:
accessing electroencephalography (EEG) data of a subject, the EEG data including responses of the subject to an activation procedure of an electroencephalography technique, , the EEG data being acquired from a plurality of electrode sites, each electrode site corresponding to an electrode channel;
analyzing the EEG data including a plurality of epochs corresponding to the responses to identify first peaks, second peaks, and third peaks in one or more epochs;
determining values of a parameter in the plurality of epochs, the parameter being a gradual change in number of B peaks between two electrode channels, wherein a B peak is commonly known in literature as "N100", "m100", or an awareness related negativity ("ARN") peak; and
generating a visual representation of the EEG data of the subject, the visual representation including an illustration of a heatmap compiled from the plurality of epochs, and the visual representation further including a graphical representation of the parameter that is presented as evidence of whether the subject is cognitively impaired.

## Patentansprüche

1. Computerimplementiertes Verfahren, umfassend:
Zugreifen auf Elektroenzephalographie(EEG)-Daten eines Subjekts, wobei die EEG-Daten Reaktionen des Subjekts auf einen Aktivierungsvorgang einer Elektroenzephalographie-Technik einschließen, wobei die EEG-Daten von einer Vielzahl von Elektrodenstellen erfasst werden, wobei jede Elektrodenstelle einem Elektrodenkanal entspricht;
Analysieren der EEG-Daten einschließlich einer Vielzahl von Epochen, die den Reaktionen entsprechen, um erste Peaks, zweite Peaks und dritte Peaks in einer oder mehreren Epochen zu identifizieren;
Bestimmen von Werten eines Parameters in der Vielzahl von Epochen, wobei der Parameter eine allmähliche Änderung der Anzahl von B-Peaks zwischen zwei Elektrodenkanälen ist, wobei ein B-Peak in der Literatur allgemein als "N100", "m100" oder ein Awareness Related Negativity ("ARN")-Peak bekannt ist; und
Generieren einer visuellen Darstellung der EEG-Daten des Subjekts, wobei die visuelle Darstellung eine Illustration einer Heatmap einschließt, die aus der Vielzahl von Epochen zusammengestellt ist, und die visuelle Darstellung ferner eine grafische Darstellung des Parameters einschließt, der als Beleg dafür präsentiert wird, ob das Subjekt kognitiv beeinträchtigt ist.

## Revendications

1. Procédé mis en œuvre par ordinateur, comprenant :
l'accès à des données d'électroencéphalographie (EEG) d'un sujet, les données d'EEG comprenant les réponses du sujet à une procédure d'activation d'une technique d'électroencéphalographie, les données d'EEG étant acquises à partir d'une pluralité de sites d'électrodes, chaque site d'électrode correspondant à un canal d'électrode ;
l'analyse des données d'EEG comprenant une pluralité d'époques correspondant aux réponses pour identifier des premiers pics, des deuxièmes pics et des troisièmes pics dans une ou plusieurs époques ;
la détermination de valeurs d'un paramètre dans la pluralité d'époques, le paramètre étant un changement progressif du nombre de pics B entre deux canaux d'électrode, un pic B étant couramment connu dans la littérature sous le nom de « N100 », « m100 » ou pic de négativité liée à la conscience (« ARN ») ; et
la génération d'une représentation visuelle des données d'EEG du sujet, la représentation visuelle comprenant une illustration d'une carte thermique compilée à partir de la pluralité d'époques, et la représentation visuelle comprenant en outre une représentation graphique du paramètre qui est présentée comme preuve de la déficience cognitive du sujet.
